# EUROPEAN PATENT APPLICATION

(11) **EP 4 520 334 A1**
(43) Date of publication of application: **12.03.2025**
(21) Application number: 23306492.2
(22) Date of filing: 07.09.2023
(51) Int. Cl.: A61K 31/546, A61K 38/00, A61K 39/00, A61K 45/06, A61P 35/00

(54) **METHODS AND COMPOSITIONS FOR IMPROVING IMMUNE RESPONSE**

(71) Applicant: Mnemo Therapeutics, 75016 Paris (FR); Institut National de la Santé et de la Recherche Médicale, 75013 Paris (FR); Institut Curie, 75005 Paris (FR); Centre National de la Recherche Scientifique, 75016 Paris (FR)
(72) Inventor: SAITAKIS, Michael, 75016 PARIS (FR); AMIGORENA, Sebastian, 75248 PARIS CEDEX 5 (FR); SUAREZ, Guadalupe, 75005 PARIS (FR)
(74) Representative: Plasseraud IP

(57) **Abstract**

The present invention relates to the use of KMT inhibitors and in particular of Suv39h1 inhibitors to increase immune response, and in particular immune cell mediated response, notably elicited by a vaccine or immunogenic composition.

## Description

### FIELD OF THE DISCLOSURE

The present invention relates to the use of KMT inhibitors and in particular of Suv39h1 inhibitors to increase immune response, and in particular immune cell mediated response, notably elicited by a vaccine or immunogenic composition.

### BACKGROUND

The advent of vaccines has introduced new opportunities to prevent and treat infectious diseases. As the vaccine developed, it was later introduced to treat more diseases, such as cancers. The initial cancer vaccine based on tumor cells and tumor lysates has been developed in 1980, but the outbreak of COVID-19 has urged the development of vaccine technology. Cancer vaccines mainly use tumor-associated antigens (TAAs) and tumor-specific antigens (TSAs) to activate the patient's immune system and induce both specific cellular immunity and humoral immune response to prevent tumor growth and ultimately eradicate tumor cells. Currently however, most cancer vaccines are still in the stage of preclinical and clinical research and more specific antigens and vaccine development platforms need to be developed. Indeed, unlike traditional vaccines with antigens from foreign pathogens, tumor antigens are endogenous with low immunogenicity such that tumor antigens are often difficult to elicit an effective immune response. The present application therefore fulfills an important need that may expand the use of broader types of cancer antigen in cancer vaccination platforms.

Infections also represent another major clinical burden and are often characterized by resistances of the infectious pathogens to available antibiotic or antiviral therapeutics. In (chronic) infections, T cells are exposed to persistent antigen and/or inflammatory signals. Vaccination therefore remains a major strategy to prevent developments of infectious diseases.

There is thus an urgent need in the art to provide novel therapeutics that would support or enhance immune response and in particular cell mediated immune response associated with vaccination.

It is an object of the present invention to comply with these needs and to provide improved therapeutic approaches for treatment of cancers, infectious diseases and other diseases and conditions defined herein. The object underlying the present invention is solved by the claimed subject matter.

### SUMMARY OF THE INVENTION

The present invention is resumed in the claims of the present application.

It has been previously shown by the inventors that OVA-specific T cells from Suv39h1-deficient mice stimulated upon Listeria m. infection express a stem-like mRNA signature and that inhibition of Suv39h1 in T cell increases T cell expansion and persistence after adoptive transfer. It was further shown that Suv39h1 KO CAR T cells are more resistant to terminal differentiation than Mock CAR T cells and maintain stemness and memory characteristics. These results strongly supported the high therapeutic interest of developing Suv39h1-deficient T cells for cellular therapy (e.g., CAR T cell therapy).

The inventors have now further discovered that the inhibition of Suv39h1 has a broader scope effect on T cells and, in particular, that Suv39h1 inhibition increases the antigen sensitivity and reactivity of naive T cells, which thus demonstrate increased activation at both low antigen affinity and low antigen density upon Suv39h1 inhibition. The inventors also provide further results showing that Suv39h1 primarily contributes to heterochromatin formation at stem/memory loci in differentiating naive T cells, as compared to lineage-committed central memory T cells. These new and exciting results now support a broader therapeutic use of Suv39h1 inhibition, in particular to promote or enhance immune response, notably cellular immune response. This finds a major application in vaccination, where Suv39h1 can be used in combination with vaccine composition to enhance cellular immunity. The inventors thus provide results that elegantly demonstrate that in a model of Heterosubtypic flu infection, CD8+ T cells show stronger expansion following vaccination when Suv39h1 is inhibited and generate increased numbers of resident memory cells in the lung tissue. This results in better protection in recall infections (such as the flu archetypical infection), suggesting that Suv39h1 inhibition is beneficial for the enhancement of cellular immunity following vaccination strategies.

### DESCRIPTION OF THE FIGURES

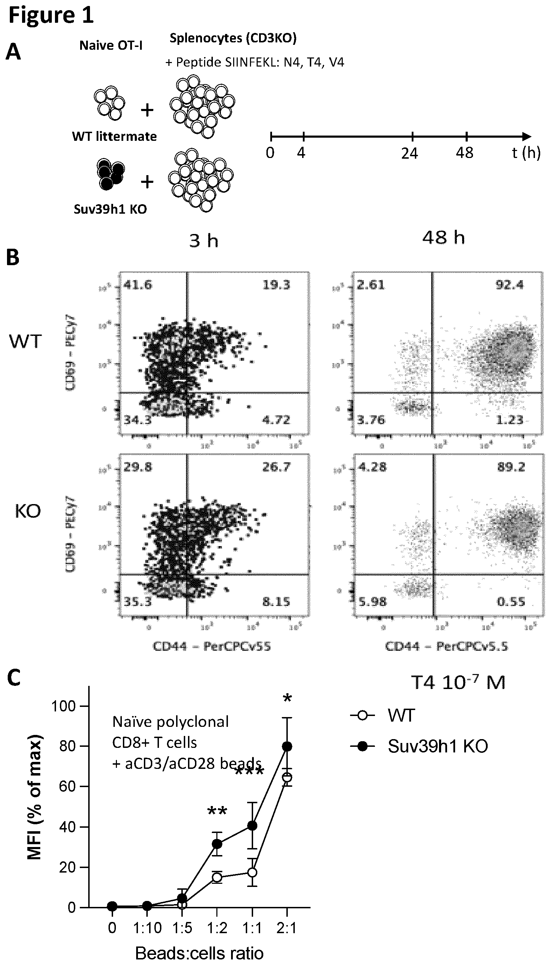

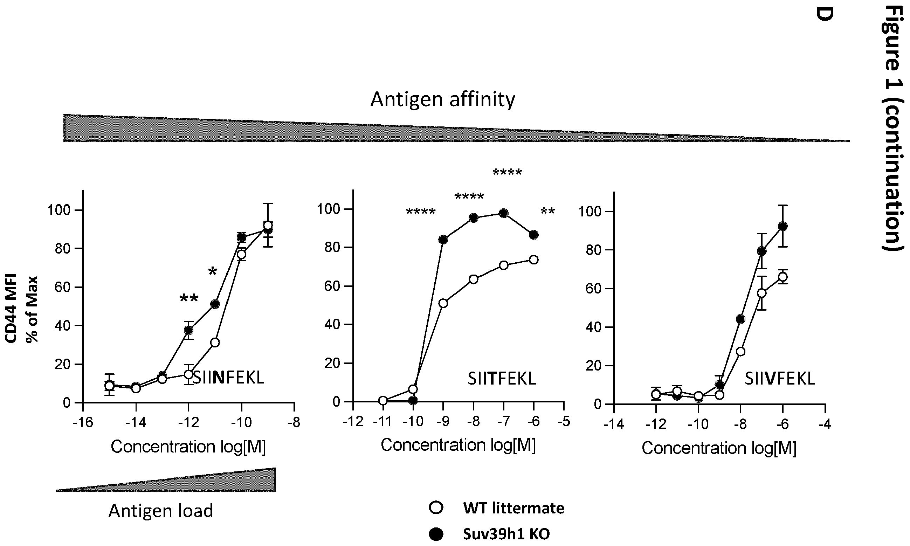

Figure 1. Naive OTI cells (Naive (CD44-) CD8+ T cells) from Suv39h1 -KO mice or WT littermates were incubated for the indicated timepoints with antigen presenting cells loaded with the indicated peptides or with anti-CD3 anti-CD28 beads at the indicated bead:cell ratios. A. Scheme of the experiment. B. Representative dot plots of the expression of CD69 and CD44 in OTI cells incubated with antigen presenting cells loaded with the T4 peptide (10-7 M) during the indicated time points. C. Median Fluorescence Intensity (MFI) of CD44 as a percentage of maximum value in OTI cells incubated for 48h at the indicated bead:cell ratios. D. MFI of CD44 (% of the maximum value) in OTI cells incubated for 48h with the indicated peptides and concentrations. * p<0.05, **p<0.01, ***p<0.001, ****p<0.0001 by two-way ANOVA followed by Šídák's multiple comparison test.

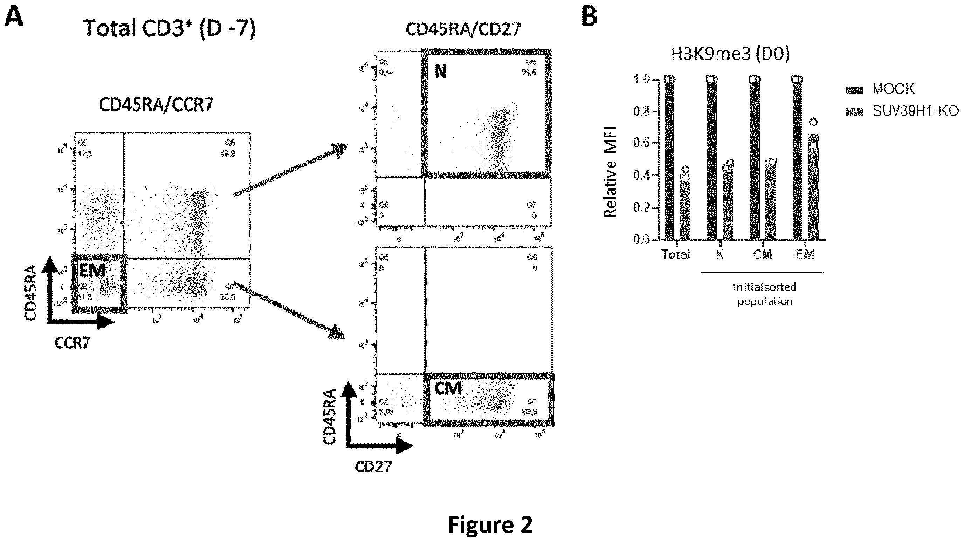

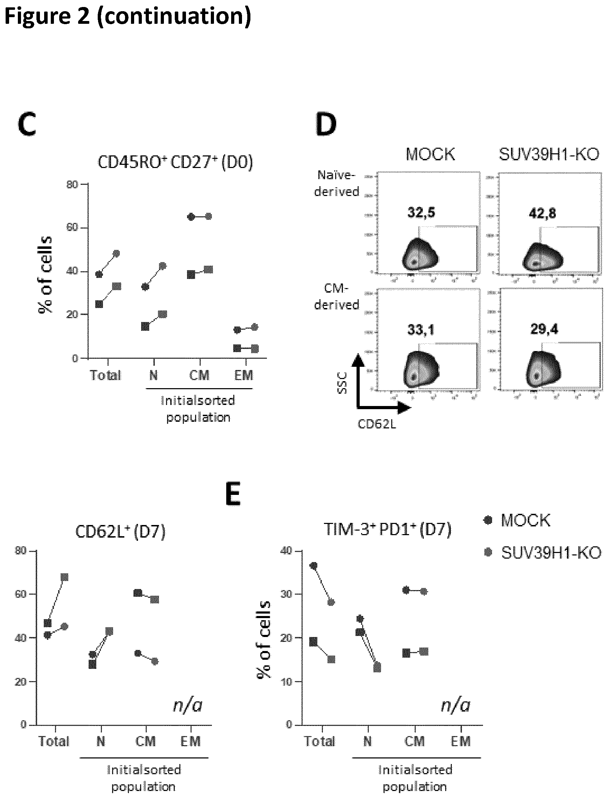

Figure 2. Effect of SUV39H1 inactivation in human naive T cells. A. Sorting strategy for naive (N, CD45RA+CCR7+CD27+), Central Memory (CM, CD45RA-CCR7+CD27+), and Effector Memory (EM, CD45RA-CCR7-) cells from PBMC-purified total CD3+ T cells. B. MFI of H3K9me3 levels measured by flow cytometry and relativized to the equivalent Mock after Crispr inactivation of SUV39H1. Experiment was performed in two independent donors. C-E. Phenotype of mock and SUV39H1-deficient cells derived from different subsets analyzed by flow cytometry. Experiment was performed in two independent donors (shown as circle and square). C. Percentage of CD45RO+CD27+ cells after SUV39H1 inactivation. D. Representative dot-plot of CD62L expression (left) and percentage of CD62L+ cells (right) in mock and SUV39H1-deficient naive-, CM-, or total CD3- derived cells after week-long stimulation. E. Percentage of TIM-3+PD-1+ cells in mock and SUV39H1-deficient naive-, CM-, or total CD3-derived cells after week-long stimulation.

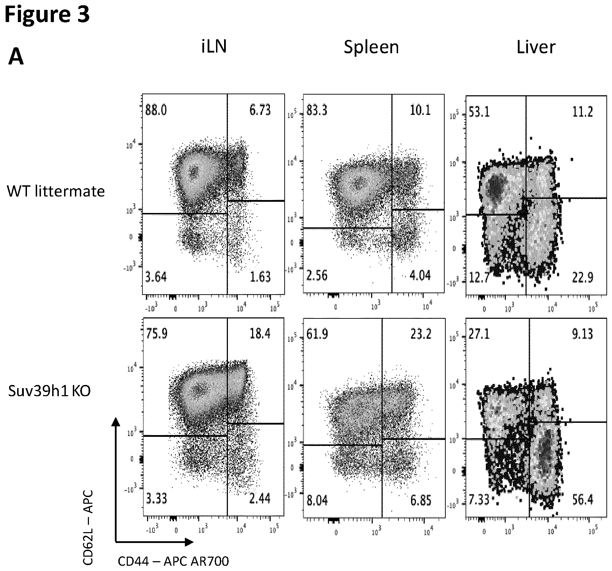

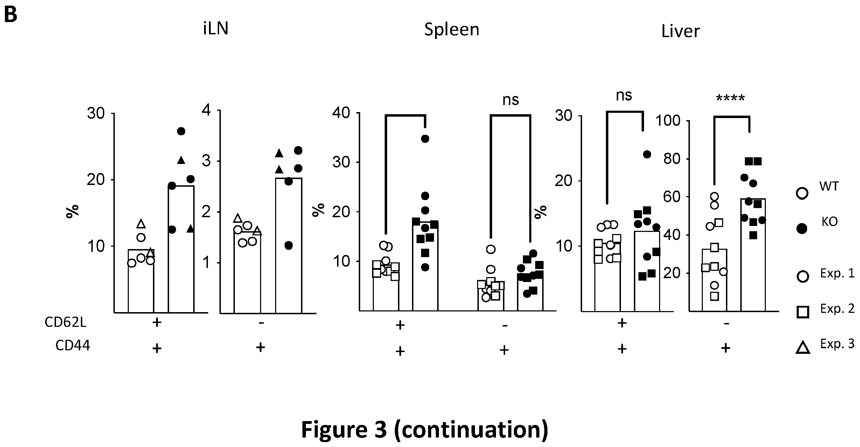

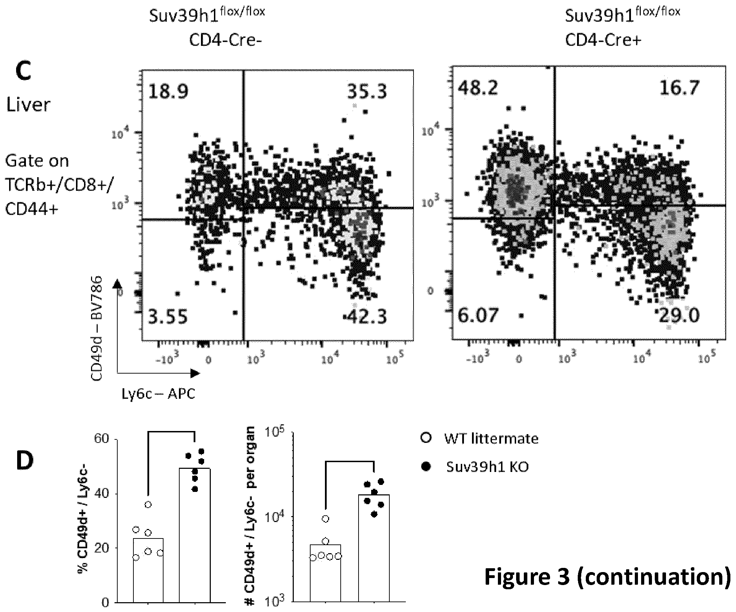

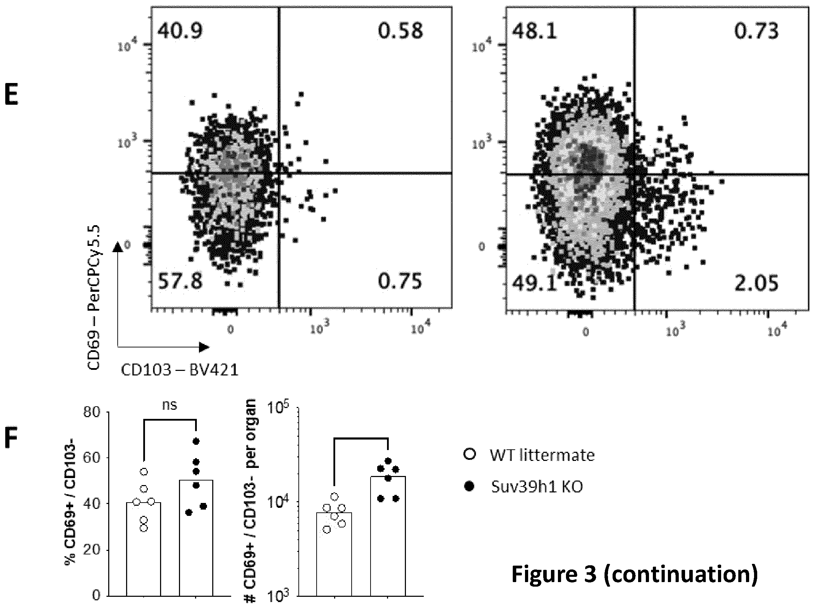

Figure 3. Analysis of the proportions and numbers of central memory (CM, CD62L+CD44+), effector memory (EM, CD62L-CD44+), and tissue resident memory (TRM, CD44+CD69+Ly6c- and CD44+CD69+CD103-) cells at steady state in mice deficient for Suv39h1. A and B. Representative dot plots (A) and quantification (B) of CD44 and CD62L expression in CD8+ T cells from the indicated organs of Suv39h1 KO and WT littermates at steady state. iLN: inguinal Lymph Node. C-F. Representative dot plots (C and E) and quantification (D and F) of the expression of the indicated markers in CD44+ CD8+ T cells from liver of conditional Suv39h1 KO mice (Cre+) or WT littermates (Cre-) at steady state.* p<0.05 ; ** p<0.01 ; *** p< 0.001 ; **** p<0.0001 ; ns: not significant by multiple unpaired t test with Holm-Šídák correction (B) or Mann Whitney test (D and F).

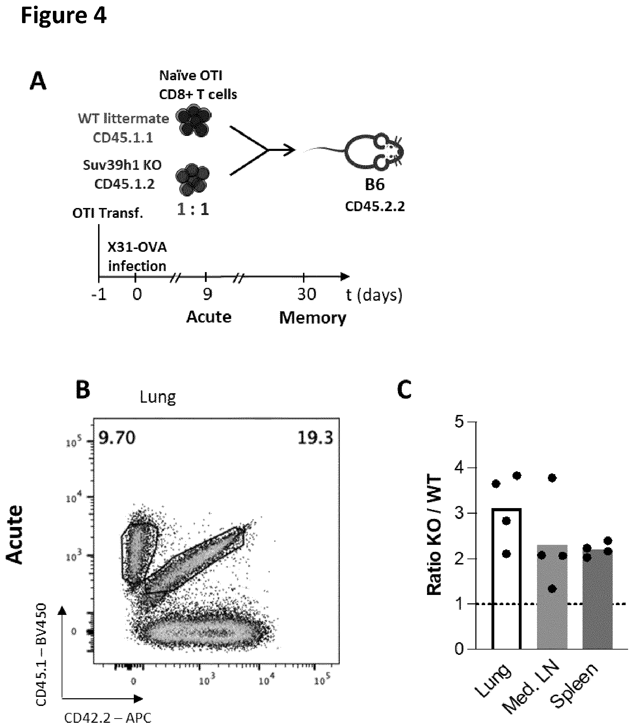

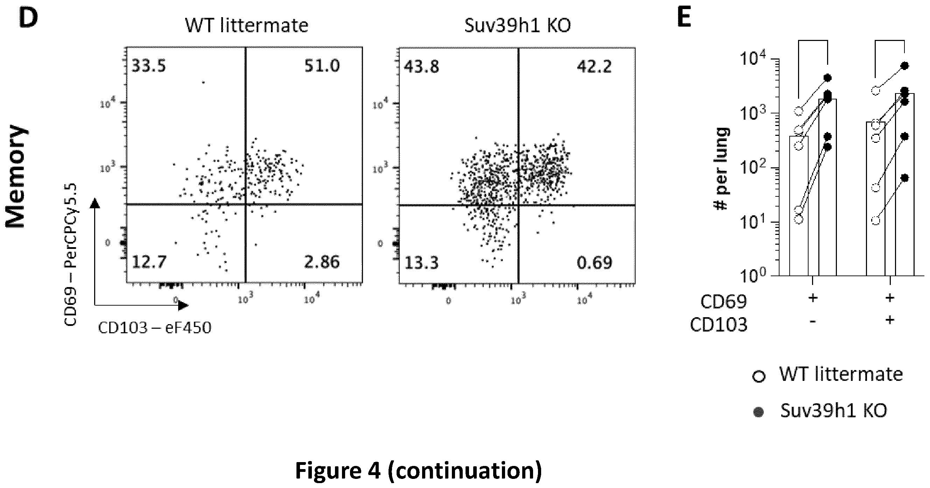

Figure 4. Naive OTI CD8+ T cells from Suv39h1-KO or WT littermate mice were adoptively transferred into B6 mice and 1 day later mice were infected with the flu strain X31 expressing OVA (X31-OVA). A. Scheme of the experiment. B and C. Representative dot plots (B) and quantification (C) of the ratio of OTI cells in the indicated organs 9 days post X31-OVA infection (Acute phase of the infection). D-F. Representative dot plot of CD69 and CD103 expression (D) and cell numbers of CD69+CD103- and CD69+CD103+ cells (E) in WT littermate (D) or Suv39h1-KO cells (E) 28 days post infection in OTI cells in lungs (Memory phase). * p<0.05 ; ** p<0.01 ; *** p< 0.001 ; **** p<0.0001 ; ns: not significant multiple ratio paired t test with Holm- Šídák correction (C) or Mann Whitney test (E).

### DETAILED DESCRIPTION

### Definitions

The terminology used herein is for the purpose of describing particular cases only and is not intended to be limiting. In this application, the use of the singular includes the plural unless specifically stated otherwise. As used herein, the singular forms"a","an" and"the" are intended to include the plural forms as well, unless the context clearly indicates otherwise.

In this application, the use of "or" means "and/or" unless stated otherwise. The terms "and/or" and "any combination thereof" and their grammatical equivalents as used herein, can be used interchangeably. These terms can convey that any combination is specifically contemplated.

The term "about" or "around" can mean within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, within 5-fold, or within 2-fold, of a value. Where particular values are described in the application and claims, unless otherwise stated the term "about" meaning within an acceptable error range for the particular value should be assumed.

As used in this specification and claim(s), the words "comprising,"having","including" or "containing" are inclusive or open-ended and do not exclude additional, unrecited elements or method steps. It is contemplated that any embodiment discussed in this specification can be implemented with respect to any method or composition of the present disclosure, and vice versa. Furthermore, compositions of the present disclosure can be used to achieve methods of the present disclosure.

Reference in the specification to "some embodiments," "an embodiment," "one embodiment" or "other embodiments" means that a particular feature, structure, or characteristic described in connection with the embodiments is included in at least some embodiments, but not necessarily all embodiments, of the present disclosures.

To facilitate an understanding of the present disclosure, a number of terms and phrases are defined below.

Adaptive immune response: The adaptive immune response is typically understood to be an antigen-specific response of the immune system. Antigen specificity allows for the generation of responses that are tailored, for example, to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naive antigen-specific T cells or different immune cells able to induce an antigen-specific immune response by antigen-presenting cells. This occurs in the lymphoid tissues and organs through which naive T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are, typically, responsible for presentation of an antigen to T-cells. Therein, presenting the antigen on MHC molecules leads to activation of T cells, which induces their proliferation and differentiation into armed effector T cells. The most important function of effector T cells is the killing of infected cells by CD8+ cytotoxic T cells and the activation of macrophages by Th1 cells, which together make up cell-mediated immunity, and the activation of B cells by both Th2 and Th1 cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Artificial nucleic acid molecule: An artificial nucleic acid molecule may typically be understood to be a nucleic acid molecule, e.g. a DNA or an RNA, that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides, which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

Cellular immunity/cell-mediated immune response (CMI): Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

« Major Histocompatibility Complex" or "MHC" is a cluster of genes that plays a role in control of the cellular interactions responsible for physiologic immune responses. In humans, the MHC complex is also known as the human leukocyte antigen (HLA) complex. For a detailed description of the MHC and HLA complexes, see, Paul, Fundamental Immunology, 3rd Ed., Raven Press, New York (1993)."Proteins or molecules of the major histocompatibility complex (MHC)","MHC molecules","MHC proteins" or "HLA proteins" are to be understood as meaning proteins capable of binding peptides resulting from the proteolytic cleavage of protein antigens and representing potential lymphocyte epitopes, ( e.g ., T cell epitope and B cell epitope) transporting them to the cell surface and presenting them there to specific cells, in particular cytotoxic T-lymphocytes, T-helper cells, or B cells. The major histocompatibility complex in the genome comprises the genetic region whose gene products expressed on the cell surface are important for binding and presenting endogenous and/or foreign antigens and thus for regulating immunological processes. The major histocompatibility complex is classified into two gene groups coding for different proteins, namely molecules of MHC class I and molecules of MHC class II. The cellular biology and the expression patterns of the two MHC classes are adapted to these different roles.

« Human Leukocyte Antigen" or "HLA" is a human class I or class II Major Histocompatibility Complex (MHC) protein (see, e.g., Stites, et al., Immunology, 8th Ed., Lange Publishing, Los Altos, Calif. (1994).

A "fragment of a sequence" is typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%, preferably at least 30%, more preferably at least 40%, more preferably at least 50%, even more preferably at least 60%, even more preferably at least 70%, and most preferably at least 80% of the total (i.e. full-length) molecule from which the fragment is derived.

Heterologous sequence: Two sequences are typically understood to be "heterologous" if they are not derivable from the same gene. I.e., although heterologous sequences may be derivable from the same organism, they naturally (in nature) do not occur in the same nucleic acid molecule, such as in the same mRNA.

"Polypeptide", "peptide" and their grammatical equivalents as used herein refer to a polymer of amino acid residues. A "mature protein" is a protein which is full-length and which, optionally, includes glycosylation or other modifications typical for the protein in a given cellular environment. Polypeptides and proteins disclosed herein (including functional portions and functional variants thereof) can comprise synthetic amino acids in place of one or more naturally-occurring amino acids.

Immunogen: In the context of the present invention, an immunogen may be typically understood to be a compound or antigen that is able to stimulate an immune response. Preferably, an immunogen is a peptide, polypeptide, or protein. In some preferred embodiments, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition or immunogenic composition: In the context of the invention, an immunostimulatory composition may be typically understood to be a composition containing at least one component which is able to induce an immune response or from which a component, which is able to induce an immune response, is derivable. Such immune response may be preferably an innate immune response or a combination of an adaptive and an innate immune response. For example, an immunostimulatory composition in the context of the invention contains at least a peptide or a nucleic acid molecule encoding thereof, notably at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

An "immunogenic" peptide or an "immunogenic" epitope or "peptide epitope" is a peptide that comprises an allele-specific motif such that the peptide will bind an HLA molecule and induce a cell-mediated or humoral response, for example, cytotoxic T lymphocyte (CTL ( e.g ., CD8+ )), helper T lymphocyte (Th (e.g, CD4+ )) and/or B lymphocyte response. Thus, immunogenic peptides described herein are capable of binding to an appropriate HLA molecule and thereafter inducing a CTL (cytotoxic) response, or a HTL (and humoral) response, to the peptide.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers, which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

According to various embodiments of the present disclosure, a nucleic acid such as RNA encoding a peptide or polypeptide is taken up by or introduced, i.e. transfected or transduced, into a cell which cell may be present in vitro or in a subject, resulting in expression of said peptide or polypeptide. The cell may, e.g., express the encoded peptide or polypeptide intracellularly (e.g. in the cytoplasm and/or in the nucleus), may secrete the encoded peptide or polypeptide, and/or may express it on the surface.

Open reading frame: An open reading frame (ORF) in the context of the invention may typically be a sequence of several nucleotide triplets, which may be translated into a peptide or protein. An open reading frame preferably contains a start codon, i.e. a combination of three subsequent nucleotides coding usually for the amino acid methionine (ATG), at its 5'-end and a subsequent region, which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "(protein) coding sequence" or, preferably, "coding sequence".

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Protein A protein typically comprises one or more peptides or polypeptides. A protein is typically folded into 3-dimensional form, which may be required for the protein to exert its biological function.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. In vivo, transcription of DNA usually results in the so-called premature RNA which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist which may be involved in regulation of transcription and/or translation.

The term "endogenous" refers to any material from or produced inside an organism, cell, tissue or system.

The term "exogenous" refers to any material introduced from or produced outside an organism, cell, tissue or system.

The term "expression cassette" is used in the context of the present invention to refer to a nucleic acid molecule which comprises at least one nucleic acid sequence that is to be expressed, e.g. a nucleic acid encoding an antigen or a part thereof, operably linked to transcription and translation control sequences. Preferably, an expression cassette includes cis-regulating elements for efficient expression of a given gene, such as promoter, initiation-site and/or polyadenylation-site. Preferably, an expression cassette contains all the additional elements required for the expression of the nucleic acid in the cell of a patient. A typical expression cassette thus contains a promoter operatively linked to the nucleic acid sequence to be expressed and signals required for efficient polyadenylation of the transcript, ribosome binding sites, and translation termination. Additional elements of the cassette may include, for example enhancers. An expression cassette preferably also contains a transcription termination region downstream of the structural gene to provide for efficient termination. The termination region may be obtained from the same gene as the promoter sequence or may be obtained from a different gene.

The term "operably linked" as used in the context of the present invention refers to an arrangement of elements, wherein the components so described are configured so as to perform their usual function. A nucleic acid is "operably linked" when it is placed into a functional relationship with another nucleic acid sequence. For example, a promoter is operably linked to one or more transgenes, if it affects the transcription of the one or more transgenes. Further, control elements operably linked to a coding sequence are capable of effecting the expression of the coding sequence. The control elements need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

The term "expression vector" refers to a polynucleotide or a mixture of a polynucleotide and proteins capable of being introduced or of introducing the collection of nucleic acids of the present invention or one nucleic acid that is part of the collection of nucleic acids of the invention into a cell, preferably a mammalian cell. Examples of vectors include but are not limited to plasmids, cosmids, phages, viruses or artificial chromosomes. In particular, a vector is used to transport the promoter and the collection of the nucleic acids or one nucleic acid that is part of the collection of nucleic acids of the invention into a suitable host cell. Expression vectors may contain "replicon" polynucleotide sequences that facilitate the autonomous replication of the expression vector in a host cell. Once in the host cell, the expression vector may replicate independently of or coincidental with the host chromosomal DNA, and several copies of the vector and its inserted DNA can be generated. In case that replication incompetent expression vectors are used - which is often the case for safety reasons - the vector may not replicate but merely direct expression of the nucleic acid. Depending on the type of expression vector the expression vector may be lost from the cell, i.e only transiently expresses the CVP encoded by the nucleic acid or may be stable in the cell. Expression vectors typically contain expression cassettes, i.e. the necessary elements that permit transcription of the nucleic acid into an mRNA molecule.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

The term"neoantigen" or "neoantigenic" means a class of tumor antigens that arises from a tumor-specific mutation(s) which alters the amino acid sequence of genome encoded proteins. Neoantigens encompass, but are not limited to, tumor antigens which arise from, for example, substitution in the protein sequence, frame shift mutation, fusion polypeptide, in-frame deletion, insertion, expression of endogenous retroviral polypeptides, and tumor-specific overexpression of polypeptides.

The terms "(neo)antigen peptide" and "(neo)antigenic peptide", used interchangeably with "peptide" in the present specification, refers to a series of residues, typically L-amino acids, connected one to the other, typically by peptide bonds between the a-amino and carboxyl groups of adjacent amino acids. Similarly, the term "polypeptide" is used interchangeably with"mutant polypeptide", "neoantigen polypeptide" and "neoantigenic polypeptide" in the present specification to designate a series of residues, e.g. , L-amino acids, connected one to the other, typically by peptide bonds between the a-amino and carboxyl groups of adjacent amino acids. The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification does not destroy the biological activity of the polypeptides as herein described. A peptide or polypeptide as used herein comprises at least one flanking sequence. The term "flanking sequence" as used herein refers to a fragment or region of the neoantigen peptide that is not a part of the neoepitope. The term"residue" refers to an amino acid residue or amino acid mimetic residue incorporated into a peptide or protein by an amide bond or amide bond mimetic, or nucleic acid (DNA or RNA) that encodes the amino acid or amino acid mimetic.

By "neoplasia", "neoplastic disease", "cancer" or "tumor" it is meant any disease that is caused by or results in inappropriately high levels of cell division, inappropriately low levels of apoptosis, or both. Examples of cancers include, without limitation, leukemia (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (e.g., Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, and retinoblastoma). Lymphoproliferative disorders are also considered to be proliferative diseases.

The term "neoplasia vaccine" or "tumor vaccine", or "cancer vaccine" is typically meant to refer to a pooled sample of neoplasia/tumor antigens, for example at least two, at least three, at least four, at least five, or more antigenic peptides. A "vaccine" is to be understood as meaning a composition for generating immunity for the prophylaxis and/or treatment of diseases (e.g., neoplasia/tumor or infectious disease). Accordingly, vaccines are medicaments which comprise antigens and are intended to be used in humans or animals for generating specific defense and protective substance by vaccination. A "vaccine composition" or a "neoplasia vaccine composition" can include a pharmaceutically acceptable excipient, earner or diluent.

The term "combination" embraces the administration of an KMT inhibitor, notably a suv39h1 inhibitor and one or additional component that elicits an immune response, typically a vaccine or immunogenic composition (e.g. a pooled sample of neoplasia/tumor specific antigens or infectious disease-associated antigens), as part of a treatment regimen intended to provide a beneficial (additive or synergistic) effect from the co-action of one or more of these therapeutic agents. The combination may also include one or more additional agents, for example, but not limited to, such as a checkpoint inhibitor, immune cell therapy, chemotherapeutic agents, anti-angiogenesis agents and agents that reduce immune-suppression. The beneficial effect of the combination includes, but is not limited to, an improved or enhanced immunogenic response resulting from the combination of therapeutic agents. Administration of these therapeutic agents or components in combination typically is carried out over a defined time period (for example, minutes, hours, days, or weeks depending upon the combination selected).

"Combination therapy" is intended to embrace administration of these therapeutic agents or components in a sequential manner, that is, wherein each therapeutic agent is administered at a different time, as well as administration of these therapeutic agents, or at least two of the therapeutic agents, in a substantially simultaneous manner. As another example, a combination of the present invention may be formulated as separate pharmaceutical compositions that can be administered at the same or different time. As used herein, the term "simultaneously" is meant to refer to administration of one or more agents at the same time. Simultaneously includes administration contemporaneously, that is during the same period of time. In certain embodiments, the one or more agents or components are administered simultaneously in the same hour, or simultaneously in the same day. Sequential or substantially simultaneous administration of each therapeutic agent can be effected by any appropriate route including, but not limited to, oral routes, intravenous routes, subcutaneous routes, intramuscular routes, direct absorption through mucous membrane tissues (e.g., nasal, mouth, vaginal, and rectal), and ocular routes (e.g., intravitreal, intraocular, etc.). The therapeutic agents can be administered by the same route or by different routes. For example, one component of a particular combination may be administered by intravenous injection while the other component(s) of the combination may be administered orally. The components may be administered in any therapeutically effective sequence. The expression "combination" embraces groups of compounds or non -drug therapies useful as part of a combination therapy.

The term "pharmaceutically acceptable" refers to approved or approvable by a regulatory agency of the Federal or a state government or listed in the U.S. Pharmacopeia or other generally recognized pharmacopeia for use in animals, including humans.

A "pharmaceutically acceptable excipient, carrier or diluent" refers to an excipient, carrier or diluent that can be administered to a subject, together with an agent, and which does not destroy the pharmacological activity thereof and is nontoxic when administered in doses sufficient to deliver a therapeutic amount of the agent.

As used herein, the terms "prevent," "preventing," "prevention," "prophylactic treatment," and the like, refer to reducing the probability of developing a disease or condition in a subject, who does not have, but is at risk of or susceptible to developing a disease or condition.

The term "prime/ boost" or "prime/ boost dosing regimen" is meant to refer to the successive administrations of a vaccine or immunogenic or immunological compositions. The priming administration (priming) is the administration of a first vaccine or immunogenic or immunological composition type and may comprise one, two or more administrations. The boost administration is the second administration of a vaccine or immunogenic or immunological composition type and may comprise one, two or more administrations, and, for instance, may comprise or consist essentially of annual administrations. In certain embodiments, administration of the neoplasia vaccine or immunogenic composition is in a prime/ boost dosing regimen.

The term "subject" or "individual" refers to an animal which is the object of treatment, observation, or experiment. By way of example only, a subject includes, but is not limited to, a mammal, including, but not limited to, a human or a non-human mammal, such as a non-human primate, bovine, equine, canine, ovine, or feline.

The terms "treat," "treated," "treating," "treatment," and the like are meant to refer to reducing or ameliorating a disorder and/or symptoms associated therewith (e.g., a neoplasia or tumor). 'Treating" may refer to administration of the combination therapy to a subject after the onset, or suspected onset, of a cancer. "Treating" includes the concepts of "alleviating", which refers to lessening the frequency of occurrence or recurrence, or the severity, of any symptoms or other ill effects related to a cancer and/or the side effects associated with cancer therapy. The term "treating" also encompasses the concept of "managing" which refers to reducing the severity of a particular disease or disorder in a patient or delaying its recurrence, e.g., lengthening the period of remission in a patient who had suffered from the disease. It is appreciated that, although not precluded, treating a disorder or condition does not require that the disorder, condition, or symptoms associated therewith be completely eliminated.

The term "therapeutic effect" refers to some extent of relief of one or more of the symptoms of a disorder (e.g., a neoplasia or tumor) or its associated pathology. "Therapeutically effective amount" as used herein refers to an amount of an agent which is effective, upon single or multiple dose administration to the cell or subject, in prolonging the survivability of the patient with such a disorder, reducing one or more signs or symptoms of the disorder, preventing or delaying, and the like beyond that expected in the absence of such treatment. 'Therapeutically effective amount" is intended to qualify the amount required to achieve a therapeutic effect. A physician or veterinarian having ordinary skill in the art can readily determine and prescribe the "therapeutically effective amount" (e.g., ED50) of the pharmaceutical composition required. For example, the physician or veterinarian could start doses of the compounds of the invention employed in a pharmaceutical composition at levels lower than that required in order to achieve the desired therapeutic effect and gradually increase the dosage until the desired effect is achieved.

The immune cells according to the disclosure are typically mammalian cells, e.g., human cells. More particularly, the cells of the disclosure are derived from the blood, bone marrow, lymph, or lymphoid organs (notably the thymus) and are cells of the immune system (i.e., immune cells), such as cells of the innate or adaptive immunity, e.g., myeloid or lymphoid cells, including lymphocytes, typically T cells and/or NK cells. Preferably according to the disclosure, cells are notably lymphocytes including T cells, B cells, NK cells and progenitors thereof. In some embodiments T cells are generated from induced pluripotent stems cells.

Cells according to the disclosure may also be immune cell progenitors, such as lymphoid progenitors and more preferably T cell progenitors. Examples of T-cell progenitors include pluripotent stem cells (PSCs), induced pluripotent stem cells (iPSCs), hematopoietic stem cells (HSCs), human embryonic stem cells (ESCs), adipocyte-derived stem cells (ADSCs), multipotent progenitor (MPP); lymphoid-primed multipotent progenitor (LMPP); common lymphoid progenitor (CLP); lymphoid progenitor (LP); thymus settling progenitor (TSP); or early thymic progenitor (ETP). Hematopoietic stem and progenitor cells can be obtained, for example, from cord blood, or from peripheral blood, e.g. peripheral blood-derived CD34+ cells after mobilization treatment with granulocyte-colony stimulating factor (G-CSF).

T cell progenitors typically express a set of consensus markers including CD44, CD117, CD135, and/or Sca-1 but see also Petrie HT, Kincade PW. Many roads, one destination for T cell progenitors. The Journal of Experimental Medicine. 2005;202(1):11-13. The cells typically are primary cells, such as those isolated directly from a subject and/or isolated from a subject and frozen. With reference to the subject to be treated, the cells of the disclosure may be allogeneic and/or autologous.

### histone lysine methyltransferase (KMT) and inhibitor's thereof

Heterochromatin Protein 1 (HP1) is a transcriptional repressor that directly binds to the methylated lysine 9 residue of histone H3 (H3K9me), which is a hallmark histone modification for transcriptionally silenced heterochromatin. The accumulation of histone H3 lysine 9 methylation (H3K9me) is thus an important mark of the heterochromatin state. HP1 proteins are characterized by two conserved domains: the chromodomain (CD) at the N-terminus, and the chromo shadow domain (CSD) at the C-terminus.The CD was shown to directly bind to H3K9me, while the CSD is implicated in various protein:protein interactions and dimerization. H3K9me3-mediated heterochromatin formation thus involves HP1 that contains a chromodomain that recognizes and binds to di- and tri-methylated H3K9. The SUV39 PKMTs and their function in heterochromatin formation are evolutionarily conserved and orthologous proteins can be detected in most organisms from fission yeast to humans including plants (see Zeng W, Ball AR Jr, Yokomori K. HP1: heterochromatin binding proteins working the genome. Epigenetics. 2010;5(4):287-292).

H3K9me in mammals is effected by multiple histone methyltransferases, which have distinct targeting specificities and degrees of methylation (e.g., mono-, di- or tri-methylation). A common feature HKMTs is the fact that they all interact with both maintenance and de novo DNA methyltrans- ferases or DNMTs.

Many H3K9-specific histone methyltransferases (HKMTs) have been characterized, all of which contain a conserved SET domain (Kouzarides, 2007) and are thus members of the SET family of histone methyltransferases (HMTs) (Cheng, X.; Collins, R.E.; Zhang, X. Structural and Sequence Motifs of Protein (Histone) Methylation Enzymes. Annu. Rev. Biophys. Biomol. Struct. 2005, 34, 267-294 ; Dillon, S.C.; Zhang, X.; Trievel, R.C.; Cheng, X. The SET-domain protein superfamily: Protein lysine methyltransferases. Genome Biol. 2005, 6, 227).

Examples of reviews and relevant articles include Weirich S, Khella MS, Jeltsch A. Structure, Activity and Function of the Suv39h1 and Suv39h2 Protein Lysine Methyltransferases. Life (Basel). 2021;11(7):703 ; Fritsch L, Robin P, Mathieu JR, et al. A subset of the histone H3 lysine 9 methyltransferases Suv39h1, G9a, GLP, and SETDB1 participate in a multimeric complex. Mol Cell. 2010;37(1):46-56 ; Shinkai Y, Tachibana M. H3K9 methyltransferase G9a and the related molecule GLP. Genes Dev. 2011;25(8):781-788. Padeken J, Methot SP, Gasser SM. Establishment of H3K9-methylated heterochromatin and its functions in tissue differentiation and maintenance. Nat Rev Mol Cell Biol. 2022;23(9):623-640).

Suv39h1 and Suv39h2 consist of two-conserved domains, one SET- and one chromodomain. The amino acid sequences of Suv39h1 and Suv39h2 are highly conserved, with 56% amino acid identity over the entire protein alignment. The SET (Su(var)3-9, Enhancer-of-zeste, Trithorax) domain is the catalytic domain of one large group of PKMTs, called SET-domain PKMTs. The structure of the Suv39h2 SET domain shows a high similarity to the known SET domain structures of other H3K9 PKMTs such as Dim-5 or G9a. This domain binds the methyl group donor S-adenosyl-L-methionine (AdoMet) and brings it in close contact to the target lysine residue in its active site pocket. Chromodomains are methylated lysine binding modules (Taverna, S.D.; Li, H.; Ruthenburg, A.; Allis, C.D.; Patel, D.J. How chromatin-binding modules interpret histone modifications: Lessons from professional pocket pickers. Nat. Struct. Mol. Biol. 2007, 14, 1025-1040; Patel, D.J.; Wang, Z. Readout of Epigenetic Modifications. Annu. Rev. Biochem. 2013, 82, 81-118). The Suv39h1 chromodomain was shown to recognize H3K9me2/3 (Wang, T.; Xu, C.; Liu, Y.; Fan, K.; Li, Z.; Sun, X.; Ouyang, H.; Zhang, X.; Zhang, J.; Li, Y.; et al. Crystal Structure of the Human Suv39h1 Chromodomain and Its Recognition of Histone H3K9me2/3. PLoS ONE 2012, 7, e52977).

The trimethylases Suv39h1 and 2/KMT1A and -1B, which contribute mainly to the establishment of pericentric heterochromatin (Peters et al., 2003; Rea et al., 2000). At the enzymatic level, Suv39h prefers mono- or dimethylated H3K9 as a primary substrate In agreement with a role in gene silencing, Suv39h1 has also been found to interact with the de novo DNA methyltransferase DNMT3B.

Another important family of H3K9 HKMTs comprises G9a/ Eu-HMTase2/KMT1C and GLP/Eu-HMTase1/KMT1D. G9a catalyzes the initial mono-methylation of H3K9. Human G9A is referenced as Q96KQ7 in UNIPROT and is encoded by the EHMT2gene located on chromosome 6 (p21.33) (gene ID: 10919 in NCBI). Human KMT1D is referenced as Q9H9B1 in UNIPROT and is encoded by the EHMT12 gene located on chromosome 9 (q34.3) (gene ID: 79813, in NCBI).

H3K9 HKMTfamily also includes SETDB1/ESET/KMT1E. SETDB1 alone dimethylates H3K9 but trimethylates this residue when associated with its cofactor hAM. Human Setdb1 is referenced as Q15047 in UNIPROT and is encoded by the Suv39h2 gene located on chromosome 1 (q21.3) (gene ID: 9869 in NCBI).

In some embodiments of the present invention, a H3K9me histone methyltransferase (also shortly named herein H3K9me HKMT) inhibitor can be selected among inhibitors of Suv39h1 (KMT1A), Suv39h2 (KMT1B), G9a (EHMT2/KMT1C), G9a-like protein 1; GLP (EHMT1/KMT1D), Setdb1 (KMT1E), and Setdb2 (KMT1F).

In some embodiments, the selected inhibitor is selective for one or more HKMT as above mentioned. By "selective" it is meant that the affinity of the inhibitor is at least 10-fold, preferably 25-fold, more preferably 100-fold, and still preferably 500-fold higher than the affinity for other histone methyltransferases and in some cases for one or more of the H3K9me HKMTs. Typically, the selected inhibitor has an IC50 of less than 20 µM, preferably less than 10 µM, more preferably less than 5 µM, even more preferably less than 1 µM or less than 0.5 µM. In some embodiments, the HKMT inhibitor has an IC50 for the other HKMTs, in in some cases for H3K9me HKMT, such as for example EZH2, G9A and/or Setbd1, of more than 10 µM, preferably more than 20 µM, more preferably more than 50 µM. In some embodiments, the selected inhibitor is selective for Suv39h1 and/or Suv39h1. In some embodiments, the selected inhibitor does not inhibit Setdb1.

In some embodiments, a selected HKMT inhibitor alternatively targets one or more heterochromatin protein 1 (HP1) proteins (see for review Zeng W, Ball AR Jr, Yokomori K. HP1: heterochromatin binding proteins working the genome. Epigenetics. 2010;5(4):287-292). Typically, HP1 proteins include HP1α, encoded by Chromobox homolog 5 (*CBX*5); HP1β, encoded by *CBX1,* and HP1γ, encoded by *CBX3.*

Human CBX1 is referenced as P83916 in UNIPROT and is encoded by the CBX1 gene located on chromosome 17q (21.32) (gene ID: 10951 in NCBI). This gene encodes a highly conserved nonhistone protein, which is a member of the heterochromatin protein family . The protein is enriched in the heterochromatin and associated with centromeres. The protein has a single N-terminal chromodomain which can bind to histone proteins via methylated lysine residues, and a C-terminal chromo shadow-domain (CSD) which is responsible for the homodimerization and interaction with a number of chromatin-associated nonhistone proteins.

Human CBX3 (also named HP1γ) is referenced as Q13185 in UNIPROT and is encoded by the CBX1 gene located on chromosome 7 (p15.2) (gene ID: 11335 in NCBI).

Human CBX5 (also named HP1α) is referenced as P5973 in UNIPROT and is encoded by the CBX5 gene located on chromosome 12 (q13.13) (gene ID: 23468 in NCBI).

Inhibitors of HP1 proteins may prevent binding of the protein to histone H3 trimethylated and or inhibit the gene expression (at the genomic and/or transcriptomic level as detailed below).

Inhibitors of Suv39h1 will be described in detail below, but it is clearly intended that the definitions below can be applied by the skilled person to any the H3K9 HKMT inhibitors (including inhibitors targeting HP1 protein) as previously described.

### Inhibitors of Suv39h1

Human Suv39h1 methyltransferase is referenced as O43463 in UNIPROT and is encoded by the Suv39h1 gene located on chromosome X (gene ID: 6839 in NCBI). One exemplary human gene sequence is SEQ ID NO: 1

GCTCTTCTCGCGAGGCCGGCTAGGCCCGAATGTCGTTAGCCGTGGGAAAGATGGCGGAAAATTTAAAAG
GTGAAGCAGTGGAGGCAGAGGCGCGGGCCCGCTGGCCGGGCCGGGTGGGCTGGAGGGGTGGCGTTGCCGG
CTCGGGGCCCCGGCGTCCGAGGCCCCGGCGCCGGGAGCCGGAAGCTCGGCGCGGAAAGGATGAGGCTCGG
GGAGCGCGGGCCTGGTGCGCGGGGCTGGGCGGGGCTCAGGGGAGGGGGCGCGCGCCTCGGAACGCAGCGG
GCCTGGGGCTAAGTGGGGACGCAGGAGGGGGGCGCGCGCTGCAGATCGCGGCCGGACCGGCGCGCGCACG
CGCGGGGGCTTGTCGAGGAGGCCGCGCCCGCGCGCGGGAAAATCGGCCCCTGGTGACCGTTACCCATTCG
GTGTCCACGCGAGACGCCGGAAGGGGAGGGAAGATAAGGGGGACAGACCCCCCCTTCAGAGTGCGGGGAC
TGAGAACTCTTCTGGGGGAAAGTATCGCCTCTCGGCGTGATGGGGGAAGGGTTTGGAATAGAGGATCTCC
CAGAAGAGAGAAATGGGGCCTTCCTAATAGGACAGCTGGACTCCCCCATGTGCAGGGAAAGAGGGGTGAC
TCAGTTATGTTGGGGGGCCCCTCTATGTGACGAAAGCCGCAGGGAGGATGGGTTCCACAAGTGGGAGGAC
CTCTGAGGGGTTCAGGAGAGTCCACCTGTGGCCAGTTCCGGAGCCAGCAGCTGGAGGGTGTTAATTGGGA
CCACTTAAGTGGGGTTTCAAATGCAAGTGAAGGAAGGGGTTTGCGAGGACAGGCTGTAGGGAGAGGAAAG
CGTGGGCACAGGCGGGCTCAGTCTCCACTTGGTCTTGCCTAAGTAGGGGGGGTAGGGCTTGCACACGAAA
TAATTTTGAAAAGCTCTTGTACGTTTTTAGGTTGACATCTAAAGTTTAGGTGTGATTTTTGCAATGGGAG
GCAGAAAATATTAAACATTTTAAAAAGATGATATAGGATGAGGACTGACTGCCCTTGGAATGTTTTGAGA
CATGATTTGATAAAATGGCTTCTAAAGTACTTTGATTAAGAATGTGCCTTGAATCAGAAAGAAATCTGTT
TTTTAAAATCCGTGTGTCTTACAAAATTTTGGCAGTCCCAGAAATATACCAGGTATATTTAAAAGAATCA
GTGTCACTAATCTGTACCGCTTTGCGTATAAATTCAGAGCATAACCCTTTGGATGAGCTTTACTCTTCAC
AGAAAGATGGATGAGACAGAAAGTCAAAATGCAGTGTGAAAAAATATACCTTGTGGATTTAAGGCACCCA
GATTGATCATTTAAGGAGCCTTCCTGTAATGTAGTATCATTCCACAATCTCTTGCTCATAATTCCGAAAT
CTAAAAAGCCCTGAAAACTGAAATCCTTTTCTTAAATTTGGCACCATTTATGGCAAAAGTAGATTATAAA
GTGGAACTCTTCTGTGAGGCTATTTTATATCTCTATTTCTCCTATTTAATGTGGGAAATTCATACTTTTG
CTGCAGGACGGTTAATGCATTTGATTATAGCTTGATGTCCCCTGGCCACGCTGGGGGTGTTAGGTGATGT
ATGGGATATCCTAGAACTAGTCTTGTAAAATCCAAAAAATTTTGAATTCCAAAACACATCTGGCCCAGGA
GTTTGAGATAAGGGATTAAAGACCAGTCTTTCCAGTTACGCCTTTGTTTGGTGCCCTGGAGGTTTTAATC
TTGTCCTCCCATCCGCCCCACCTCGGGGCACTGCATCCTACTGAGATGGGGGGTTGTTCTCTTTCTTTTG
CAAAGTGGAGAGAGGGGTGATCAGGTCCTGAACCCAAAGCAGGTTTGCAGGCAGTTTTAGGAATGACATG
GAGTTTGAAGCTCTGGAAATAGATTCCTTTAGGACAATTCATGTTGCAAACCCCGCTTAGAGGTCTGCTG
CTCCAGGGGCAGCTTTGGGCAGTGAGTATTGGGGTTCAAAGCACATTTCTGTTGTAGAACAGTCTCAGCA
CTGGGGAAGGCCTGTGGATTAAATTTCCCTGACTCTTAGTCAGTCTTTGGGATGTCCCAGGGAGGATTTG
GGGTCCCCTTTGACTCCTGGCCTAGTCAGGCTGCCCAGTAATAGTTCTTTCTGCCCCGCTTGATCCAGGC
TGCAGCGTGTGTTGCAAGTCTTCTTGGAATCAGCTGCAGGACCTGTGCCGCCTGGCCAAGCTCTCCTGCC
CTGCCCTCGGTATCTCTAAGAGGAACCTCTATGACTTTGAAGTCGAGTACCTGTGCGATTACAAGAAGAT
CCGCGTGAGTCTGGGGTGACCATGCTCTGGGAGGGGACAGGTCACAAGGAAGTTAGTGTCCTGCCCTCTT
GTCCCCAACTGCCCACATCTGTTTCCAAAGGAATTTTCTCCTTTGACCCTGAAAACACAGGATGAATAG
ACCCTTTTATCCCTATGTTACAGATAGGGAAACTAAGTAGCTTTCTGGGCAGGAGCCGGAATGAATTCCC
AAATAAATCTCTTCCCATCTCCCTAGGAATACAAAGTAATTGCAGAGATTGAGATGCTGAACCCTGGAGT
GGAAATGTCAATAGCTAATGGCTATTGAGCACTTTCAGTGTGCCAGGCGTTTCCTCTCGGAAACTCATTT
AATCCCCTAACAACTGAAATGGGGATAGTTATTGCCAGAAGGGGGAGTGGCAGGATGGTTAGGAGAACAA
ACTGGAGTCAGCATGCTTGGGTTCCAGTCCTGGTTGTGTTACCCCAGGCTACCTACCCAAACCTTCAGTA
CTTCATTTTCCTTGTCGTAAAAGAGGGATAACTAGGAGCATCTACCTCATGTGGGGTTGTGAGGATTAAG
TTAGTTAATAATACATGTAAGCTGCCTAGAACAGGCAAGGACTTGTCAGGGTCCAGGTGAGCCAGGGTGG
TGGCAGTGATGATGGTTTGTCACTGGACGTATTTTGAAGGTGGGGCCAACGGGATGTGCAGAGGGTGGGT
GTCGGAAGTGTGAACAAAAGAGAAGTCAAGGATGACTCCAGGAGTTCCGGTCTGAACAGCTAGAAGGGAG
GGAGCTGATGATGGGGAAGACCAAGGAGAGGAGTGGGTTTGGGAGATTAATTAAAAGTTGGAACAGTTTG
GACAGACACAGTAAGGGGGAGAGGAGGATTTGAACCTAGTAATCCTCACTCTAGAGCCCATGCTTTTTAATC
ACCGCATGAAGTCATTCCAGAAAAGGGGTGGGGTGGGGTAGGTGTTATCCATCAGCTTTGGGATGGAAGC
CCCATGAAGGCTGGCCCAAAGATCTCACTACTTACGGTACCCCCGTCCCCCTACCCACCCCCAACAGGAA

CAGGAATATTACCTGGTGAAATGGCGTGGATATCCAGACTCAGAGAGCACCTGGGAGCCACGGCAGAATC
TCAAGTGTGTGCGTATCCTCAAGCAGTTCCACAAGGACTTAGAAAGGGAGCTGCTCCGGCGGCACCACCG
GTCAAAGACCCCCCGGCACCTGGACCCAAGCTTGGCCAACTACCTGGTGCAGAAGGCCAAGCAGAGGCGG
GCGCTCCGTCGCTGGGAGCAGGAGCTCAATGCCAAGCGCAGCCATCTGGGACGCATCACTGTAGAGAATG
AGGTGGACCTGGACGGCCCTCCGCGGGCCTTCGTGTACATCAATGAGTACCGTGTTGGTGAGGGCATCAC
CCTCAACCAGGTGGCTGTGGGCTGCGAGTGCCAGGACTGTCTGTGGGCACCCACTGGAGGCTGCTGCCCG
GGGGCGTCACTGCACAAGTTTGCCTACAATGACCAGGGCCAGGTGCGGCTTCGAGCCGGGCTGCCCATCT
ACGAGTGCAACTCCCGCTGCCGCTGCGGCTATGACTGCCCAAATCGTGTGGTACAGAAGGGTATCCGATA
TGACCTCTGCATCTTCCGCACGGATGATGGGCGTGGCTGGGGCGTCCGCACCCTGGAGAAGATTCGCAAG
AACAGCTTCGTCATGGAGTACGTGGGAGAGGTAGGGAGATGGGACCCGGTGTGTGTGTGCAGCTCTTCCC
ACCCTGTGTTCACAGTGTGCACCCGGCTCTCCTCACTGTGTGTCTGAAACTCCCTGCTTTCCCTATGGGA
AAGGCCTGGGAGACTGATCTGAGTGTCACAGGGACATTGGGCAGGGGCTAGTATTCCAAGCTGGTACACA
CCAGCATGGCCATGCTGGTGGTTACAGGAACACCAAAATACTTCTGCACTGGGTTGACTTTATCAGGGTA
CTTAGCAAAGAAGAAAAGTGGGAGCTGAGATCCAGGCAGCCTTAGCATTCCTTGTAACAGGCCATGTGGT
CTGGCATAGGGCTGCATCAGCTGGAAAGGCACTTCCTCTTCTCATTTCACAAGGGCCCATCTTATCACCA
AGCAGTATCTGCCTGAGGAGCATTGTTTTACCAGTCTGCACAGAAGGAACGCAGTCTGGAGTAGGATGAG
ACATGCCTTGGACAAAGGTTGGCTAACTCCTTTAGACTTTCAGGACACTGGGGAAGGCCGAGTGTATTAG
GCAAGCCAGCCATTGTAAAAACAACCCCCTCATCTTAGTGACTTTGCACATTACAAGTTTGCTTGCTCAT
GGAACAGTCCATTGTGGGGGTTTGAGTGGGTATCCTTTCACACAGCAACTGGGACACCTAGGCTCTTTCC
CTCCTGTGCCTTTGCTGTGCCTTGAAGCCTTGGAGTCACTACCAGATCCTCAGCATTGCCTGGCAGGCGG
GCCAAGTGGGGGATAGAAGGGGCCCATCCTAGTGGGCCAGGCCTAGATGCAGCACCCATCACTTCTGCCC
ATGCTCCACTGGCTGGAACTGAGTCACTGGCCTTACCTCCATGCAAGAGAGGCAACAGGATGTGCTTTAG
CCATGTGCCCAGCTGCACAAGGCTAAAGCACAGCCATAACTAGCAACTAGATAACTAGCAAGTATCTACA
ACAGAAAAATGTCGCATTCTAAGATGGGATGCAGTTAGCATTTGGGATGAGATGATTTTTCGTTATGTAG
AACTGTCTCATGCATTGGGGGACATTTAGCATCCCTGGACCTTGACCATTTAAATCCATGAGTGTGCCCT
GTCATAGTGACAACCCCAGATACCTCTATATATAGCCATAGGTCCCTTAGGGGTGTGATATAGTTGGAAA
GTGACAGAAACTGGCCTGAGGAACACTCACATGAGGGTGTCCACACTGTAGCACTGATACTGATGACAAG
GAGAGGCTCCTACTCTGAGAAACTGTACAGAGGGTCACATCTGACAGGGACAGAGACAATCTTGGGTAGA
ACAATCTACACCACAGAGGACCCTGACAGTCTGGGCAAGGAGACAGCCACACCCTGTCCAGATAACCCTG
CTGGCCTGCTGGAGGAGATGTCTACATGCCACAGGACCTGGCCATCTGGAAGGAGGAGGTGGGGAGGTGT
CCACACCCTGAGGGCTCCTGACAGTGGGCGGGGGAAGGAGTCCTGATAATCTGCAGGAAGTCCATGTCCC
ACAGTGACCCCAGTGACCTGGAGGAAGAGCTTGTGCTCTCCACACTCTAGAGGACCTGGTGGTCTGACTT
AGGAAGTGTTCACACCCATGTTGTCATGACAGGAAAAAGAAGTGTCCTCACCCCAGAGGGATCCGGACAG
CCTGGAGATCTAGTCTACACCCAAGGGCACCCTGACAGTATCGGGGGAAGTGTCCATGCCCTCAATGTCC
TGACGCTCTGGAGGAAGTGTCCAGAGGTTGAAGGAACCCAAACTCTCTGGAGGAGATGTCCAGACCCCAT
AGGACCCAGACAGTCCACCGTCCTAGGGGCTGCACAGAGCAGGAAGTATCTCAGCCTCACTGTACTGGCA
TCTGGAGGAAACACTGTCTACCACACAGGGTCCTGACAGTCGAGGAGGAAGACTACACCCCAAGGGCAGC
CTCAGACCCTCCTGGGCCTGAGACTTTTCTGGCTGCTCTTCCTTAGTGTCAGTGTCTTTAATCCTCCCAG
GGATCCTCTCAGCCCTGTCCTCACCCTTCCTGCTCTCCACACATTCTTGCTGCACTATTTCCTGACCGCC
CTCCAGCACCCTCAGACCCATGGGTCCTCCTGATGGTGCCACAGGCCTCCGGAAAGAGCCAAACTCATCT
TCCCGCATTCCCCTCTGAAGAGACAGTATAGCAAATGGTTAGGAGGGTGGACTCTGGATCCTGACTGCCC
GAGGCTGAATCCCAGCTCCACTACTCATCAGCAATCACTAACCTCTGTGCCTGCATTTACTATGTAAAAT
GGGAACAATAATAGCACTCACCTCACAGGCTTGTTACAAAAATTAAATGAGTTACTATGTATCAAGCACT
TGGAAAAAGCACTTGAACAGTGTTCAGTACACAGTAAGTTTGATACAAATGTTATCAGCATAGTTCAAAC
TTCAGCAAATCCAAAATGTCTGGAAGACCAAGTTTTTTTGTAACCCATCTGGCTACAGGACCCAGCCTGA
CCGGATAGGGTCCTATTTGTAGTTTTTATTTATCCTACTTGGGGTAATAGTCATACATTTCGTTGCAGCA
GTTCTAATTTACTTGATTCTGTAGTGCCGTCCCAGATGCTGCCAGGGTGTTACGGAATCAAGGGCCTATC
TGCACACCATGCTATCTACTGTCCTTCTTCAGCTGGGACATGTACTGACAGTCTGTACCAAGAGCTCCCT
CCACGGGAGCTGCCCTTCCCTCCTTTGCAGCCTCCCACATTGGGTCAGAACTTTCACGTTCCTGCAGAAC
ATTCCCATCCTCACCCAAGAGACAATGCCAGCCTATTTTATCCATTCTAAAACATGCATTTTCTTCCTAT
TTTAACATCTCGGAAATGAAGATGTCTTAATTAAATCATGGTGTATCATTTATAATTAACAGCAGTTTTT
CCTTTCTTGGTAAAGGGCACATAAAATTAAGGGTGTGCCTTACATTTATAACTGGTCCTCATAACCACTC
CTAACATTGCTCACTCTCCTATCAGAAACAGGAACACCTTTCACGTCCATGATCTTGTATGGATCTTTAC
AACAGCCCCTGTCAGGTTGGGATTTTGTCACTGTCATTCCCATTCTGCAGATGAGAAAACTGAGGCCCAG
AGAGGTAGAATAACTTGATTAAAATCACAAAAGTAGTACAATGGATAAAAAAATAAAGGCCCAAATAATA
TCTAAAACTGGTACCCTGCCAGGCTGTAGCTTTTTTTTTTTTTTTAGCTGAGGAAACAGGCACAGAGA
GAACATTCAGGATCCCATATCCAGGAGGGGGCAGAGCAGCAGCTGACTCCAAGTCTTCTAGTTCCCATTC
ACGTCAGCACCCTCTCGGAACTGCCAGCTTACAGCCTGTCTTTTCCATTCATTCTGGAGAGCTGTTAGGG
AGCAGAGACAGGCCCATCTGTTCCCAAGCTTTCAGGGTTGACCAATCATTAGAGAATGGGACTGAAAAGA
AGATGAGAAGGAACCAAGAAGTGTTTGTGCCCTGGAGGGCCTGCAGTATGACTCACTCCTGGTGAGACTG
GGAGAGCAGGTAGTGGCAGAATAGGCATGAAGATGGAGACTAGGAAGGATGTGGGCACTCCTCCTGGCTC

AAGGAGGGCCTACGGTGAAAAAGATGGCTCATACCCCACCCACTAGGCCTGTGAGCCTGGGAGAGGCTGC
CCTTTGATCCCAGGACTTGATCAGACAAGTGGCCAGCTTCTGAGGTAGGGGAGGAACGGAAGCTTGGAA
GGCTGCCTAGAGAGGTCCCATCTGTTTGAGCTGATGCCACAAGGAAGACTGTTCTCCAGGAGGTGAAGGG
AGACTGATCTTGACATATTCCACCCTCATGTTGTGGCCCTTAGGAGAGAGTCCCCACTCTTCCACCAGGT
GGTAAAGCCCTTTTATGATCTGCAACTCGTTTTAGAGAATCCGTCTCTTCTCCATTCCCCCTCAAGCCCT
AAGCACTGTTTCCTGATCACAGTCTTACACTGTGGGTGTGTGAATCTGTCTCTCTGTGACCAGACTTTGA
CCCCTTGAGGGCAGGGACCAATCCTGGTTCCTGTGTATCCCCTACCCACCTGTGACCCCTGAGAGTGGAC
CGGGCCTGGTCCCTGTGTCTCCCTGATCAGACCGTGAGCCCTGCATGCTGGGACTGGGCACAAGGAAGGC
CTCAATAAAAACCCTGGCAAGGGAGCCAGGTTCCTCAAGGAACAGAACTGGAGCACGGGGCAGTGGGTAG
TGTGGCCCGAGGCAGGGTAAATCTTGGTTTCCTCAAGGCATTGTTAGTCAGCCTTTGGCATGGAACACAG
CCTTGAGGGGACAGCCAGGGAGAACACCAGTGTGGCCATCCTAGCTAAGGAGATGGATTCCCAAGTCAGA
AGGGCATGGTCACCGGCCCACATGAGGGTGAGCAAGGAAGGAAGCCCGAGAGGGGTCTCTGGAGAAGGGA
GGGGAAGGAAGAAGTGTGGCGGGGGAGTACTGGAGCTACCTGGAGGGCCGCCACTTGGACAGAGGACAGG
CTGCTAAGCCAGGAAGCAGAATCAAAGTAAACAGCAGTCCCCATGTGTCTCCTGTGTGCTGTGCACTGTT
CTTGGCTGAGGTACAGTGGACCCCTACCCAGGGAAGGGCCTGGTCAGCTACGCTTGGGGATTGGCAGGA
GGCTGCCGTTGCTGGCCTCAGGGAAGAGGGGCCATGGGGCCGGAGATTTGTTGGCGTGGATTGGGAGGCA
AGGACAGGCTGGGGCTAGGATGGCAGCAAAGCTTCCCATTTCCTAGGACAGGTAGGGAACCAGCTGGGGA
GGAGGCACCCACTGCCGTCTGAAGCACCAGTTGGCACTGCGCAGCCCTGGGCCGAGGGAGAGCTTCTCTC
ACTGTGCTCTCCCTTCATCTTTCCTCCTCCTCTGCTGCTGTCTCTGAAGTGTGTGGCCTCAGCCTCTTTC
TGGCTCTCTGAACCTGGGCCTCCCTCTGCCTCTGCCTCCACCTCTCTTGTCTTGAGGCCCTTGCCCCCTT
TCCCCATGGCCTGGGTCTCTTCTTCCCCTCTCTCCACGCCCTCCCTGCTGGGTCCTGCATGGGCTCAGGC
TCCCTTGCCTGGATCAGTAAGTATCTCTCCCTGTGGCACCCTGGGTCACTTTCTTTCTTGCCCTTGCTTT
GGTCTCTCCTGATCACCTTCCTGCTCCGTCCCAAGGCACTGGAGGCTCCAGGGGTTCTTGGGCAGCTCTT
GCTGGGTGGCGGCAGGAGGTGCTCGCGTGGCCCCAGTTGGGCCCTGCAGCCAGGCCAGCCTGCACTCACC
GCCAGGGACTCCTCCTGCTAGCCAGAGGCCTGAGGCGGGCCAGGCAGTTGCCAGCTGAGGCCCAGCTCCT
CAGGAAGGGCCGACAGACTCTCTGCGTCACTGCCCATGTGTGTCCACGGGCAGGGGTGCCTCCTGCTCCC
TGTCCTTCAGAAGTCAGGAGCAGTGCCGGCTTTCCTGAGACTTCGCGGCCAATCTCCCCTTACCCATGGC
CCTTTCCCTAGATCATTACCTCAGAGGAGGCAGAGCGGCGGGGCCAGATCTACGACCGTCAGGGCGCCAC
CTACCTCTTTGACCTGGACTACGTGGAGGACGTGTACACCGTGGATGCCGCCTACTATGGCAACATCTCC
CACTTTGTCAACCACAGTGTGGGTACCCCCGGCAGGCGGGCAAGGGGTCGAGAGGGGCAGGCTGGAGCCC
CTCCTCACTCTCCCAACTGTTCTTTCTCGCCCAGTGTGACCCCAACCTGCAGGTGTACAACGTCTTCATA
GACAACCTTGACGAGCGGCTGCCCCGCATCGCTTTCTTTGCCACAAGAACCATCCGGGCAGGCGAGGAGC
TCACCTTTGATTACAACATGCAAGGTGGGGGTGGCAAGGGACTGGGGGCAGGGGCGCACTGTGACTTGGG
ACACAAGGACCCCTCCCAAAGGCACACCAGTCACTCAGAGAACCAAGGAAAATTCTTTAGCGGGAGCTTT
AAGATGCTCTTCCTGACTCCCAGTCCCCCATTCGGACTACTAGACCCCTGCCTACCCCTCGAGTCCCCTC
ATTCCCAGCCCCTGAACCCTAACCCTGAGGCATTCATACCAACTTCTCCCAAGATTCCTGGAGAGTTAAG
AAACTCCTGACTCCAGTCTTGACTGAGTTTCCCTCATCCCCCAGCCCCTCCCCACTCCTAGCTCCCTTTG
GCCTTCTTCATCCCAAGCCTCTGGACACCCTTGTGGCCTCCAGCCAGTCCCCTCCTTGATAAGCTAGCCA
CCTCTGAGACCCTCAAGGGCAGGTCTTTGGCAGGAGAGTTCAGATGTACCTCTGGATGCCCAGGTGGCCC
TCTATTCCTGCCCCTGCCCCCCCCCACACACCTAAAACGACCCTCCTGGTTCCTTGACGGCTCCCTGCCT
CCCCCAGCATTCTTCAACCCCTGGCCTGAACAACCTCCAGTGCTGAGCCCTGGCCCTAGCCCCAAACCAC
CAACCCTAAGATCTCCCAACTCAAATCCCAGAGTTGCTCCCAGCCCCAAAGATCCTTGGTCTCATGGTAT
AAAGGGTAGTGGGACCCTTCATCCAGGCCTTCTAACAAGTCCCCTGCCTCCTTCCCTACCTTCCTCCCTC
CCTCCTTCTCCCCCTCCCTCCCTGCCTCCTTCCCTCCTCTCCTCCCTGGCTGTGACTCCACAGTGGACCC
CGTGGACATGGAGAGCACCCGCATGGACTCCAACTTTGGCCTGGCTGGGCTCCCTGGCTCCCCTAAGAAG
CGGGTCCGTATTGAATGCAAGTGTGGGACTGAGTCCTGCCGCAAATACCTCTTCTAGCCCTTAGAAGTCT
GAGGCCAGACTGACTGAGGGGGCCTGAAGCTACATGCACCTCCCCCACTGCTGCCCTCCTGTCGAGAATG
ACTGCCAGGGCCTCGCCTGCCTCCACCTGCCCCCACCTGCTCCTACCTGCTCTACGTTCAGGGCTGTGGC
CGTGGTGAGGACCGACTCCAGGAGTCCCCTTTCCCTGTCCCAGCCCCATCTGTGGGTTGCACTTACAAAC
CCCCACCCACCTTCAGAAATAGTTTTTCAACATCAAGACTCTCTGTCGTTGGGATTCATGGCCTATTAAG
GAGGTCCAAGGGGTGAGTCCCAACCCAGCCCCAGAATATATTTGTTTTTGCACCTGCTTCTGCCTGGAGA
TTGAGGGGTCTGCTGCAGGCCTCCTCCCTGCTGCCCCAAAGGTATGGGGAAGCAACCCCAGAGCAGGCAG
ACATCAGAGGCCAGAGTGCCTAGCCCGACATGAAGCTGGTTCCCCAACCACAGAAACTTTGTACTAGTGA
AAGAAAGGGGGTCCCTGGGCTACGGGCTGAGGCTGGTTTCTGCTCGTGCTTACAGTGCTGGGTAGTGTTG
GCCCTAAGAGCTGTAGGGTCTCTTCTTCAGGGCTGCATATCTGAGAAGTGGATGCCCACATGCCACTGGA
AGGGAAGTGGGTGTCCATGGGCCACTGAGCAGTGAGAGGAAGGCAGTGCAGAGCTGGCCAGCCCTGGAGG
TAGGCTGGGACCAAGCTCTGCCTTCACAGTGCAGTGAAGGTACCTAGGGCTCTTGGGAGCTCTGCGGTTG
CTAGGGGCCCTGACCTGGGGTGTCATGACCGCTGACACCACTCAGAGCTGGAACCAAGATCTAGATAGTC
CGTAGATAGCACTTAGGACAAGAATGTGCATTGATGGGGTGGTGATGAGGTGCCAGGCACTGGGTAGAGC
ACCTGGTCCACGTGGATTGTCTCAGGGAAGCCTTGAAAACCACGGAGGTGGATGCCAGGAAAGGGCCCAT
GTGGCAGAAGGCAAAGTACAGGCCAAGAATTGGGGGTGGGGGAGATGGCTTCCCCACTATGGGATGACGA

GGCGAGAGGGAAGCCCTTGCTGCCTGCCATTCCCAGACCCCAGCCCTTTGTGCTCACCCTGGTTCCACTG
GTCTCAAAAGTCACCTGCCTACAAATGTACAAAAGGCGAAGGTTCTGATGGCTGCCTTGCTCCTTGCTCC
CCCACCCCCTGTGAGGACTTCTCTAGGAAGTCCTTCCTGACTACCTGTGCCCAGAGTGCCCCTACATGAG
ACTGTATGCCCTGCTATCAGATGCCAGATCTATGTGTCTGTCTGTGTGTCCATCCCGCCGGCCCCCCAGA
CTAACCTCCAGGCATGGACTGAATCTGGTTCTCCTCTTGTACACCCCTCAACCCTATGCAGCCTGGAGTG
GGCATCAATAAAATGAACTGTCGACTGAACAAA

One exemplary human protein sequence is SEQ ID NO: 2

MAENLKGCSVCCKSSWNQLQDLCRLAKLSCPALGISKRNLYDFEVEYLCDYKKIREQEYY
LVKWRGYPDSESTWEPRQNLKCVRILKQFHKDLERELLRRHHRSKTPRHLDPSLANYLVQ
KAKQRRALRRWEQELNAKRSHLGRITVENEVDLDGPPRAFVYINEYRVGEGITLNQVAVG
CECQDCLWAPTGGCCPGASLHKFAYNDQGQVRLRAGLPIYECNSRCRCGYDCPNRVVQKG
IRYDLCIFRTDDGRGWGVRTLEKIRKNSFVMEYVGEIITSEEAERRGQIYDRQGATYLFD
LDYVEDVYTVDAAYYGNISHFVNHSCDPNLQVYNVFIDNLDERLPRIAFFATRTIRAGEE
LTFDYNMQVDPVDMESTRMDSNFGLAGLPGSPKKRVRIECKCGTESCRKYLF

It is understood that polymorphisms or variants with different sequences exist in various subject's genomes. The term Suv39h1 according to the invention thus encompasses all mammalian variants of Suv39h1, and genes that encode a protein at least 75%, 80%, or typically 85%, 90%, or 95% identical to SEQ ID NO: 2 that has Suv39h1 activity (i.e., the methylation of Lys-9 of histone H3 by H3K9-histone methyltransferase).

Human Suv39h2 methyltransferase is referenced as G5E9A8 in UNIPROT and is encoded by the Suv39h2 gene located on chromosome 10 (p13) (gene ID: 79723 in NCBI).

Inhibition of Suv39h1 refers to a decrease of at least 30%, 40%, 50%, 60%, 70%, 80%, 90%, 95% or 99% or more of Suv39h1 protein activity, and/or protein level, *Suv39h1* gene expression, and/or *Suv39h1* transcript level in a cell or in an organism as compared to normal level (e.g., normal level typically refers to the expression level in a corresponding normal cell or organism, wherein the expression has not been modified). It is of course intended that reduced or decrease activity of Suv39h1 can indeed be achieved by reduced gene expression (at the genomic or transcript level) and/or by the expression of a non-functional Suv39h1 protein.

Inhibition of Suv39h1 activity according to the present invention can thus be achieved at the genomic, transcriptomic, and/or at the protein level. As per the invention, an inhibitor of Suv39h1 can thus be selected among any natural compound or not having the ability to inhibit Suv39h1 activity and/or gene expression (transcription and/or translation). The inhibiting activity of a compound may be determined using various methods as described in Greiner D. Et al. Nat Chem Biol. 2005 Aug;I(3): 143-5 or Eskeland, R. et al. Biochemistry 43, 3740-3749 (2004).

"Inhibition of Suv39h1 activity" as used herein refers to a decrease of Suv39h1 activity of at least 30%, 40%, 50%, 60%, 70%, 80%, 90%, 95% or 99% or more as compared to the activity or level of the Suv39h1 protein which is not inhibited (e.g., as compared to a corresponding cell, tissue, or organism without inhibition). Preferentially, the inhibition of Suv39h1 activity leads to the absence in the cell, tissue, or organism of substantial detectable activity of Suv39h1. The inhibition level of Suv39h1 activity (i.e., Suv39h1 inactivation efficiency) can be assessed typically by assessing the levels of H3K9me3.

"Reduced expression of Suv39h1" as per the invention refers to a decrease of Suv39h1 protein and or/transcript expression of at least 30%, 40%, 50%, 60%, 70%, 80%, 90%, 95% or 99% or more as compared to normal level (e.g., normal level typically refers to the expression level in a corresponding normal cell or organism, wherein the expression has not been modified).

By "non-functional" Suv39h1 protein it is herein intended a protein with a reduced activity, or a lack of detectable activity as described above. Thus, inhibitors of Suv39h1 activity in a cell according to the invention can be selected among any compound or agent natural or not having the ability of inhibiting the methylation of Lys-9 of histone H3 by H3K9-histone methyltransferase, or inhibiting the H3K9-histone methyltransferase Suv39h1 gene expression.

As detailed below, a Suv39h1 inhibitor can be selected from a nucleic acid, a peptide, a ribonucleic complex, a ribonucleoprotein (RNP) complex, a small molecule, as well derivatives and combinations thereof.

In the context of the present invention, an inhibitor Suv39h1 according to the present invention is preferably selective for Suv39h1, as compared with other histone methyltransferases such EZH2, G9A or Setdb1. The inhibitor of Suv39h1 can also be selective for Suv39h1 as compared with the histone methyltransferase Suv39h2. By "selective" it is meant that the affinity of the inhibitor is at least 10-fold, preferably 25-fold, more preferably 100-fold, and still preferably 500-fold higher than the affinity for other histone methyltransferases.

Typically, the inhibitor of Suv39h1 of the invention has an IC50 of less than 20 µM, preferably less than 10 µM, more preferably less than 5 µM, even more preferably less than 1 µM or less than 0.5 µM. Typically also the inhibitor of SUV39H1 has an IC50 for the other methyltransferase such as for example EZH2, G9A or Setbd1 of more than 10 µM, preferably more than 20 µM, more preferably more than 50 µM.

In some embodiments, the Suv39h1 inhibitor specifically inhibits Suv39h1 activity (e.g. relative to other histone methyltransferases (HMTs) such as one or more of G9a, DOT1, EZH1, EZH2, GLP, MLL1, MLL2, MLL3, MLL4, NSD2, SETIb, SET7/9, SET8, SETMAR, SMYD2, Suv39h2). The inhibition may be at least about 2, 3, 4, 5, 10, 100, or 1000 fold greater inhibition relative to inhibition of other histone methyltransferases such as one or more of G9a, DOT1, EZH1, EZH2, GLP, MLL1, MLL2, MLL3, MLL4, NSD2, Setlb, SET7/9, SET8, SETMAR, SMYD2, Suv39h2. In embodiments, the inhibition of Suv39h1 may be at least 2; 3; 4; 5; 6; 7; 8; 9 or 10 fold greater than the inhibition of other HMTs described herein.

In some embodiments, the inhibition of Suv39h1 may be at least 10; 20, 30, 40; 50; 60, 70; 80; 90 or 100 fold greater than the inhibition of other HMTs described herein.

In some embodiments, Suv39h1 inhibition is achieved using a Suv39h1 inhibitor selected among natural compounds or not having the ability to inhibit the methylation of Lys-9 of histone H3 by H3K9-histone methyltransferase, for example by using a small molecule inhibitor (i.e., a small organic molecule). In some embodiments, the inhibitor of Suv39h1 according to the present invention is not triptolide and/or is not chaetocin.

### Small molecules:

The term "small organic molecule" refers to a molecule of a size comparable to those organic molecules generally used in pharmaceuticals. The term excludes biological macro molecules (e. g., proteins, nucleic acids, etc.). Preferred small organic molecules range in size up to about 5000 Da, more preferably up to 2000 Da, and most preferably up to about 1000 Da.

In a particular embodiment, the inhibitor of H3K9 -histone methyltransferase Suv39h1 is chaetocin (CAS 28097-03-2) as described by Greiner D, Bonaldi T, Eskeland R, Roemer E, Imhof A. "Identification of a specific inhibitor of the histone methyltransferase SU(VAR)3-9". Nat Chem Biol. 2005 Aug;I(3): 143-5.; Weber, H. P., et al, "The molecular structure and absolute configuration of chaetocin", Acta Cryst, B28, 2945-2951 (1972) ; Udagawa, S., et al, "The production of chaetoglobosins, sterigmatocystin, O-methylsterigmatocystin, and chaetocin by Chaetomium spp. and related fungi", Can. J. microbiol, 25, 170-177 (1979); and Gardiner, D. M., et al, "The epipolythiodioxopiperazine (ETP) class of fungal toxins: distribution, mode of action, functions and biosynthesis", Microbiol, 151, 1021-1032 (2005). For example, chaetocin is commercially available from Sigma Aldrich.

An inhibitor of Suv39h1 can also be ETP69 (Rac-(3S,6S,7S,8aS)-6-(benzo[d][1,3]dioxol-5-yl)-2,3,7-trimethyl-1,4-dioxohexahydro-6H-3,8a-epidithiopyrrolo[1,2-a]pyrazine-7-carbonitrile), a racemic analog of the epidithiodiketopiperazine alkaloid chaetocin A (see WO2014066435 but see also Baumann M, Dieskau AP, Loertscher BM, et al. Tricyclic Analogues of Epidithiodioxopiperazine Alkaloids with Promising In Vitro and In Vivo Antitumor Activity. Chemical science (Royal Society of Chemistry: 2010). 2015;6:4451-4457, and Snigdha S, Prieto GA, Petrosyan A, et al. H3K9me3 Inhibition Improves Memory, Promotes Spine Formation, and Increases BDNF Levels in the Aged Hippocampus. The Journal of Neuroscience. 2016;36(12):3611-3622).

Identification of new small molecule inhibitors can be achieved according to classical techniques in the field. The current prevailing approach to identify hit compounds is with a high throughput screen (HTS).

### Aptamers, intrabodies and affibodies

In another embodiments, the inhibitor Suv39h1 is an aptamer. Aptamers are a class of molecules that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. and Gold L., 1990. The random sequence library is obtainable by combinatorial chemical synthesis of DNA. In this library, each member is a linear oligomer of a unique sequence that is optionally chemically modified. Possible modifications, uses and advantages of this class of molecules have been reviewed in Jayasena S.D., 1999. Peptide aptamers consists of a conformationally constrained antibody variable region displayed by a platform protein, such as E. coli Thioredoxin A that are selected from combinatorial libraries by two hybrid methods (Colas P, Cohen B, Jessen T, Grishina I, McCoy J, Brent R. "Genetic selection of peptide aptamers that recognize and inhibit cyclin-dependent kinase 2". Nature. 1996 Apr 11;380(6574):548-50).

Inhibition of Suv39h1 in a cell according to the invention may also be achieved with intrabodies. Intrabodies are antibodies that bind intracellularly to their antigen after being produced in the same cell (for a review se for example, Marschall AL, Dübel S and Böldicke T "Specific in vivo knockdown of protein function by intrabodies", MAbs. 2015;7(6):1010-35. but see also Van Impe K, Bethuyne J, Cool S, Impens F, Ruano-Gallego D, De Wever O, Vanloo B, Van Troys M, Lambein K, Boucherie C, et al. "A nanobody targeting the F-actin capping protein CapG restrains breast cancer metastasis". Breast Cancer Res 2013; 15:R116; Hyland S, Beerli RR, Barbas CF, Hynes NE, Wels W.. "Generation and functional characterization of intracellular antibodies interacting with the kinase domain of human EGF receptor. Oncogene 2003; 22:1557-67"; Lobato MN, Rabbitts TH. "Intracellular antibodies and challenges facing their use as therapeutic agents". Trends Mol Med 2003; 9:390-6, and Donini M, Morea V, Desiderio A, Pashkoulov D, Villani ME, Tramontano A, Benvenuto E. "Engineering stable cytoplasmic intrabodies with designed specificity". J Mol Biol. 2003 Jul 4;330(2):323-32.). Intrabodies can be generated by cloning the respective cDNA from an existing hybridoma clone or more conveniently, new scFvs/Fabs can be selected from *in vitro* display techniques such as phage display which provide the necessary gene encoding the antibody from the onset and allow a more detailed predesign of antibody fine specificity. In addition, bacterial-, yeast-, mammalian cell surface display and ribosome display can be employed. However, the most commonly used *in vitro* display system, for selection of specific antibodies, is phage display. In a procedure called panning (affinity selection), recombinant antibody phages are selected by incubation of the antibody phage repertoire with the antigen. This process is repeated several times leading to enriched antibody repertoires comprising specific antigen binders to almost any possible target. To date, *in vitro* assembled recombinant human antibody libraries have already yielded thousands of novel recombinant antibody fragments. It is to be noted that the prerequisite for a specific protein knockdown by a cytoplasmic intrabody is that the antigen is neutralized/inactivated through the antibody binding. Five different approaches to generate suitable antibodies have emerged : 1) In vivo selection of functional intrabodies in eukaryotes such as yeast and in prokaryotes such as E.coli (antigen-dependent and independent); 2) generation of antibody fusion proteins for improving cytosolic stability; 3) use of special frameworks for improving cytosolic stability (e.g., by grafting CDRs or introduction of synthetic CDRs in stable antibody frameworks); 4) use of single domain antibodies for improved cytosolic stability; and 5) selection of disulfide bond free stable intrabodies. Those approaches are notably detailed in Marschall, A. L et al., mAbs 2015, as mentioned above.

The most frequent format for intrabodies is the scFv, which consists of the H- and L-chain variable antibody domain (VH and VL) held together by a short, flexible linker sequence (frequently (Gly4Ser)3), to avoid the need for separate expression and assembly of the 2 antibody chains of a full IgG or Fab molecule. Alternatively, the Fab format comprising additionally the C1 domain of the heavy chain, and the constant region of the light chain has been used. Recently, a new possible format for intrabodies, the scFab, has been described. The scFab format promises easier subcloning of available Fab genes into the intracellular expression vector, but it remains to be seen whether this provides any advantage over the well-established scFv format. In addition to scFv and Fab, bispecific formats have been used as intrabodies. A bispecific Tie-2 x VEGFR-2 antibody targeted to the ER demonstrated an extended half-life compared to the monospecific antibody counterparts. A bispecific transmembrane intrabody has been developed as a special format to simultaneously recognize intra- and extracellular epitopes of the epidermal growth factor, combining the distinct features of the related monospecific antibodies, i.e., inhibition of autophosphorylation and ligand binding.

Another intrabody format particularly suitable for cytoplasmic expression are single domain antibodies (also called nanobodies) derived from camels or consisting of one human VH domain or human VL domain. These single domain antibodies often have advantageous properties, e.g., high stability; good solubility; ease of library cloning and selection; high expression yield in E. *coli* and yeasts.

The intrabody gene can be expressed inside the target cell after transfection with an expression plasmid or viral transduction with a recombinant virus. Typically, the choice is aimed at providing optimal intrabody transfection and production levels. Successful transfection and subsequent intrabody production can be analyzed by immunoblot detection of the produced antibody, but, for the evaluation of correct intrabody/antigen-interaction, co-immunoprecipitation from HEK293 cell extracts transiently co-transfected with the corresponding antigen and intrabody expression plasmids may be used.

In other embodiments, inhibition of Suv39h1 in a cell may also be achieved with affibody molecules. Affibody are small proteins engineered to bind to a large number of target proteins or peptides with high affinity, imitating monoclonal antibodies, which are therefore a member of the family of antibody mimetics (see for review Löfblom J, Feldwisch J, Tolmachev V, Carlsson J, Ståhl S, Frejd FY. Affibody molecules: engineered proteins for therapeutic, diagnostic and biotechnological applications. FEBS Lett. 2010 Jun 18;584(12):2670-80). Affibody molecules are based on an engineered variant (the Z domain) of the B-domain in the immunoglobulin-binding regions of staphylococcal protein A, with specific binding for theoretically any given target. Affibody molecule libraries are generally constructed by combinatorial randomization of 13 amino acid positions in helices one and two that comprise the original Fc-binding surface of the Z-domain. The libraries have typically been displayed on phages, followed by bio-panning against desired targets. Should the affinity of the primary be increased, affinity maturation generally results in improved binders and may be achieved by either helix shuffling or sequence alignment combined with directed combinatorial mutagenesis. The newly identified molecules with their altered binding surface generally keep the original helical structure as well as the high stability, although unique exceptions with interesting properties have been reported. Due to their small size and rapid folding properties, affibody molecules can be produced by chemical peptide synthesis.

In some embodiments, Suv39h1 inhibition is achieved through repression of the *Suv39h 1* gene expression, through inactivation of the *Suv39h 1* gene of the cell, or through expression of exogenous inhibitors. For example, *Suv39h1* repression (at the genomic and/or transcriptomic level) may reduce expression of Suv39h1 in the cell by at least 50, 60, 70, 80, 90, or 95% as to the same cell produced by the method in the absence of the repression. Gene disruption may a lead to a reduced expression of the Suv39h1 protein or to the expression of a non-functional Suv39h1 protein. Inhibition or repression of Suv39h1 activity, notably including inhibition or repression of Suv39h1 expression, in one or more cell, or in an individual, according to the present invention can be permanent and irreversible or transient or reversible. In some embodiments, *Suv39h1* inhibition, disruption or repression is permanent and irreversible.

### Antagonistic nucleic acids

In some embodiments, a Suv39h1 inhibitor reduces the expression of a Suv39h1 protein, for example by gene repression/suppression through gene knockdown using antagonistic nucleic acids which are typically capable of inhibiting or reducing the expression of a gene or a transcript thereof and translation into a protein. Antagonistic nucleic acids typically include short interfering RNA (siRNA) short hairpin RNA (shRNA), IncRNA or ribozymes. Suitable Suv39h1 inhibitors include, for example, agents that hybridize or bind to the Suv39h1 gene or its regulatory elements, such as aptamers that block or inhibit Suv39h1 expression or activity as well as nucleic acid molecules that block transcription or translation, such as antisense molecules complementary to Suv39h1 (e.g. antisens RNAs) such as RNA interfering agents (such as a small interfering RNAs (siRNAs), small hairpin RNAs (shRNAs), microRNAs (miRNAs), piwiRNAs (piRNAs)); long non-coding RNAs (IncRNAs), ribozymes and combinations thereof.

Antisense RNAs or asRNAs are single or double-stranded RNA molecules exhibiting preferably at least 90%, more preferably 95% and especially 100% (of the nucleotides of a dsRNA) sequence identity to a section of a naturally occurring mRNA sequence.

siRNAs are small (^{~}12-35 nucleotide) non-coding RNA molecules capable of inducing RNAi. siRNAs comprise an RNA duplex (double-stranded region) formed by complement base pairing with phosphorylated 5'-ends and hydroxylated 3'-ends, optionally with one or two single-stranded overhanging nucleotides. The duplex portion typically comprises between 17 and 29 nucleotides. siRNA may be generated from two RNA molecules that hybridize together or may alternatively be generated from a single RNA molecule that includes a self-hybridizing portion (shRNA). The duplex portion of an siRNA may, but typically does not, include one or more bulges containing one or more unpaired and/or mismatched nucleotides in one or both strands of the duplex or may contain one or more non-complementary nucleotide pairs. One strand of a siRNA (referred to as the antisense strand) includes a portion that hybridizes with a target transcript (e.g. a target mRNA). The antisense strand may be precisely complementary with a complementary region of the target transcript (i.e. the siRNA antisense strand may hybridize to the target sequence without a single mismatch, wobble base pairing or nucleotide bulge) or one or more mismatches, wobble (G:U) base pairings and/or nucleotide bulges between the siRNA antisense strand and the complementary region of the target transcript may exist.

"MicroRNAs" or "miRNAs" are small (^{~}20-24 nucleotide) non-coding double-stranded RNAs (dsRNAs) capable of recruiting the AGO-2 RISC complex to a complementary target transcript, thereby preferably inducing the miRNA-mediated RNAi pathway. MicroRNAs are typically processed from pri-microRNA to short stem-loop structures called pre-microRNA and finally to mature miRNA. Both strands of the stem of the pre-microRNA may be processed to a mature microRNA. After processing, the mature single-stranded microRNAs, associated with Argonaute 2 (AGO2) in the RNA-induced silencing complex (RISC), typically bind to the 3' UTRs of their cytosolic mRNA targets, resulting in either reduced translation or deadenylation and degradation of the mRNA transcript. The predominant function of microRNAs is thus to (negatively) regulate protein translation by binding to complementary sequences of target mRNAs. The term "microRNA" includes miRNAs, mature single stranded miRNAs, precursor miRNAs (pre-miRNA), primary miRNA transcripts (pri-miRNA), duplex miRNAs and variants thereof. MicroRNAs are particularly envisaged to be capable of binding to a target site within a 3" untranslated region of a target nucleic acid.

Anti-sense oligonucleotides, including anti-sense RNA molecules and anti-sense DNA molecules, would act to directly block the translation of H3K9-histone methyltransferase Suv39h1 and thus preventing protein translation or increasing mRNA degradation, thus decreasing the level of H3K9-histone methyltransferase SUV39H1 and thus its activity in a cell. For example, antisense oligonucleotides of at least about 15 bases and complementary to unique regions of the mRNA transcript sequence encoding H3K9-histone methyltransferase SUV39H1 can be synthesized, e.g., by conventional phosphodiester techniques and administered by e.g., intravenous injection or infusion. Methods for using antisense techniques for specifically inhibiting gene expression of genes whose sequence is known are well known in the art (see for example U.S. Pat. Nos. 6,566,135; 6,566, 131; 6,365,354; 6,410,323; 6,107,091; 6,046,321; and 5,981,732).

An "RNA interfering agent" as used herein, is defined as any agent, which interferes with or inhibits expression of a target biomarker gene by RNA interference (RNAi). Such RNA interfering agents include, but are not limited to, nucleic acid molecules including RNA molecules, which are homologous to the target gene of the invention (e.g., Suv39h1), or a fragment thereof, short interfering RNA (siRNA), and small molecules which interfere with or inhibit expression of the target nucleic acid by RNA interference (RNAi).

Small inhibitory RNAs (siRNAs) can also function as inhibitors of expression for use in the present invention. H3K9-histone methyltransferase Suv39h1 gene expression can be reduced by contacting a subject or cell with a small double stranded RNA (dsRNA), or a vector or construct causing the production of a small double stranded RNA, such that H3K9-histone methyltransferase *Suv39h1* expression is specifically inhibited (i.e., RNA interference or RNAi). Methods for selecting an appropriate dsRNA or dsRNA-encoding vector are well known in the art for genes whose sequence are well known by persons skilled in the art (see for example Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836). All or part of the phosphodiester bonds of the siRNAs of the invention are advantageously protected. This protection is generally implemented via the chemical route using methods that are currently used in the field. The phosphodiester bonds can be protected, for example, by a thiol or amine functional group or by a phenyl group. The 5'- and/or 3'- ends of the siRNAs of the invention are also advantageously protected, for example, using the technique described above for protecting the phosphodiester bonds. The siRNAs sequences advantageously comprise at least twelve contiguous dinucleotides or derivatives thereof.

As used herein, the term "siRNA derivatives" with respect to the present nucleic acid sequences refers to a nucleic acid having a percentage of identity of at least 90% with erythropoietin or fragment thereof, preferably of at least 95%, as an example of at least 98%, and more preferably of at least 98%.

shRNAs (short hairpin RNA) can also function as inhibitors of expression for use in the present invention. shRNAs are typically composed of a short (e.g., 19-25 nucleotide) antisense strand, followed by a 5-9 nucleotide loop, and the analogous sense strand.

Alternatively, the sense strand may precede the nucleotide loop structure and the antisense strand may follow.

Ribozymes can also function as inhibitors of expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of H3K9-histone methyltransferase SUV39H1 mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable.

In some embodiments, the Suv39h1 inhibitor is a IncRNA. It has indeed been shown that antisense IncRNA, AF196970.3 has silencing function against SUV39H1 in human cells, inhibiting SUV39H1 expression and thus lowering levels of SUV39H1 protein in the cell. AF196970.3 has an expression pattern very similar to the expression pattern of SUV39H1. It is detected in many different cell types, with endothelial cells, fibroblasts and myocytes showing the highest levels, similar to SUV39H1. The gene locus of SUV39H1 is on the X chromosome (position p11.23, 48695554-48709016, in the GRCh38.p13 assembly). At the same locus, in anti-parallel orientation, the non-annotated gene ENSG00000232828 is located at positions 48698963-48737163. Both genes have annotated promoter regions. SEQ ID NO: 3: IncRNA AF196970.3 RNA sequence: 
(CGAGGCAGGGCUUUGGCUACUGGAGAUCGUAGGUUCGAAUCCCGUCUGGGA
AGUUCAACUUGUGCACCUGUAAAAGAAGCUGGCAUUAUUGGCUUGUACUCAA
GGGCUGGCACAGAGUGUGUCGGGGUGCGGACGCCCCAGCCACGCCCAUCAU
CCGUGCGGAAGAUGCAGAGGUCAUAUCGGAUACCCUUCUGUACCACACGAUU
UGGGCAGUCAUAGCCGCAGCGGCAGCGGGAGUUGCACUCGUAGAUGGGCAG
CCCGGCUCGAAGCCGCACCUGGCCCUGGUCAUUGUAGGCAAACUUGUGCAG
 
UGACGCCCCCGGGCAGCAGCCUCCAGUGGGUGCCCACAGACAGUCCUGGCA
CUCGCAGCCCACAGCCACCUGGUUGAGGGUGAUGCCCUCACCAACACGGUAC
UCAUUGAUGUACACGAAGGCCCGCGGAGGGCCGUCCAGGUCCACCUCAUUCU
CUACAGUGAUGCGUCCCAGAUGGCUGCGCUUGGCAUUGAGCUCCUGCUCCC
AGCGACGGAGCGCCCGCCUCUGCUUGGCCUUCUGCACCAGGUAGUUGGCCA
AGCUUGGGUCCAGGUGCCGGGGGUCUUUGACCGGUGGUGCCGCCGGAGC
AGCUCCCUUUCUAAGUCCUUGUGGAACUGCUUGAGGAUACGCACACACUUGA
GAUUCUGCCGUGGCUCCCAGGUGCUCUCUGAGUCUGGAUAUCCACGCCAUU
UCACCAGGGUCAAAGGAGAAAUUCCCUUUGGAAACAGAUGUGGGCAGUUGG
GGACAAGAGGGCAGGACACUAACUUCCUUGUGACCUGUCCCCUCCCAGAGCA
UGGUCACCCCAGACUCACGCGGAUCUUCUUGUAAUCGCACAGGUACUCGACU
UCAAAGUCAUAGAGGUUCCUCUUAGAGAUACCGAGGGCAGGGCAGGAGAGC)
 is the predicted RNA sequence expressed from ENSG00000232828, after transcription and splicing. SEQ ID NO: 1 is a 925 base sequence that includes three exons. AF196970.3 exon 1 is 125 bases in length and does not have significant complementarity to the SUV39H1 gene. AF196970.3 exon 2 is 600 bases in length and is anti-parallel to a substantial portion of exon 3 of the SUV39H1 gene with 100% similarity. AF196970.3 exon 3 is 200 bases in length and is anti-parallel to a portion (42.5% similarity) of exon 2 of the SUV39G1 gene, as well as a portion of the adjacent intron. Inhibitory polynucleotides of the disclosure, for use in the cells and methods of the disclosure, include AF196970.3 (SEQ ID NO: 1) or fragments or variants thereof.

Both oligonucleotides and ribozymes useful as inhibitors of expression can be prepared by known methods. These include techniques for chemical synthesis such as, e.g., by solid phase phosphoramadite chemical synthesis. Alternatively, anti-sense RNA molecules can be generated by *in vitro* or *in vivo* transcription of exogenous DNA sequences encoding the RNA molecule(s). Such DNA sequences can be incorporated into a wide variety of vectors that incorporate suitable RNA polymerase promoters (such as the T7 or SP6 polymerase promoters). Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-0-methyl rather than phosphodiesterase linkages within the oligonucleotide backbone.

In some embodiments, inactivation of Suv39h1 in a cell or in an organism, according to the present invention, is achieved *via* repression or disruption of the *Suv39h1* gene, such as by deletion (*e*.*g*., deletion of the entire gene, exon, or region, and/or replacement with an exogenous sequence), mutation (*e*.*g*., frameshift or missense mutation) within the gene typically within an exon of the gene. In some embodiments, the disruption results in a premature stop codon being incorporated into the gene, such that the Suv39h1 protein is not expressed or is non-functional. The disruption is generally carried out at the DNA level. The disruption generally is permanent, irreversible, or not transient.

### Gene editing agents

Suitable Suv39h1 inhibitors can also include gene editing agent such as targeted nucleases, including *e*.*g*., Zinc finger, TALE and Cas nucleases. For example, a Suv39h1 inhibitor can include an exogenous nucleic acid comprising a) an engineered, non-naturally occurring Clustered Regularly Interspaced Short Palindromic Repeats (CRISPR) guide RNA (gRNA) that hybridizes with *Suv39h1* genomic nucleic acid sequence and/or b) a CRISPR Cas protein (for example a Type-II Cas9 protein) or a derivative thereof or a nucleotide sequence encoding thereof.

Suitable Suv39h1 inhibitors can also include non-functional Suv39h1. In some embodiments the inhibitor is a dominant negative *Suv39h1* gene that expresses non-functional gene product at a level that inhibits activity of the wildtype Suv39h1. This may comprise overexpression of the dominant negative *Suv39h1* gene.

In some embodiments, the gene inactivation is achieved using gene editing agents such as a DNA-targeting molecule, such as a DNA-binding protein or DNA-binding nucleic acid, or complex, compound, or composition, containing the same, which specifically binds to or hybridizes to the gene. In some embodiments, the DNA-targeting molecule comprises a DNA-binding domain, e.g., a zinc finger protein (ZFP) DNA-binding domain, a transcription activator-like protein (TAL) or TAL effector (TALE) DNA-binding domain, a clustered regularly interspaced short palindromic repeats (CRISPR) DNA-binding domain, or a DNA-binding domain from a meganuclease.

Zinc finger, TALE, and CRISPR system binding domains can be "engineered" to bind to a predetermined nucleotide sequence.

In some embodiments, the DNA-targeting molecule, complex, or combination contains a DNA-binding molecule and one or more additional domain, such as an effector domain to facilitate the repression or disruption of the gene. For example, in some embodiments, the gene disruption is carried out by fusion proteins that comprise DNA-binding proteins and a heterologous regulatory domain or functional fragment thereof.

Typically, the additional domain is a nuclease domain. Thus, in some embodiments, gene disruption is facilitated by gene or genome editing, using engineered proteins, such as nucleases and nuclease-containing complexes or fusion proteins, composed of sequence-specific DNA-binding domains fused to, or complexed with, non-specific DNA-cleavage molecules such as nucleases.

These targeted chimeric nucleases or nuclease-containing complexes carry out precise genetic modifications by inducing targeted double- stranded breaks or single-stranded breaks, stimulating the cellular DNA -repair mechanisms, including error-prone nonhomologous end joining (NHEJ) and homology-directed repair (HDR). In some embodiments the nuclease is an endonuclease, such as a zinc finger nuclease (ZFN), TALE nuclease (TALEN), an RNA-guided endonuclease (RGEN), such as a CRISPR-associated (Cas) protein, or a meganuclease. Such systems are well-known in the art (see, for example, U.S. Pat. No. 8,697,359; Sander and Joung (2014) Nat. Biotech. 32:347-355; Hale et al. (2009) Cell 139:945-956; Karginov and Hannon (2010) Mol. Cell 37:7; U.S. Pat. Publ. 2014/0087426 and 2012/0178169; Boch et al. (2011) Nat. Biotech. 29: 135-136; Boch et al. (2009) Science 326: 1509-1512; Moscou and Bogdanove (2009) Science 326: 1501; Weber et al. (2011) PLoS One 6:el9722; Li et al. (2011) Nucl. Acids Res. 39:6315-6325; Zhang et al. (2011) Nat. Biotech. 29: 149-153; Miller et al. (2011) Nat. Biotech. 29: 143-148; Lin et al. (2014) Nucl. Acids Res. 42:e47). Such genetic strategies can use constitutive expression systems or inducible expression systems according to well-known methods in the art.

### ZFPs and ZFNs; TALs, TALEs, and TALENs

In some embodiments, the DNA-targeting molecule includes a DNA-binding protein such as one or more zinc finger protein (ZFP) or transcription activator-like protein (TAL), fused to an effector protein such as an endonuclease. Examples include ZFNs, TALEs, and TALENs. See Lloyd et al., Frontiers in Immunology, 4(221), 1-7 (2013). In some embodiments, the DNA-targeting molecule comprises one or more zinc-finger proteins (ZFPs) or domains thereof that bind to DNA in a sequence- specific manner. A ZFP or domain thereof is a protein or domain within a larger protein, that binds DNA in a sequence-specific manner through one or more zinc fingers regions of amino acid sequence within the binding domain whose structure is stabilized through coordination of a zinc ion. Generally, sequence-specificity of a ZFP may be altered by making amino acid substitutions at the four helix positions (-1, 2, 3 and 6) on a zinc finger recognition helix. Thus, in some embodiments, the ZFP or ZFP-containing molecule is non-naturally occurring, e.g., is engineered to bind to the target site of choice. See, for example, Beerli et al. (2002) Nature Biotechnol. 20: 135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416.

In some embodiments, the DNA-targeting molecule is or comprises a zinc-finger DNA binding domain fused to a DNA cleavage domain to form a zinc-finger nuclease (ZFN). In some embodiments, fusion proteins comprise the cleavage domain (or cleavage half-domain) from at least one Type IIS restriction enzyme and one or more zinc finger binding domains, which may or may not be engineered. In some embodiments, the cleavage domain is from the Type IIS restriction endonuclease Fok I. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982.

In some aspects, the ZFNs efficiently generate a double strand break (DSB), for example at a predetermined site in the coding region of the targeted gene (i.e. SUV39H1). Typical targeted gene regions include exons, regions encoding N-terminal regions, first exon, second exon, and promoter or enhancer regions. In some embodiments, transient expression of the ZFNs promotes highly efficient and permanent disruption of the target gene in the engineered cells. In particular, in some embodiments, delivery of the ZFNs results in the permanent disruption of the gene with efficiencies surpassing 50%. Many gene-specific engineered zinc fingers are available commercially. For example, Sangamo Biosciences (Richmond, CA, USA) has developed a platform (CompoZr) for zinc-finger construction in partnership with Sigma-Aldrich (St. Louis, MO, USA), allowing investigators to bypass zinc-finger construction and validation altogether, and provides specifically targeted zinc fingers for thousands of proteins. Gaj et al., Trends in Biotechnology, 2013, 31(7), 397-405. In some embodiments, commercially available zinc fingers are used or are custom designed. (See, for example, Sigma-Aldrich catalog numbers CSTZFND, CSTZFN, CTI1-1KT, and PZD0020).

In some embodiments, the DNA-targeting molecule comprises a naturally occurring or engineered (non-naturally occurring) transcription activator-like protein (TAL) DNA binding domain, such as in a transcription activator-like protein effector (TALE) protein, See, e.g., U.S. Patent Publication No. 20110301073. In some embodiments, the molecule is a DNA binding endonuclease, such as a TALE-nuclease (TALEN). In some aspects the TALEN is a fusion protein comprising a DNA-binding domain derived from a TALE and a nuclease catalytic domain to cleave a nucleic acid target sequence. In some embodiments, the TALE DNA-binding domain has been engineered to bind a target sequence within genes that encode the target antigen and/or the immunosuppressive molecule. For example, in some aspects, the TALE DNA-binding domain may target CD38 and/or an adenosine receptor, such as A2AR.

In some embodiments, the TALEN recognizes and cleaves the target sequence in the gene. In some aspects, cleavage of the DNA results in double-stranded breaks. In some aspects, the breaks stimulate the rate of homologous recombination or non-homologous end joining (NHEJ). Generally, NHEJ is an imperfect repair process that often results in changes to the DNA sequence at the site of the cleavage. In some aspects, repair mechanisms involve rejoining of what remains of the two DNA ends through direct re-ligation (Critchlow and Jackson, Trends Biochem Sci. 1998 Oct;23(10):394-8) or via the so-called microhomology-mediated end joining. In some embodiments, repair via NHEJ results in small insertions or deletions and can be used to disrupt and thereby repress the gene. In some embodiments, the modification may be a substitution, deletion, or addition of at least one nucleotide. In some aspects, cells in which a cleavage-induced mutagenesis event, i.e. a mutagenesis event consecutive to an NHEJ event, has occurred can be identified and/or selected by well-known methods in the art.

TALE repeats can be assembled to specifically target *Suv39h1.* (Gaj et al., Trends in Biotechnology, 2013, 31(7), 397-405). A library of TALENs targeting 18,740 human protein-coding genes has been constructed (Kim et al., Nature Biotechnology. 31, 251-258 (2013)). Custom-designed TALE arrays are commercially available through Cellectis Bioresearch (Paris, France), Transposagen Biopharmaceuticals (Lexington, KY, USA), and Life Technologies (Grand Island, NY, USA). Specifically, TALENs that target CD38 are commercially available (See Gencopoeia, catalog numbers HTN222870-1, HTN222870-2, and HTN222870-3, available on the World Wide Web at www. genecopoeia.com/product/search/detail.php?prt=26&cid=&key=HTN222870). Exemplary molecules are described, e.g., in U.S. Patent Publication Nos. US 2014/0120622, and 2013/0315884.

In some embodiments the TALENs are introduced as transgenes encoded by one or more plasmid vectors. In some aspects, the plasmid vector can contain a selection marker which provides for identification and/or selection of cells which received said vector.

### RGENs (CRISPR/Cas systems)

The gene repression or downregulation as per the present method can be carried out using one or more DNA -binding nucleic acids, such as disruption via an RNA-guided endonuclease (RGEN), or other form of repression by another RNA-guided effector molecule. For example, in some embodiments, the gene repression can be carried out using clustered regularly interspaced short palindromic repeats (CRISPR) and CRISPR-associated proteins (See Sander and Joung, Nature Biotechnology, 32(4): 347-355).

In general, "CRISPR system" refers collectively to transcripts and other elements involved in the expression of, or directing the activity of, CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), and/or other sequences and transcripts from a CRISPR locus.

Typically, the CRISPR/Cas nuclease or CRISPR/Cas nuclease system includes a non-coding RNA molecule (guide) RNA, which sequence-specifically binds to DNA, and a CRISPR protein, with nuclease functionality (e.g., two nuclease domains). One or more elements of a CRISPR system can derive from a type I, type II, or type III CRISPR system, such as a Cas protein (e.g. a Cas nuclease). Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cpf1, or a variant thereof. In particular embodiments, the Cas protein is Cas9 nuclease. Preferably, the CRISPR protein is a Cas enzyme such as Cas9. Cas enzymes are well-known in the field; for example, the amino acid sequence of S. pyogenes Cas9 protein may be found in the SwissProt database under accession number Q99ZW2.

In some embodiments, a Cas nuclease and gRNA are introduced into the cell. In some embodiments, the CRISPR system induces DSBs at the target site, followed by disruptions as discussed herein. Common to all these Cas9 or Casç-like)-mediated editing strategies is the induction of double-strand breaks (DSBs) in DNA which are subsequently repaired by either non-homologous end joining (NHEJ) or homology-directed repair (HDR). Incomplete repair of these breakage points may give rise to indels leading to frameshift mutations and subsequent knock-out of one or more targeted genes. When combined with DNA donor template, the same toolkit allows the targeted integration of exogenous DNA inserts at defined genomic loci. Indeed, in general, a CRISPR system is characterized by elements that promote the formation of a CRISPR complex at the site of the target sequence. Typically, in the context of formation of a CRISPR complex, "target sequence" generally refers to a sequence to which a guide sequence is designed to have complementarity, where hybridization between the target sequence and a guide sequence promotes the formation of a CRISPR complex. Full complementarity is not necessarily required, provided there is sufficient complementarity to cause hybridization and promote formation of a CRISPR complex. The target sequence may comprise any polynucleotide, such as DNA or RNA polynucleotides. Generally, a sequence or template that may be used for recombination into the targeted locus comprising the target sequences is referred to as an "editing template" or "editing polynucleotide" or "editing sequence". In some aspects, an exogenous template polynucleotide may be referred to as an editing template. In some aspects, the recombination is homologous recombination. In some embodiments, Cas9 variants, deemed "nickases" can be used to nick a single strand at the target site. Paired nickases can also be used, e.g., to improve specificity, each directed by a pair of different gRNAs targeting sequences. In still other embodiments, catalytically inactive Cas9 can be fused to a heterologous effector domain, such as a transcriptional repressor, to affect gene expression.

In some embodiments, nuclease-inactive platforms including base and prime editors can be used as they offer a potentially safer route to rewriting genetic sequences and introducing large segments of transgenic DNA without inducing double-strand breaks (DSBs) (see notably Biederstädt A, Manzar GS, Daher M. Multiplexed engineering and precision gene editing in cellular immunotherapy. Front Immunol. 2022;13:1063303; and Liang, Y., Chen, F., Wang, K., & Lai, L. (2023). Base editors: development and applications in biomedicine. Frontiers of medicine, 10.1007/s11684-023-1013-y; see also Collantes JC, Tan VM, Xu H, et al. Development and Characterization of a Modular CRISPR and RNA Aptamer Mediated Base Editing System. CRISPR J. 2021;4(1):58-68, related to Pin-Point editing).

Base editing typically refers to the sgRNA-directed exchange of single nucleotides mediated by modified forms of Cas9 protein, Cas9 nickases (nCas9), which lack the capacity to cleave DNA, but instead are fused to bacterial deaminases to substitute single nucleotides. (see Komor AC, Kim YB, Packer MS, Zuris JA, Liu DR. Programmable editing of a target base in genomic DNA without double-stranded DNA cleavage. Nature (2016) 533(7603):420-4) (see also for CAR-T cell engineering: Webber BR, Lonetree C-I, Kluesner MG, Johnson MJ, Pomeroy EJ, Diers MD, et al. Highly efficient multiplex human T cell engineering without double-strand breaks using Cas9 base editors. Nat Commun (2019) 10(1):5222.)). It is an attractive strategy to knock out genes or correct pathogenic single nucleotide polymorphisms without the need for genomic double-strand breaks or co-delivering a homology-directed repair template (HDRT). For nCas9-mediated base editing, single-strand DNA point mutations are subsequently resolved in the process of replication and are used to deliberately alter the codon sequence including insertion of premature STOP codons. Base editing requires three elements. Broadly, a Cas nickase or Cas fused to a deaminase that makes the edit, a gRNA targeting Cas to a specific locus, and a target base for editing within the editing window specified by the Cas protein.

Recently CRISPR-free all-protein base editors have been described that are also suitable (Mok BY, Kotrys AV, Raguram A, Huang TP, Mootha VK, Liu DR. Crispr-free base editors with enhanced activity and expanded targeting scope in mitochondrial and nuclear DNA. Nat Biotechnol (2022) 40:1378-87).

Cytosine base editors (CBEs) that convert C·G to T·A base pairs (Komor A.C., Kim Y.B., Packer M.S., Zuris J.A., Liu D.R. Programmable editing of a target base in genomic DNA without double-stranded DNA cleavage. Nature. 2016;533:420-424) and adenine base editors (ABEs) that convert A·T to G·C base pairs (Gaudelli N.M., Komor A.C., Rees H.A., Packer M.S., Badran A.H., Bryson D.I., Liu D.R. Programmable base editing of A • T to G • C in genomic DNA without DNA cleavage. Nature. 2017;551:464-471) are thus relevant tools to knock-out genes according to the present invention (see Knipping F, Newby GA, Eide CR, et al. Disruption of HIV-1 co-receptors CCR5 and CXCR4 in primary human T cells and hematopoietic stem and progenitor cells using base editing. Mol Ther. 2022;30(1):130-144)

Prime editing links nCas9 to a reverse transcriptase, both of which are guided to a desired genomic locus by a customizable prime editing guide RNA (pegRNA). It is a versatile and precise genome editing method that directly writes new genetic information into a specified DNA site using a catalytically impaired Cas9 fused to an engineered reverse transcriptase, programmed with a prime editing guide RNA (pegRNA) that both specifies the target site and encodes the desired edit. (Anzalone AV, Randolph PB, Davis JR, Sousa AA, Koblan LW, Levy JM, et al.. Search-and-Replace genome editing without double-strand breaks or donor DNA. Nature (2019) 576(7785):149-57; Nelson JW, Randolph PB, Shen SP, Everette KA, Chen PJ, Anzalone AV, et al.. Engineered pegrnas improve prime editing efficiency. Nat Biotechnol (2022) 40(3):402-10). After binding to the target region, prime editing allows for single base exchanges as well as insertions and deletions of synthetic DNA sequences of limited length. Prime editing notably allows to incorporate DNA sequences of up to 5kb length using a platform dubbed twin prime editing (TwinPE) (Anzalone AV, Gao XD, Podracky CJ, Nelson AT, Koblan LW, Raguram A, et al.. Programmable deletion, replacement, integration and inversion of Large DNA sequences with twin prime editing. Nat Biotechnol (2022) 40(5):731-40).

Administration of a Suv39h1 inhibitor according to the present invention can be performed *in vitro, ex vivo,* or *in vivo* in an individual. Selection of the suitable inhibitor will of course depend on the type of administration. In some embodiments, Suv39h1 gene inhibitors such as agents that hybridize or bind to the *Suv39h1* gene or its regulatory elements can be favored (such as aptamers that block or inhibit *Suv39h1* expression or activity and nucleic acid molecules that block transcription or translation, such as antisense molecules complementary to *Suv39h1* and various RNA interfering agents (such as a small interfering RNAs (siRNAs), small hairpin RNAs (shRNAs), microRNAs (miRNAs), piwiRNAs (piRNAs)); long non-coding RNAs (IncRNAs), ribozymes and combinations thereof).

### Immune response and vaccine or immunogenic compositions

The present invention relates to the use of an inhibitor of H3K9 lysine methyl transferase (KMT), notably an inhibitor of Suv39h1 to improve the immune response, in particular the cell mediated immune response to one or more antigen. An inhibitor of H3K9 lysine methyl transferase (KMT), notably an inhibitor of Suv39h1 can thus typically be used in combination with a pharmaceutical composition that elicits an immune response in an individual for the treatment and/or the prevention of a disease or a condition associated with the elevated expression of an antigen, typically an infectious disease or a tumor.

In some embodiments, the immune response is a cell-mediated immune response.

The present invention encompasses a medical preparation comprising:
- a first component that elicits an immune response in an individual
- a second component comprising an inhibitor of a H3K9 lysine methyl transferase (KMT);
wherein the first and second components can be administered to the individual sequentially in any order or simultaneously.

In some embodiments, the medical preparation is for treating and/or preventing a disease, disorder or condition associated with the expression or the elevated expression of an antigen, typically wherein the disease is an infectious disease or a tumor.

In some embodiments, the medical preparation prevents recall infections or tumor relapses, in more particular embodiments, the medical preparation prevents, reduces or delays tumor metastasis growth.

The present invention also encompasses a method of treating and/or preventing an infectious disease or a tumor in an individual, said method comprising the administration to the individual of a medical preparation comprising:
- a first component that elicits an immune response in the individual;
- a second component comprising an inhibitor of a H3K9 KMT;
wherein the first and second components can be administered sequentially in any order or simultaneously.

The present invention further encompasses a method of treating and/or preventing a disease, disorder or condition associated with the expression or the elevated expression of an antigen in an individual, optionally wherein the disease is an infectious disease or a neoplastic disease (tumor), said method comprising the administration to the individual of:
- a first component that elicits an immune response in the individual,
- a second component comprising an inhibitor of a H3K9 KMT;
wherein the first and second components can be administered sequentially in any order or simultaneously.

In some embodiments, the pharmaceutical composition that elicits an immune response comprises a modified immune cell that has been engineered to express at it surface one or more antigen receptors. In some embodiments, the immune cells express antigen-specific receptors on the surface. The cells thus may comprise one or more nucleic acids that encode one or more antigen-specific receptors, optionally operably linked to a heterologous regulatory control sequence. Typically, such antigen-specific receptors bind the target antigen with a Kd binding affinity of 10⁻⁶M or less, 10⁻⁷ M or less, 10⁻⁸ M or less, 10⁻⁹ M or less, 10⁻¹⁰ M or less, or 10⁻¹¹ M or less (lower numbers indicating greater binding affinity). In some embodiments, the antigen-binding domain binds a target antigen with a KD affinity of about 1 × 10⁻⁷ or less, about 5 × 10⁻⁸ or less, about 1 × 10⁻⁸ or less, about 5 × 10⁻⁹ or less, about 1 × 10⁻⁹ or less, about 5 × 10⁻¹⁰ or less, about 1 × 10⁻¹⁰ or less, about 5 × 10⁻¹¹ or less, about 1 × 10⁻¹¹ or less, about 5 × 10⁻¹² or less, or about 1 × 10⁻¹² or less. Typically, the nucleic acids are exogenous (e.g. heterologous), (i.e., for example which are not ordinarily found in the cell being engineered and/or in the organism from which such cell is derived). In some embodiments, the nucleic acids are not naturally occurring, including chimeric combinations of nucleic acids encoding various domains from multiple different cell types. The nucleic acids and their regulatory control sequences are typically heterologous. For example, the nucleic acid encoding the antigen-specific receptor may be heterologous to the immune cell and operatively linked to an endogenous promoter of the T-cell receptor such that its expression is under control of the endogenous promoter. In some embodiments, the nucleic acid encoding a modified TCR or CAR is operatively linked to an endogenous TRAC promoter. Among the antigen-specific receptors as per the disclosure are recombinant modified T cell receptors (TCRs) and components thereof, as well as functional non-TCR antigen-specific receptors, such as chimeric antigen receptors (CAR).Engineered immune cells and engineered antigen receptors suited to the present invention have been for example described in EP3359563B1, in international patent application publication numbers WO200014257, WO2013126726, WO2012/129514, WO2014031687, WO2013/166321, WO2013/071154, WO2013/123061, WO2023126458A1, WO2021013950A1, U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen-specific receptors include a CAR as described in U.S. Patent No.: 7,446,190, and those described in International Patent Application Publication No.: WO/2014055668 A1.

In some embodiments, the pharmaceutical composition that elicits an immune response is an immunogenic or a vaccine composition.

The present invention also encompasses a method of stimulating an immune response against an infectious disease or a tumor in an individual, said method comprising administering a vaccination regimen and an immune stimulation regimen to the individual; wherein the immune stimulation regimen comprises administration of an inhibitor of a H3K9 KMT inhibitor, preferably a Suv39H1 inhibitor; wherein the immune stimulation regimen and the vaccination regimen are administered sequentially in any order or simultaneously.

The present invention also encompasses a method of raising, stimulating or enhancing an immune response to one or more antigen(s) in an individual, comprising administering to the individual an effective amount of an inhibitor of a H3K9 KMT, notably an inhibitor of Suv39h1.

Typically, the immune response is a cell-mediated immune response, notably a T cell mediated immune response, more particularly the immune response comprises the generation, activation or expansion of antigen-specific T cells and/or the induction or cytolotic event or activity mediated by immune cell and in particular T cell such as CD8 T cells. The immune response also encompasses a response to a vaccination regimen (e.g., the administration of a vaccine or immunogenic composition).

Inhibitors of H3K9 KMT (KMT inhibitor), in particular inhibitors of Suv39h1 suitable with the present invention have been described above. Typically, a KMT inhibitor usable according to the present invention significantly increases the sensitivity and initial response of naive T cells, in particular at low antigen load and/or at low antigen affinity.

In some embodiments such effect can be assessed as illustrated in the examples of the present application (e.g. incubating naive antigen specific normal CD8+ T cells (such as OTI CD8+ T cells) or deficient for a H3K9 KMT (such as Suv39h1) expressing a TCR against a given antigen (e.g., from wild-type and Suv39h1KO OTI mice, e.g. T cell expressing a specific TCR recognizing an OVA antigen in combination with CMH I) (typically around 1x104) for 4 to 48h with antigen presenting cells (typically around 2.5x104) (e.g., splenocytes from CD3-KO mice) preloaded with antigenic peptides having variable affinity for the said TCR (such as the exemplified peptide).

In some embodiments a KMT inhibitor increases expansion and/or recall response of central memory T cells (e.g., CD44+CD62L+ T cells) (typically in the absence of inflammation as illustrated in figure 3), notably in lymphoid organs (e.g., spleen and/or lymph nodes). In some embodiments a KMT inhibitor increases the number of effector memory T cells (e.g., CD44+CD62L-) (typically in the absence of inflammation as illustrated in figure 3), notably in tissues (e.g., liver).

As per the present invention, an immune response is a protective immune response, e.g., reducing partially or completely the severity of one or more symptoms and/or time over which one or more symptoms are experienced by a subject, reducing the likelihood of developing an established infection after challenge, preventing tumor relapses, metastasis, secondary infections, and/or slowing progression of an associated illness (e.g. extending survival). Immune response may include a humoral immune response and/or a cell-mediated immune response.

Typically, the immune response according to the present invention comprises a cell-mediated immune response, notably a T cell mediated immune response. Typically, the generation of a T cell mediated immune response includes the generation of antigen-specific T cells, in other words T lymphocytes comprising a T cell receptor (TCR) that is directed against an antigen. By recognized an antigen, it is intended that the TCR binds to the said antigen in combination with an HLA molecule. The said antigen is typically responsible for the induction of the immune response, once presented as the surface of antigen presenting cells in combination with an HLA molecule. Said antigen can typically be included in an immunogenic or vaccine composition.

### Antigens or immunogens

An immunogen refers to a molecule that is capable of eliciting an immune response by an organism's immune system, whereas an antigen refers to a molecule that is capable of binding to the product of that immune response. So, an immunogen is necessarily an antigen, but an antigen may not necessarily be an immunogen. Typically, according to the invention, an antigen is an immunogen.

The term "epitope" or "antigenic determinant" typically refers to the part of an antigen which is recognized by the adaptive immune system. An "antigen" is a substance, which is capable of being recognized (typically via its epitope(s)) by the immune system, preferably by the adaptive immune system, and which is capable of eliciting an antigen-specific immune response, e.g. by formation of antibodies and/or antigen-specific T cells as part of an adaptive immune response.

The antigen may be a whole-organism, a protein/polypeptide, a polysaccharide, a peptide, a protein-polysaccharide conjugate, or a hapten capable of raising an immune response in a human or an animal, each of these types of antigen, or any combination of two or more thereof, being specifically contemplated as a possible antigen in specific embodiments of the composition. The terms "protein" and "polypeptide" are herein synonymous and interchangeable.

Typically, an antigen is or comprises a peptide or protein, which canbe presented to (antigen-specific) T-cells on MHC surface molecules by antigen-presenting cells. In some embodiments, an antigen is the product of translation of a provided nucleic acid molecule, preferably an epitope-encoding RNA. Fragments or variants of an antigen (such as a peptide or a protein) comprising at least one epitope are understood as antigens.

Such a fragment comprising or consisting of an epitope can typically comprise from about 5 to about 20 amino acids. Epitopes can be distinguished in T cell epitopes and B cell epitopes. T cell epitopes or parts of the proteins in the context of the present invention can comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC surface molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form. The term "epitope" includes "conformational" (or "discontinuous") epitopes, which are composed of discontinuous sequences of the amino acids of the antigen but are brought together in the three-dimensional structure, and "linear" epitopes, which are formed by a continuous sequence of amino acids from the antigen.

The immune response may be raised against a pathogen, such as for example, viruses, bacteria, parasites or fungus. That is, in one embodiment, the antigen is derived from a human pathogen. In some embodiments, the antigen is derived from a human pathogen selected from the group consisting of bacteria, virus, fungi, parasitic microorganisms and multicellular parasites. In some embodiments, the antigen is derived from a combination of two or more bacteria, virus, fungi, parasitic microorganisms and multicellular parasites.

Alternatively, the antigen may be derived from a tumor cell (e.g., the antigen may be a tumor- associated antigen or a tumor specific antigen), and the vaccine composition may be useful for the treatment or the prevention of cancers.

The expression, "an antigen derived from an organism" encompasses, in particular, the organism as a whole (whole-organisms, such as for example a whole-virus or a whole-bacterium), or one or more molecules only from the organism. The antigen may be the naturally occurring whole-organism, and the one or more molecules for instance one or more polypeptides, from the organism may be isolated and purified from such naturally occurring whole-organism.

Alternatively, the antigen may be artificially produced, for example, using recombinant technology or using chemical synthesis. Such recombinant antigens may be in a wild-type form, i.e. their nucleotide sequence, or amino acid sequence, is identical to the sequence of the corresponding antigens derived from the naturally occurring whole-organism. Alternatively, said recombinant antigens may advantageously comprise one or more mutations, i.e. their nucleotide sequence, or amino acid sequence, comprises one or more mutations, as compared with the sequence of the corresponding wild type antigens. Whole-organisms may be live attenuated or killed/inactivated. Inactivation processes using physical and/or chemical means are known to the skilled person. Such recombinant/modified/designed antigens are considered to be within the definition of "derived from" an organism within the context of the present disclosure.

In some embodiments, the antigen comprises at least one B or T cell epitope, an antigen comprises B and T cell epitopes. The elicited immune response may be an antigen specific B cell response which produces neutralizing antibodies. The elicited immune response may be an antigen specific T cell response, which may be a systemic and/or a local response. The antigen specific T cell response may comprise a CD4+ T cell response, such as a response involving CD4+ T cells expressing a plurality of cytokines, e.g. IFN gamma, TNF alpha and/or IL2. Alternatively, or additionally, the antigen specific T cell response comprises a CD8+ T cell response, such as a response involving CD8+ T cells expressing a plurality of cytokines, e.g., IFN gamma, TNF alpha and/or IL2.

Suitably the encoded antigen contains 3000 residues or fewer, especially 2000 residues or fewer, in particular 1500 residues or fewer. The encoded antigen may contain 1000 residues or fewer, 800 residues or fewer, 600 residues or fewer, 400 residues or fewer or 200 residues or fewer. Suitably the antigen contains 50 residues or more, especially 100 residues or more, in particular 150 residues or more. Suitably the antigen contains 50 to 3000 residues, especially 100 to 1500 residues, in particular 200 to 1000 residues.

An antigen according to the present invention is typically associated with a disease or disorder such as an infection or a tumor. By associated with a disease it is typically intended that the expression level of the said antigen is increased in cells from a subject suffering from said disease or disorder. In some cases, the expression of the said antigen in an individual may be cell specific, typically in case of a tumor, where the expression of a tumor antigen is usually tumor tissue specific.

### Virus, bacterial and parasite antigens

An antigen according to the present invention, notably an antigen used in an immunogenic or vaccine composition may derive from a virus. Accordingly, in particular embodiments, the antigen derives from a virus. In particular, the antigen may be a whole-virus. The whole virus may be live attenuated or killed/inactivated. Alternatively, the antigen may be a polypeptide derived from a virus.

Suitable viruses are from the families Orthomyxoviridae, such as for instance influenza viruses, Paramyxoviridae, such as for instance respiratory syncytial viruses (RSV), mumps virus or measles, Togaviridae, such as for instance rubella virus, Papovaviridae, such as for instance human papillomaviruses (HPV), Herpesviridae, such as for instance herpes simplex viruses (HSV), human cytomegaloviruses (HCMV), Epstein-Barr viruses (EBV), or varicella-zoster viruses (VZV), Picornaviridae, such as for instance enteroviruses, rhinoviruses, polioviruses, Fiaviviridae, such as for instance Dengue viruses or hepatitis C virus (HCV), Hepadnaviridae, such as for instance hepatitis B virus (HBV), Retroviridae, such as for instance human immunodeficiency viruses (HIV), Reoviridae, such as for example rotaviruses, Rhabdoviridae, such as for instance rabies viruses, or Flloviridae, such as for example Ebola virus. In one embodiment, the antigen used either in or with the adjuvant composition derives from a virus selected from the group consisting of influenza virus, RSV, HPV, measles, rubella virus, mumps virus, HCMV, VZV, Dengue virus, poliovirus, HIV, HBV, Ebola virus and rotavirus, or any combination of two or more thereof.

In a particular embodiment, the antigen derives from HCMV. Suitably, the HCMV antigen is the glycoprotein gB, which may lack the transmembrane domain (as disclosed in EP0802979), optionally in combination with one or more of the HCMV proteins pp65, IE1, pUL131, gL, gH, pUL128, and pUL130. Suitably, the HCMV antigen is a combination of gB, gL, gH, pUL131, pUL128 and pUL130. Alternatively, the HCMV antigen is a combination of gL, gH, pUL131, pUL128 and pUL130.

In some embodiments, the antigen is a varicella-zoster viruses (VZV) antigen. In one embodiment, the VZV antigen is a gE antigen. Suitably, the VZV antigen is the glycoprotein gE, which may be deleted from its transmembrane domain, as disclosed in EP0405867 B1. In one embodiment, the VZV antigen is a protein derived Varicella Zoster Virus which is one of gpl, gpll or g pill as defined on pages 5-7 of EP 0 405 867 B1 and is missing from 4-20 percent of the total amino acid residues of the full length glycoprotein at the carboxy terminal end.

In other embodiments, the antigen derives from RSV. Suitably, the RSV antigen is a polypeptide selected from the group consisting of the fusion protein (F), the attachment protein (G), the matrix protein (M2) and the nucleoprotein (N). Particularly suitable as an RSV polypeptide antigen to be included in or administered with the adjuvant composition are conformationally constrained F polypeptides. Conformationally constrained F polypeptides have previously been described in both the prefusion (PreF) and postfusion (PostF) conformations. Exemplary F protein antigens conformationally constrained in the prefusion conformation have been described in the art and are disclosed in detail in e.g. WO 09/079796, WO 10/149745, WO 11/008974 and WO 12/158613. Likewise, F protein antigens conformationally constrained in the postfusion conformation are also well known in the art and can be used in or administered with the adjuvant composition. Examples of postfusion conformationally constrained F protein polypeptides are disclosed in details in e.g. WO 11/008974, and Swanson et al. (PNAS, 2011, Vol. 108: 9619-9624). In particular embodiments, the adjuvant composition comprises an antigen polypeptide derived from RSV selected from the group consisting of: F protein, preF protein, N protein and M2 protein.

In a further embodiments, the antigen derives from HBV. Suitably, the antigen is the Hepatitis B surface antigen (HBS)

In some embodiments, the antigen is derived from an RNA virus. In one embodiment, the antigen is derived from a coronavirus, particularly from SARS-CoV-2.

A plurality of antigens maybe encoded. Consequently, in some embodiments the antigen is derived from at least one coronavirus, for example from SARS-CoV-2. In some embodiments the antigen is derived from more than one coronavirus (such as 2, 3, 4 or 5), for example from SARS- CoV-2 (such as a plurality of SARS-CoV-2 variant antigens).

SARS-CoV-2 makes use of a densely glycosylated spike (S) protein to gain entry into host cells. In coronaviruses, the S protein is a trimeric class I fusion protein which exists in a metastable prefusion conformation that undergoes a substantial structural rearrangement to fuse the viral membrane with the host cell membrane (Li F. Structure, Function, and Evolution of Coronavirus Spike Proteins. Annu Rev Virol. 2016 Sep 29;3(1):237-261; Bosch BJ, van der Zee R, de Haan CA, Rottier P The coronavirus spike protein is a class I virus fusion protein: structural and functional characterization of the fusion core complex. J Virol. 2003 Aug;77(16):8801-II.).

A coronavirus protein of use is a fragment or variant of a native coronavirus protein which is capable of eliciting neutralising antibodies and/or a T cell response (such as a CD4 or CD8 T cell response) to a coronavirus, suitably a protective immune response.

A SARS-CoV-2 S protein of use comprises, such as consists of, a fragment or variant of a native SARS-CoV-2 S protein which is capable of eliciting neutralising antibodies and/or a T cell response (such as a CD4 or CD8 T cell response) to SARS-CoV-2, suitably a protective immune response.

In one embodiment, the antigen is a human cytomegalovirus (CMV) antigen.

In one embodiment, the antigen is a Zika virus antigen.

In one embodiment, the antigen is a human parainfluenza virus (PIV) antigen, such as a human PIV type 3 antigen.

In one embodiment the antigen is a human metapneumovirus (hMPV) antigen.

In one embodiment the antigen is a respiratory syncytial virus (RSV) antigen.

In one embodiment the antigen is an influenza virus antigen, such as a hemagglutinin or a neuraminidase.

In one embodiment the antigen is an Epstein-Barr virus (EBV) antigen.

In one embodiment, the antigen is an Herpes simplex virus (HSV) antigen, such as a gE and/or a gl antigen. Suitable antigens are disclosed in WO 2021/013798.

The antigen may also derive from a bacterium. Accordingly, in particular embodiments, the antigen derives from a bacterium. In one embodiment, the antigen is from a bacterium selected from the group consisting of: B. pertussis, S. Pneumoniae, and N. meningitidis, or any combination of two or more thereof.

The antigen may be a whole-bacterium and may be killed/inactivated or live attenuated. Particular whole-bacterium antigens for use in the present invention are Bordetella pertussis. In one embodiment, the B. pertussis antigen is the whole-bacterium (Pw antigen), optionally in combination with tetanus toxoid (T) and/or diphtheria toxoid (D).

The antigen may also derive from a parasite. In some embodiments, the antigen may derive from parasites causing Malaria. Accordingly, in some embodiments, the antigen is derived from parasites that cause Malaria, such as for example, Plasmodium falciparum or Plasmodium vivax. Suitably, the Plasmodium falciparum-derived antigen is RTS,S.

### Tumor antigens

In some embodiments, the antigen is a tumor antigen (also named cancer or neoplastic antigens). Tumor antigens recognized by T lymphocytes are central to the efficacy of cancer vaccines. The ideal antigen for a cancer vaccine should be highly immunogenic, explicitly expressed in all cancer cells (not in normal cells or at low level, i.e. can be a TS or a TAA with tissue differentiation) and optionally necessary for the survival of cancer cells.

The cancer may be a solid cancer or a "liquid tumor" such as cancers affecting the blood, bone marrow and lymphoid system, also known as tumors of the hematopoietic and lymphoid tissues, which notably include leukemia and lymphoma. Liquid tumors include for example acute myelogenous leukemia (AML), chronic myelogenous leukemia (CML), acute lymphocytic leukemia (ALL), and chronic lymphocytic leukemia (CLL), (including various lymphomas such as mantle cell lymphoma, non-Hodgkins lymphoma (NHL), adenoma, squamous cell carcinoma, laryngeal carcinoma, gallbladder and bile duct cancers, cancers of the retina such as retinoblastoma).

Solid cancers notably include cancers affecting one of the organs selected from the group consisting of colon, rectum, skin, endometrium, lung (including non-small cell lung carcinoma), uterus, bones (such as Osteosarcoma, Chondrosarcomas, Ewing's sarcoma, Fibrosarcomas, Giant cell tumors, Adamantinomas, and Chordomas), liver, kidney, esophagus, stomach, bladder, pancreas, cervix, brain (such as Meningiomas, Glioblastomas, Lower-Grade Astrocytomas, Oligodendrocytomas, Pituitary Tumors, Schwannomas, and Metastatic brain cancers), ovary, breast, head and neck region, testis, prostate and the thyroid gland.

In some embodiments, the tumor antigen is a tumor associated antigen (TAA) or a tumor specific antigen (TSA).

TAAs are typically shared tumor-shared antigens (archetypal examples are HER2 and human telomerase transcriptase cancer mutants) and include self-antigens" such as differentiated antigens, overexpressed antigens, cancer-testicular antigens and viral-original "non-self" antigens. TAAs typically include tissue differentiated antigens are expressed by tumor cells and normal cells of the same tissue origin as tumor cells, such as prostate-specific antigen (PSA) expressed in the prostate gland and prostate cancer, and melanoma antigens tyrosinase expressed by normal melanocytes, and melanoma cells. Cancer-testis antigens (CTAs) are thought to provide high tumour specificity, as they are not expressed in normal adult tissues, except by germline and trophoblastic cells, but are highly expressed across cancers. Typical CTAs include the melanoma-associated antigen (MAGE) family (such as MAGE-A3), sarcoma antigen 1 (SAGE1), WT1 and cancer-testis antigen 1 (CTAG1A; commonly known as NY-ESO-1).

TSAs are a class of proteins or peptides specifically expressed in tumor cells. TSAs are often referred to as neoantigens. In some embodiments TSAs drive oncogenesis. TSAs viral antigens, such the Epstein-Barr virus encoded antigens (including latent membrane proteins (LMP1 and LMP2), which are expressed in various tumors or HPV E6 and E7 antigens. In some embodiments TSA further include overexpressed mutant self proteins , such as EGFRvIII which is a constitutively active, somatically mutated EGFR variant

In some embodiments, the antigen is a universal tumor antigen. The term "universal tumor antigen" refers to an immunogenic molecule, such as a protein, that is, generally, expressed at a higher level in tumor cells than in non-tumor cells and also is expressed in tumors of different origins. In some embodiments, the universal tumor antigen is expressed in more than 30%, 40%, 50%, 60%, 70%, 75%, 80%, 85%, 90% or more of human cancers. In some embodiments, the universal tumor antigen is expressed in at least three, at least four, at least five, at least six, at least seven, at least eight or more different types of tumors. In some cases, the universal tumor antigen may be expressed in non-tumor cells, such as normal cells, but at lower levels than it is expressed in tumor cells. In some cases, the universal tumor antigen is not expressed at all in non-tumor cells, such as not expressed in normal cells. Exemplary universal tumor antigens include, for example, human telomerase reverse transcriptase (hTERT), survivin, mouse double minute 2 homolog (MDM2), cytochrome P450 1B1 (CYP1B), HER2/neu, p95HER2, Wilms' tumor gene 1 (WT1), livin, alphafetoprotein (AFP), carcinoembryonic antigen (CEA), mucin 16 (MUC16), MUC1, prostate-specific membrane antigen (PSMA), p53 or cyclin (DI). In some aspects, the antigen is expressed on multiple myeloma, such as CD38, CD138, and/or CS-1. Other exemplary multiple myeloma antigens include CD56, TIM-3, CD33, CD123, and/or CD44. Typical antigen examples include include orphan tyrosine kinase receptor ROR1, tEGFR, Her2, p95HER2, LI-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, EPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, Ll-cell adhesion molecule, MAGE-A1, mesothelin, MUC1, MUC16, PSCA, NKG2D Ligands, NY-ESO-1, MART-1, gplOO, oncofetal antigen, ROR1, TAG72, VEGF-R2, carcinoembryonic antigen (CEA), prostate specific antigen, PSMA, Her2/neu, p95HER2, estrogen receptor, progesterone receptor, ephrinB2, CD 123, CS-1, c-Met, GD-2, and MAGE A3, CE7, Wilms Tumor 1 (WT-1), a cyclin, such as cyclin Al (CCNA1), CLDN6, gp100, melan1, p53, and/or biotinylated molecules, and/or molecules expressed by HIV, HCV, HBV or other pathogens.

In some embodiments one or more antigens can be selected from the following list (Gene Name (Protein Accession No. Of UniProt, RefSeq or Genbank)): 1A01_HLA-AIm (P30443); 1A02 (P01892); 5T4 (Q13641); ACRBP (Q8NEB7); AFP (P02771); AKAP4 (Q5JQC9); alpha-actinin_{-_}4/m (B4DSX0); alpha-actinin-_4/m (B4E337); alpha-actinin-_4/m (043707); alpha-methylacyl-coenzyme_A_racemase (A0A024RE16); alpha-methylacyl-coenzyme_A_racemase (A8KAC3); ANDR (P10275); ART-4 (Q9ULX3); ARTC1/m (P52961); AURKB (Q96GD4); B2MG (P61769); B3GN5 (Q9BYG0); B4GN1 (Q00973); B7H4 (Q7Z7D3); BAGE-1 (Q13072); BASI (P35613); BCL-2 (A9QXG9); bcr/abl (A9UEZ4); bcr/abl (A9UEZ7); bcr/abl (A9UEZ8); bcr/abl (A9UEZ9); bcr/abl (A9UF00); bcr/abl (A9UF01); bcr/abl (A9UF03); bcr/abl (A9UF04); bcr/abl (A9UF05); bcr/abl (A9UF06); bcr/abl (A9UF08); beta-catenin/m (P35222); beta-catenin/m (Q8WYA6); BING-4 (015213); BIRC7 (Q96CA5); BRCA1/m (A0A024R1V0); BRCA1/m (A0A024R1V7); BRCA1/m (A0A024R1Z8); BRCA1/m (A0A068BFX7); BRCA1/m (C6YB45); BRCA1/m (C6YB47); BRCA1/m (G3XAC3); BY55 (095971); calreticulin (B4DHR1); calreticulin (B4E2Y9); calreticulin (P27797); calreticulin (Q96L12); CAMEL (095987); CASP-8/m (Q14790); CASPA (Q92851-4); cathepsin_B (A0A024R374); cathepsin_B (P07858); cathepsin_L (A0A024R276); cathepsin_L (P07711); cathepsin_L (Q9HBQ7); CD1A (P06126); CD1B (P29016); CD1C (P29017); CD1D (P15813); CD1E (P15812); CD20 (P11836); CD22 (O60926); CD22 (P20273); CD22 (QOEAF5); CD276 (Q5ZPR3); CD33 (B4DF51); CD33 (P20138); CD33 (Q546G0); CD3E (P07766); CD3Z (P20963); CD44_Isoform_1 (P16070); CD44_Isoform_6 (P16070-6); CD4 (P01730); CD52 (P31358); CD52 (Q6IBD0); CD52 (V9HWN9); CD55 (B1AP15); CD55 (D3DT85); CD55 (D3DT86); CD55 (P08174); CD56 (P13591); CD80 (A0N0P2); CD80 (P33681); CD86 (P42081); CD8A (P01732); CDC27/m (G5EA36); CDC27/m (P30260); CDE30 (P28908); CDK4/m (A0A024RBB6); CDK4/m (P11802); CDK4/m (Q6LC83); CDK4/m (Q96BE9); CDKN2A/m (D1LYX3); CDKN2A/m (G3XAG3); CDKN2A/m (K7PML8); CDKN2A/m (L8E941); CDKN2A/m (Q8N726); CEA (NP_004354); CEAM6 (P40199); CH3L2 (Q15782); CLCA2 (Q9UQC9); CML28 (Q9NQT4); CML66 (Q96RS6); COA-1/m (Q5T124); coactosin-like_protein (Q14019); collagen XXIII (L8EAS4); collagen XXIII (Q86Y22); COX-2 (Q6ZYK7); CP1B1 (Q16678); CSAG2 (Q9Y5P2-2); CSAG2 (Q9Y5P2); CT45A1 (Q5HYN5); CT55 (Q8WUE5); CT-_9/BRD6 (Q58F21); CTAG2_Isoform_LAGE-1A (075638-2); CTAG2_Isoform_LAGE-1B (O75638); CTCFL (Q8NI51); Cten (Q8IZW8); cyclin_B1 (P14635); cyclin_D1 (P24385); cyp-B (P23284); DAM-10 (P43366); DEP1A (Q5TB30); E7 (P03129); E7 (P06788); E7 (P17387); E7 (P06429); E7 (P27230); E7 (P24837); E7 (P21736); E7 (P26558); E7 (P36831); E7 (P36833); E7 (Q9QCZ1); E7 (Q81965); E7 (Q80956); EF1A2 (Q05639); EFTUD2/m (Q15029); EGFR (A0A0B4J1Y5); EGFR (E7BSV0); EGFR (L0R6G1); EGFR (P00533-2); EGFR (P00533); EGFR (Q147T7); EGFR (Q504U8); EGFR (Q8NDU8); EGLN3 (Q9H6Z9); ELF2/m (B7Z720); EMMPRIN (Q54A51); EpCam (P16422); EphA2 (P29317); EphA3 (P29320); EphA3 (Q6P4R6); ErbB3 (B3KWG5); ErbB3 (B4DGQ7); ERBB4 (Q15303); ERG (P11308); ETV6 (P41212); EWS (Q01844); EZH2 (F2YMM1); EZH2 (G3XAL2); EZH2 (L0R855); EZH2 (Q15910); EZH2 (S4S3R8); FABP7 (015540); FCGR3A (P08637); FGF5 (P12034); FGF5 (Q60518); FGFR2 (P21802); fibronectin (A0A024R516); fibronectin (A0A024RB01); fibronectin (A0A024RDT9); fibronectin (A0A024RDV5); fibronectin (A6NH44); fibronectin (A8K6A5); fibronectin (B2R627); fibronectin (B3KXM5); fibronectin (B4DIC5); fibronectin (B4DN21); fibronectin (B4DS98); fibronectin (B4DTH2); fibronectin (B4DTK1); fibronectin (B4DU16); fibronectin (B7Z3W5); fibronectin (B7Z939); fibronectin (G5E9X3); fibronectin (Q9H382); FOS (P01100); FOXP3 (Q9BZS1); FUT1 (P19526); G250 (Q16790); GAGE-1 (AAA82744); GAGE-2 (Q6NT46); GAGE-3 (Q13067); GAGE-4 (Q13068); GAGE-5 (Q13069); GAGE-6 (Q13070); GAGE7b (Q76087); GAGE-8_GAGE-2D (Q9UEU5); GASR (P32239); GnT-V (Q09328); GPC3 (I6QTG3); GPC3 (P51654); GPC3 (Q8IYG2); GPNMB/m (A0A024RA55); GPNMB/m (Q14956); GPNMB/m (Q8IXJ5); GPNMB/m (Q96F58); GRM3 (Q14832); HAGE (Q9NXZ2); hepsin (B2ZDQ2); hepsin (P05981); Her2/neu (B4DTR1); Her2/neu (L8E8G2); Her2/neu (P04626); Her2/neu (Q9UK79); HLA-A2/m (Q95387); HLA-A2/m (Q9MYF8); homeobox_NKX3.1 (Q99801); HOM-TES-85 (B2RBQ6); HOM-TES-85 (Q9P127); HPG1 Pubmed: 12543784); HS71A (PODMV8); HS71B (PODMV9); HST-2 (P10767); hTERT (094807); iCE (O00748); IF2B3 (O00425); IL10 (P22301); IL-13Ra2 (014627); IL2-RA (P01589); IL2-RB (P14784); IL2-RG (P31785); IL-5 (P05113); IMP3 (Q9NV31); ITA5 (P08648); ITB1 (P05556); ITB6 (P18564); kallikrein-2 (A0A024R4J4); kallikrein-2 (A0A024R4N3); kallikrein-2 (B0AZU9); kallikrein-2 (B4DU77); kallikrein-2 (P20151); kallikrein-2 (Q6T774); kallikrein-2 (Q6T775); kallikrein-4 (A0A0C4DFQ5); kallikrein-4 (Q5BQA0); kallikrein-4 (Q96PTO); kallikrein-4 (Q96PT1); kallikrein-4 (Q9Y5K2); KI20A (O95235); KIAA0205 (O92604); KIF2C (Q99661); KK-LC-1 (Q5H943); LDLR (P01130); LGMN (Q99538); LIRB2 (Q8N423); LY6K (Q17RY6); MAGA5 (P43359); MAGA8 (P43361); MAGAB (P43364); MAGE-A10 (A0A024RC14); MAGE-A12 (P43365); MAGE-A1 (P43355); MAGE-A2 (P43356); MAGE-A3 (P43357); MAGE-A4 (A0A024RC12); MAGE-A4 (P43358); MAGE-A4 (Q1RN33); MAGE-A6 (A8K072); MAGE-A6 (P43360); MAGE-A6 (Q6FH15); MAGE-A9 (P43362); MAGE-B10 (Q96LZ2); MAGE-B16 (A2A368); MAGE-B17 (A8MXT2); MAGE-_B1 (Q96TG1); MAGE-B2 (015479); MAGE-B3 (015480); MAGE-B4 (015481); MAGE-B5 (Q9BZ81); MAGE-B6 (Q8N7X4); MAGE-C1 (O60732); MAGE-C2 (Q9UBF1); MAGE-C3 (Q8TD91); MAGE-D1 (Q9Y5V3); MAGE-D2 (Q9UNF1); MAGE-D4 (Q96JG8); MAGE-_E1 (Q61AI7); MAGE-E1_(MAGE1) (Q9HCI5); MAGE-E2 (Q8TD90); MAGE-F1 (Q9HAY2); MAGE-H1 (Q9H213); MAGEL2 (Q9UJ55); mammaglobin_A (Q13296); mammaglobin_A (Q6NX70); MART-1/melan-A (Q16655); MART-2 (Q5VTY9); MC1_R (Q01726); MC1_R (Q1JUL4); MC1_R (Q1JUL6); MC1_R (Q1JUL8); MC1_R (Q1JUL9); MC1_R (Q1JUM0); MC1_R (Q1JUM2); MC1_R (Q1JUM3); MC1_R (Q1JUM4); MC1_R (Q1JUM5); MC1_R (Q6UR92); MC1_R (Q6UR94); MC1_R (Q6UR95); MC1_R (Q6UR96); MC1_R (Q6UR97); MC1_R (Q6UR98); MC1_R (Q6UR99); MC1_R (Q6URA0); MC1_R (Q86YW1); MC1_R (V9Q5S2); MC1_R (V9Q671); MC1_R (V9Q783); MC1_R (V9Q7F1); MC1_R (V9Q8N1); MC1_R (V9Q977); MC1_R (V9Q9P5); MC1_R (V9Q9R8); MC1_R (V9QAE0); MC1_R (V9QAR2); MC1_R (V9QAW3); MC1_R (V9QB02); MC1_R (V9QB58); MC1_R (V9QBY6); MC1_R (V9QC17); MC1_R (V9QC66); MC1_R (V9QCQ4); MC1_R (V9QDF4); MC1_R (V9QDN7); MC1_R (V9QDQ6); M-CSF (P09603); mesothelin (Q13421); MITF (O75030-8); MITF (O75030-9); MITF (O75030); MMP1_1 (B3KQS8); MMP7 (P09237); MUC-1 (AAA60019); MUM-1/m (NP_116242); MUM-2/m (Q9Y5R8); MYCN (P04198); MYO1A (Q9UBC5); MYO1B (O43795); MYO1C (000159); MYO1D (O94832); MYO1E (Q12965); MYO1F (000160); MYO1G (B0I1T2); MYO1H (NP_001094891); NA17 (Q3V5L5); NA88-A Pubmed: 10790436); Neo-PAP (Q9BWT3); NFYC/m (Q13952); NGEP (Q61WH7); NPM (P06748); NRCAM (Q92823); NSE (P09104); NUF2 (Q9BZD4); NY-ESO-1 (P78358); OA1 (P51810); OGT (015294); OS-9 (B4DH11); OS-9 (B4E321); OS-9 (B7Z8E7); OS-9 (Q13438); osteocalcin (P02818); osteopontin (A0A024RDE2); osteopontin (A0A024RDE6); osteopontin (A0A024RDJ0); osteopontin (B7Z351); osteopontin (F2YQ21); osteopontin (P10451); p53 (P04637); PAGE-4 (O60829); PAI-1 (P05121); PAI-2 (P05120); PAP (Q06141); PAP (Q53S56); PATE (Q8WXA2); PAX3 (P23760); PAX5 (O02548); PD1L1 (Q9NZQ7); PDCD1 (Q15116); PDEF (O95238); PECA1 (P16284); PGCB (Q96GW7); PGFRB (P09619); Pim-1_-Kinase (A0A024RD25); Pin-1 (015428); Pin-1 (Q13526); Pin-1 (Q49AR7); PLAC1 (Q9HBJ0); PMEL (P40967); PML (P29590); POTEF (A5A3E0); POTE (Q86YR6); PRAME (A0A024R1E6); PRAME (P78395); PRDX5/m (P30044); PRM2 (P04554); prostein (Q96JT2); proteinase-3 (D6CHE9); proteinase-3 (P24158); PSA (P55786); PSB9 (P28065); PSCA (D3DWI6); PSCA (O43653); PSGR (Q9H255); PSM (Q04609); PTPRC (NP_002829); RAB8A (P61006); RAGE-1 (Q9UQ07); RARA (P10276); RASH (P01112); RASK (P01116); RASN (P01111); RGS5 (015539); RHAMM/CD168 (O75330); RHOC (P08134); RSSA (P08865); RU1 (Q9UHJ3); RU2 (Q9UHG0); RUNX1 (Q01196); S-100 (V9HW39); SAGE (Q9NXZ1); SART-_1 (O43290); SART-2 (Q9UL01); SART-3 (Q15020); SEPR (012884); SERPINB5 (P36952); SIA7F (Q969X2); SIA8A (Q92185); SIAT9 (Q9UNP4); SIRT2/m (A0A024R0G8); SIRT2/m (Q8IXJ6); SOX10 (P56693); SP17 (Q15506); SPNXA (Q9NS26); SPXN3 (Q5MJ09); SSX-1 (Q16384); SSX-2 (Q16385); SSX3 (Q99909); SSX-4 (O60224); ST1A1 (P50225); STAG2 (Q8N3U4-2); STAMP-1 (Q8NFT2); STEAP-1 (A0A024RA63); STEAP-1 (Q9UHE8); Survivin-2B (015392-2); survivin (015392); SYCP1 (A0A024R012); SYCP1 (B7ZLS9); SYCP1 (Q15431); SYCP1 (Q3MHC4); SYT-SSX-1 (A4PIV7); SYT-SSX-1 (A4PIV8); SYT-SSX-2 (A4PIV9); SYT-SSX-2 (A4PIW0); TARP (Q0VGM3); TCRg (A2JGV3); TF2AA (P52655); TGFB1 (P01137); TGFR2 (P37173); TGM-4 (B2R7D1); TIE2 (Q02763); TKTL1 (P51854); TPI/m (P60174); TRGV11 (099601); TRGV9 (A4D1X2); TRGV9 (O99603); TRGV9 (O99604); TRPC1 (P48995); TRP-p8 (Q7Z2W7); TSG10 (Q9BZW7); TSPY1 (001534); TVC_TRGV3 (M13231.1); TX101 (Q9BY14-2); tyrosinase (A0A024DBG7); tyrosinase (L8B082); tyrosinase (L8B086); tyrosinase (L8B0B9); tyrosinase (O75767); tyrosinase (P14679); tyrosinase (U3M8NO); tyrosinase (U3M9D5); tyrosinase (U3M9J2); TYRP1 (P17643); TYRP2 (P40126); UPA (Q96NZ9); VEGFR1 (B5A924); WT1 (A0A0H5AUY0); WT1 (P19544); WT1 (Q06250) and XAGE1 (Q9HD64).

In some embodiments, the antigen is a personalized antigen used to design personalized antigen vaccines that includes variations of DNA and RNA extraction from tumor and germline tissue for exome and RNA sequencing as well as HLA typing. Somatic mutations are often selected that are present in the tumor and absent in the germ line, because they have low 'false discovery rate' and cause non-synonymous protein changes. Potentially immunogenic neo-epitopes (or neoantigens) are selected from among somatic mutations by in silico prediction of their binding to that patients' HLA alleles using approaches similar to the NetMHC algorithm. Highly expressed neo-epitopes are prioritized by assessment of tumoral RNA-sequencing data, from which generally up to 20 neo-epitopes are selected and good manufacturing practice (GMP)-grade neo-epitope peptides, RNA or viral vectors are produced.

In some embodiments the antigen is an "ignored neoantigen". characterized by a moderate level of neoepitope presentation that is below the threshold for priming of naive T cells but above the level for recognition by memory T cells. Ignored neoantigens typically require a vaccination to generate neoantigen presentation levels in the lymph node that allow priming. Said neoantigen should typically exhibit low expression and/or high-affinity MHC binding or high expression and/or low-affinity MHC binding such that T cells can be activated for effector functions in the tumour.

In some embodiments, the antigen may be a peptide derived from non-canonical open reading frame (ORFs). ORFs are termed "non-canonical" to distinguish them from CDSs included in reference gene annotation - i.e. Ensembl-GENCODE - even though their translation occurs through mechanisms of ribosome activity similar to that of CDSs. Throughout this text the term "non-canonical open reading frame" is therefore defined as any open reading frame that is not an annotated CDS, an in-frame extension or truncation (either N-terminal or C-terminal), or an in-frame intron retention of an annotated CDS. Thus, in some embodiments the antigen is from the grey or dark genome. Typical dark or grey antigen can be derived from non canonical ORFs induced by frameshit and/or spliced variants as well as from ORFs from transcripts annotated as IncRNAs, pseudogenes, TE, or retroviral elements in the genome. In some embodiments, the extracellular antigen binding domain binds to any of the tumor neoantigenic peptides disclosed in Int'l Pat. Pub. No. WO 2021/043804, incorporated by reference herein in its entirety. For example, the antigen-binding domain binds to any of the peptides of SEQ ID NO: 1-117 or to a neoantigenic peptide comprising at least 8, 9, 10, 11 or 12 amino acids that is encoded by a part of an open reading frame (ORF) of any of the fusion transcript sequences of any one of SEQ ID NO: 118-17492 of WO 2021/043804 or described in any of WO 2018/234367, WO 2022/189620, WO 2022/189626, and WO-2022/189639. Noncanonical cancer or tumor polypeptides include for example peptides derived from TE (transposable elements) such as in Bonté PE, Arribas YA, Merlotti A, et al. Single-cell RNA-seq-based proteogenomics identifies glioblastoma-specific transposable elements encoding HLA-I-presented peptides. Cell Rep. 2022;39(10):110916 ; LTR elements as in Attig J, Young GR, Hosie L, et al. LTR retroelement expansion of the human cancer transcriptome and immunopeptidome revealed by de novo transcript assembly. Genome Res. 2019;29(10):1578-1590 ; mid exon splicing such as in Kahles A, Lehmann KV, Toussaint NC, Hüser M, Stark SG, Sachsenberg T, Stegle O, Kohlbacher O, Sander C; Cancer Genome Atlas Research Network, et al. 2018. Comprehensive analysis of alternative splicing across tumors from 8,705 patients. Cancer Cell 34: 211-224.e6 ; intron retention such as in Smart AC, Margolis CA, Pimentel H, He MX, Miao D, Adeegbe D, Fugmann T, Wong KK, Van Allen EM. 2018. Intron retention is a source of neoepitopes in cancer. Nat Biotechnol 36: 1056-1058. 10.1038/nbt.4239, non-canonical splicing junctions such as described in Merlotti A, Sadacca B, Arribas YA, et al. Noncanonical splicing junctions between exons and transposable elements represent a source of immunogenic recurrent neo-antigens in patients with lung cancer. Sci Immunol. 2023;8(80):eabm6359 or in Shah NM, Jang HJ, Liang Y, et al. Pan-cancer analysis identifies tumor-specific antigens derived from transposable elements. Nat Genet. 2023;55(4):631-639). In some embodiments, the tumor antigen is selected from an intracellular peptide as described in WO 2022/189626 or WO 2022/189639. In these embodiments the antigen binding domain of the antigen receptor as herein described is typically a pMHC restricted antibody (e.g. a monoclonal antibody or mAbs) or a fragment thereof. In other embodiment, the tumor antigen is a surface antigen and notably a non-canonical surface polypeptide as defined for example in WO 2022/189620.

In some embodiments, the tumor antigen is a predefined antigen. Predefined antigens can be further classified by the frequency of expression across patient cohorts. Shared antigens are those expressed in a sufficient proportion of patients such that vaccinologists can target these patient groups (frequently within patient subsets of tumor types) using standard testing. Shared antigen vaccines can thus target both TSAs and TAAs.

In some embodiment the antigen derived from cancer mutations (somatic cancer variants) that can be shared or not. Shared cancer antigens include nucleotide cancer variants or KRAS, IDH1, TP53, H3-3A, JAK2, PIK3A, BRAF, CDK4, CDK12, NRAS, CTNNBA, GAS7. It may also include cancer indel variants of genes such as NPM1, CALR, TGFBR2 as well as cancer fusion genes such as BCR-ABL, DEK-CAN, SYT-SSX, PML-RARA, PAX-FKHR or ETV6-AML1 or cancer spliced variants.

Shared antigen vaccines are distinguished from personalized antigen vaccines in that the former can be assessed with standard testing such as cytology, immunohistochemistry and flow cytometry. Personalized antigens are unique to the vaccinated patient.

In some embodiment, the tumor antigen is a variant of a tumor antigen , wherein the tumor antigen wherein the antigen can be selected from one of the above mentioned tumor antigens.. A "variant" of said tumor antigen may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective naturally occurring (wild type) full-length tumor antigen, wherein the tumor antigen is an antigen selected from the antigens listed above.

In some embodiments, the antigen comprises a fragment of an antigen (notably a tumor antigen) as described above (or a variant thereof) preferably comprising at least one epitope of said antigen (or variant thereof), wherein the tumor antigen is an antigen selected from above listed antigens notably tumor antigens, or a variant thereof. A fragment of said tumor antigen (or variant thereof) may typically comprise or consist of an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective full-length tumor antigen (or variant thereof.

In some embodiments, the tumor antigens are anonymous antigens. Instead of being classified by their antigen identity, anonymous antigens can be classified by their method and location of APC loading. Anonymous antigen ex vivo vaccines are derived from excised tumor cells that are lysed and delivered to autologous APCs. Ex vivo antigen isolation may require extraction of tumor cells (excisional biopsy), processing raw tissue into a more antigenic form and colocalization with APCs. Injected tumor cells may be taken up and their antigens may be presented by APCs, or the tumor cells themselves may present their antigens to T cells. Anonymous antigen in situ vaccines are conceptually similar to ex vivo vaccines and bypass developing custom, GMP-compliant therapies for each patient.

In some embodiments, the antigen is expressed at low density, e.g., less than about 6,000 molecules of the target antigen per cell. In some embodiments, the antigen is expressed at an expression level of less than about 5,000 molecules, less than about 4,000 molecules, less than about 3,000 molecules, less than about 2,000 molecules, less than about 1,000 molecules, or less than about 500 molecules of the target antigen per cell. In some embodiments, the antigen is expressed at a density of less than about 2,000 molecules, such as e.g., less than about 1,800 molecules, less than about 1,600 molecules, less than about 1,400 molecules, less than about 1,200 molecules, less than about 1,000 molecules, less than about 800 molecules, less than about 600 molecules, less than about 400 molecules, less than about 200 molecules, or less than about 100 molecules of the target antigen per cell. In some embodiments, the antigen is expressed at a density of less than about 1,000 molecules, such as e.g., less than about 900 molecules, less than about 800 molecules, less than about 700 molecules, less than about 600 molecules, less than about 500 molecules, less than about 400 molecules, less than about 300 molecules, less than about 200 molecules, or less than about 100 molecules of the target antigen per cell. In some embodiments, the antigen is expressed at a density ranging from about 5,000 to about 100 molecules of the target antigen per cell, such as e.g., from about 5,000 to about 1,000 molecules, from about 4,000 to about 2,000 molecules, from about 3,000 to about 2,000 molecules, from about 4,000 to about 3,000 molecules, from about 3,000 to about 1,000 molecules, from about 2,000 to about 1,000 molecules, from about 1,000 to about 500 molecules, from about 500 to about 100 molecules of the target antigen per cell.

It is of course intended that more than one antigens as described above can be combined in a vaccine composition to achieve the ideal immune response. In some embodiments, a plurality or more than one, preferably 2 to 20, more preferably 2 to 20, most preferably 2 to 6 antigens, Said combinations typically comprise more than one different tumor antigens, or pathogenic antigens.

### Production of antigens

One of skill in the art will appreciate that there are a variety of ways in which to produce antigens as above defined. Typically, antigens can be produced either in vitro or in vivo.

They may be produced in vitro as peptides or polypeptides, which may then be formulated into a vaccine or immunogenic composition and administered to a subject. Such in vitro production may occur by a variety of methods known to one of skill in the art such as, for example, peptide synthesis or expression of a peptide/polypeptide from a DNA or RNA molecule in any of a variety of bacterial, eukaryotic, or viral recombinant expression systems, followed by purification of the expressed peptide/polypeptide.

Alternatively, antigens may be produced in vivo by introducing molecules (e.g., DNA, RNA, viral expression systems, and the like) encoding antigens into a subject, whereupon the encoded antigens are expressed.

### Vaccine or immunogenic compositions

A "vaccine" or immunogenic composition is typically understood to be a prophylactic or therapeutic material providing at least one antigen, typically at least one epitope of an antigen, preferably an immunogen. "Providing at least on epitope" means, for example, that the vaccine comprises the epitope or that the vaccine comprises a molecule that, e.g., codes for the epitope or a molecule comprising the epitope. For example a vaccine composition can comprise at least one epitope-encoding RNA comprising at least one coding sequence encoding an epitope (or an antigen or variant or fragment thereof comprising said epitope). Said epitope (or antigen or variant or fragment thereof comprising said epitope) is derived from an infectious pathogen or from a tumor or cancer cell, and triggers an adaptive immune response which preferably eliminates said infectious pathogen or tumor or cancer cell.

Typically as previously mentioned, in the medical preparation and treatment method as herein disclosed the first component that elicits an immune response, notably a cell-mediated immune response, notably a composition comprising one or more antigens as previously defined is provided as a pharmaceutical composition or as a vaccine also named herein immunogenic composition.

The (pharmaceutical) composition or vaccine provided herein may further comprise at least one pharmaceutically acceptable excipient, adjuvant or further component (e.g. additives, auxiliary substances, and the like).

According to some preferred embodiments, the (pharmaceutical) composition or the vaccine according to the invention comprises the combination of the present invention, wherein the at least one coding sequence of the at least one epitope-encoding RNA encodes at least one epitope of an antigen, notably at least one epitope of a tumor antigen or a fragment or variant of any one of these antigens as defined herein. The (pharmaceutical) composition or vaccine according to the invention may thus comprise at least one antigen, for example at least one epitope-encoding RNA, wherein the RNA encodes one specific epitope (or an antigen as defined herein or a variant or fragment thereof comprising said epitope), together with a KMT inhibitor as previously defined.

Alternatively, the (pharmaceutical) composition or vaccine of the present invention may comprise, in addition to the KMT inhibitor (notably the Suv39h1 inhibitor), at least one antigen, notably at least one epitope encoding nucleic acid, in particular at least one epitope-encoding RNA as defined herein, wherein said at least one epitope-encoding nucleic acid encodes at least two, three, four, five, six, seven, eight, nine, ten, eleven or twelve distinct epitopes (or antigens or variants or fragments thereof comprising said epitopes) as defined herein. In such embodiments, the (pharmaceutical) composition or vaccine preferably comprises several classes of the epitope-encoding nucleic acid (notably encoding RNA) as previously defined in combination with the KMT inhibitor, notably the Suv39h1 inhibitor, wherein each epitope-encoding nucleic acid species, notably RNA species encodes a distinct epitope (or an antigen or a variant or fragment thereof comprising said epitope) as defined herein.

In some embodiments, the epitope-encoding nucleic acid comprised in the (pharmaceutical) composition or vaccine is a bi- or multicistronic RNA, which encodes the at least two, three, four, five, six, seven, eight, nine, ten, eleven or twelve distinct epitopes (or antigens or variants or fragments thereof comprising said epitopes). Mixtures between these embodiments are also envisaged, such as compositions comprising more than one epitope-encoding RNA species, wherein at least one epitope-encoding RNA species may be monocistronic, while at least one other epitope-encoding RNA species may be bi- or multicistronic.

In some embodiments effectively activating a cellular immune response for a vaccine or immunogenic composition can be achieved by expressing the relevant one or more antigens in a vaccine or immunogenic composition in a non-pathogenic microorganism. Well-known examples of such microorganisms are Mycobacterium bovis BCG, Salmonella and Pseudomona (See, U.S. Patent No. 6,991,797, hereby incorporated by reference in its entirety).

In some embodiments a Poxvirus is used in the vaccine or immunogenic composition. These include orthopoxvirus, avipox, vaccinia, MV A, NYVAC, canarypox, ALVAC, fowlpox, TROVAC, etc. (See e.g., Verardiet ah, Hum Vaccin Immunother. 2012 Jul;8 (7):96I-70; and Moss, Vaccine. 2013: 31(39): 4220-4222). Poxvirus expression vectors were described in 1982 and quickly became widely used for vaccine development as well as research in numerous fields. Advantages of the vectors include simple construction, ability to accommodate large amounts of foreign DNA and high expression levels.

Vaccines or immunogenic compositions according to the present invention include inactivated vaccines, live-attenuated vaccines, messenger RNA (mRNA) vaccines, subunit, recombinant, polysaccharide, and conjugate vaccines, toxoid vaccines and viral vector vaccines.

Live-attenuated vaccines contain live pathogens from either a bacteria or a virus that have been "attenuated," or weakened (e.g., Measles, mumps, and rubella (MMR) vaccine, varicella (chickenpox) vaccine).

Inactivated vaccines take a live pathogen and inactivate or kill it. When the vaccine is then introduced to a human through a shot, for example, the inactivated pathogen is strong enough to create an immune response, however, is incapable of causing disease. Multiple doses are often needed in order to build up immunity and offer full protection (e.g., polio vaccine and influenza vaccine).

Subunit/antigen, recombinant, polysaccharide, and conjugate vaccine compositions are made from a piece of a pathogen, not the whole organism, so they do not contain any live pathogens.

Some important subunit vaccines are polysaccharide vaccines, conjugate vaccines, and protein-based vaccines (can be recombinant or not) (e.g., Haemophilus influenzae type B (Hib) vaccine (conjugate), pneumococcal vaccine (polysaccharide or conjugate), shingles vaccine (recombinant protein), hepatitis B (recombinant protein), acellular pertussis, MenACWY (conjugate)). Several approaches have been described to enhance the immunogenicity and stability of subunit vaccines, such as virus-like particle (VLP) vaccines and nanoparticle (NP) vaccines. VLP vaccines use platforms capable of producing particles that mimic the structure of authentic viruses. VLP vaccines can be produced by expressing antigenic proteins in a eukaryotic or prokaryotic system, resulting in the formation of particles with an inherent ability of the antigenic proteins to self-assemble. Alternatively, VLP vaccines can also be made by producing blank VLP templates and then chemically linking antigenic peptides onto the pre-formed particles.

Toxoid vaccines use inactivated toxins to target the toxic activity created by the bacteria, rather than targeting the bacteria itself (e.g., tetanus vaccine and diphteria vaccine).

Viral vector vaccines rely on antigen delivered on an unrelated, non-pathogenic viral backbone (rVSV-ZEBOV Ebola vaccine, AstraZeneca Covid-19 vaccine). A number of viruses have been developed as vectors for vaccine development, including poxviruses, adenoviruses, herpesviruses, arenaviruses, retroviruses, paramyxoviruses, and flaviviruses, among others (see Draper SJ, Heeney JL. Viruses as vaccine vectors for infectious diseases and cancer. Nat Rev Microbiol (2010) 8:62-73). Replication-defective and single-cycle viral vectors (such as single cycle AAV vectors) are particularly relevant as they have an increased safety profile and, in some cases, are still able to elicit a strong immune response (see for example the following reviews: Dudek T, Knipe DM. Replication-defective viruses as vaccines and vaccine vectors. Virology (2006) 344:230-9Barry M. Single-cycle adenovirus vectors in the current vaccine landscape. Expert Rev Vaccines (2018)).

Nucleic acid-based vaccine such as DNA or mRNA vaccines use genetic materials that code for the one or more antigens (e.g., the coronavirus's spike protein or a tumor antigen) to trigger an immune response. Recently approved COVID-19 mRNA vaccines include the Moderna's Spikevax and Pfizer's BNT162b2. Then, the expressed tumor antigens could induce immune killing effects against cancer cells. The nucleic acid vaccine usually induces strong MHC I mediated CD8 + T cell responses. Nucleic acid vaccines can simultaneously deliver multiple antigens to trigger humoral and cellular immunity. Additionally, nucleic acid vaccines can encode full-length tumor antigens, allowing APC to cross-present various epitopes or present several antigens simultaneously. More particularly, DNA vaccines use small DNA molecules (e.g., plasmids), while mRNA vaccines use the mRNA encoding the antigen. Typically, DNA vaccines need to enter the nucleus to synthesize the antigen, however, once plasmid DNA enters the nucleus, a single plasmid DNA can produce multiple mRNA copies. mRNA vaccines require a shorter route as mRNA just need to reach the cytoplasm for the synthesis of the antigen and mRNA have low risk of insertion and integration into the genome.

mRNA vaccines are mainly divided into non-replicating mRNA and self-amplifying RNA (SAM). Non-replacing mRNA is composed of 7-methylguanosine (m7G) 5' cap, 5'-untranslated region (5'-UTR), open reading frame (OFR), 3'-untranslated region (3'-UTR), and 3'poly(A) tail (see notably Pollard C, De Koker S, Saelens X, Vanham G, Grooten J. Challenges and advances towards the rational design of mRNA vaccines. Trends Mol Med. 2013;19(12):705-13). S elf-amplifying RNA (SAM) comprises 2 ORFs. One encodes the antigen, and te second encodes viral replication component, enabling long-lasting RNA amplification in cells. SAM is originated from alphavirus, and it replicates and magnifies in vivo to induce a persistent and efficient immune response. SAM may allow low doses of vaccination to produce large amounts of the antigen over a certain period.

Some of these vaccine compositions will be further detailed below in relation to cancer vaccines but it is to be understood that such vaccine can be applied to any other pathogen antigen as previously detailed.

Cancer vaccines can be described according to the type of antigen being introduced: whole tumor, tumor cells, protein, peptides (long or short), RNA or DNA (directly or virally); and the adjuvants with which antigen is introduced: carrier protein, cells (for example, dendritic cells (DCs)), proteins (for example, CD40 ligand (CD40L)) or chemicals (for example, oil-water emulsions and Toll-like receptor (TLR) agonists). Cancer vaccines have also been described as belonging to at least four categories: cell-based vaccines, peptide-based vaccines, viral-based vaccines, and nucleic acid-based vaccines. These categories typically include whole tumor cells, tumor lysates, gene-modified tumor cells, purified or recombinant peptide or protein TAA-based vaccines, nucleic acid-based vaccines, and genetically modified cancer cells expressing immunostimulatory cytokines.

### Cell-based (cancer) vaccines

The cell-based vaccine compositions can be divided into (tumor) cell-based vaccine composition and immune cell vaccine composition. Cancer vaccine technologies have been recently reviewed in Liu, J., Fu, M., Wang, M. et al. Cancer vaccines as promising immuno-therapeutics: platforms and current progress. J Hematol Oncol 15, 28 (2022). These technologies are summarized below.

Cell-based cancer vaccines are often prepared from whole cells or cell fragments, containing almost tumor antigens, inducing a broader antigen immune response (see Le DT, Pardoll DM, Jaffee EM. Cellular vaccine approaches. Cancer J. 2010;16(4):304-10 fr review). Immune cell-based is typically represented by antigen presenting cell (APC) vaccine and more particularly dendritic cell (DC) vaccine.

Whole tumor cell vaccines can be autologous or allogenic. Sipuleucel-T (Provenge^{®}) is an autologous active cellular immunotherapy with unknown precise mechanism of action; however, it is believed to induce T-cell responses through antigen presenting cells (APCs) to specifically target prostatic acid phosphatase (PAP). The active component of Sipuleucel-T is achieved by cultivation of patient-derived APCs with PA2024 protein containing a granulocyte-macrophage colony-stimulating factor (GM-CSF). PA2024-activated APCs are shown with elevated level of co-stimulatory molecules, which are then infused back to the patients

Six different approaches have been developed for the production of DC-based vaccines, which differ in the method used for antigen delivery or DC modifications (see Najafi S, Mortezaee K. Advances in dendritic cell vaccination therapy of cancer. Biomed Pharmacother. 2023;164:114954 summarized below): (1) DCs are not exposed to tumor antigens and instead used to form iDCs or in vitro DC maturation. This process is conducted via treatment with activating stimuli like interleukins. (2) *Ex vivo* pulsing of DCs either with tumor lysate, tumor antigens or tumor-derived mRNA, in which induced extracted DCs are treated with sources of antigens and then infused back to the recipient. (3) *In vivo* treatment of DCs with antigens, which aims at targeting the antigens to DCs and development of anticancer vaccines within the host body. (4) *In situ* stimulation of DCs, which is conducted through intratumoral administration of immunomodulators to induce development of anticancer immunity (see L. Hammerich, N. Bhardwaj, H.E. Kohrt, J.D. Brody ; In situ vaccination for the treatment of cancer. Immunotherapy, 8 (3) (2016), pp. 315-330) (5) Transfection of DCs with antigen-encoding vectors or tumor cells-extracted RNA. Transfected DCs express antigens and are active to stimulate T cells upon infusion to the recipient. (6) DC-derived exosomes as a source of antigens for immunization. This approach is considered as an alternative to DC adoptive therapy in which antigen-presenting exosomes are treated with antigens and then used to eradicate cancer cells in treated recipients. Among those approaches, pulsing DCs with tumor antigens or lysate and maturation using standard maturation cocktails is the approach that is commonly used. In typical personalized or semi-personnalized approaches, neoantigens in the tumor predicted to bind the major histocompatibility complex protein HLA-A*02:01 are identified for individual patients with a cancer. Dendritic cells from the patient are matured ex vivo, then incubated with neoantigens. The activated dendritic cells are then administered to the patient. In some embodiments, following administration of the cancer vaccine, robust T-cell immunity against the neoantigen is detectable.

Dendritic cells are a diverse population of antigen presenting cells found in a variety of lymphoid and non-lymphoid tissues. (See Liu, Cell 106:259-62 (2001); Steinman, Ann. Rev. Immunol. 9:271-96 (1991)) and include lymphoid dendritic cells of the spleen, Langerhans cells of the epidermis, and veiled cells in the blood circulation. Collectively, dendritic cells are classified as a group based on their morphology, high levels of surface MHC-class II expression, and absence of certain other surface markers expressed on T cells, B cells, monocytes, and natural killer cells. In particular, monocyte-derived dendritic cells (also referred to as monocytic dendritic cells) usually express CD 11c, CD80, CD86, and are HLA-DR+, but are CD 14".

Monocytic dendritic cell precursors (typically monocytes) are usually CD14+. Monocytic dendritic cell precursors can be obtained from any tissue where they reside, particularly lymphoid tissues such as the spleen, bone marrow, lymph nodes and thymus. Monocytic dendritic cell precursors can also be isolated from the peripheral blood , typically from peripheral blood mononuclear cells (PBMCs) (isolated from peripheral blood and identified as any blood cell with a round nucleus (i.e. lymphocytes, monocytes, natural killer cells (NK cells) or dendritic cells)). Umbilical cord blood is another source of monocytic dendritic cell precursors.

In certain embodiments, thenmonocytic dendritic cell precursors and/or immature dendritic cells can be isolated from a healthy subject or from a subject in need of immunostimulation, such as, for example, a cancer subject or other subject for whom cellular immunostimulation can be beneficial or desired (i.e., a subject having a bacterial or viral infection, and the like). Dendritic cell precursors and/or immature dendritic cells also can be obtained from an HLA-matched healthy individual for partial activation and administration to an HLA-matched subject in need of immunostimulation. Methods of isolating and modifying dendritic cell precursors can be found in U.S. Pat. Pub. No. 20060057120 which is herein incorporated by reference.

Dendritic cells can be administered directly into a tumor, into the tumor bed subsequent to surgical removal or resection of the tumor, peri-tumorily, into a draining lymph node in direct contact with the tumor, into a blood vessel or lymph duct leading into, or feeding a tumor or organ afflicted by the tumor, e.g., the portal vein or a pulmonary vein or artery, and the like. The administration of partially mature dendritic cells can be either simultaneous with or subsequent to other treatments for the tumor, such as chemotherapy or radiation therapy. Further, partially mature dendritic cells can be co - administered with another agent, which agent acts as an adjuvant to the maturation of the dendritic cell and/or the processing of antigen within the tumor or region near or adjacent to the tumor. In addition, the dendritic cells can also be formulated or compounded into a slow release matrix for implantation into a region in or around the tumor or tumor bed such that cells are slowly released into the tumor, or tumor bed, for contact with the tumor antigens.

Partially mature dendritic cells can also be administered by any means appropriate for the formulation and mode of administration. For example, the cells can be combined with a pharmaceutically acceptable carrier and administered with a syringe, a catheter, a cannula, and the like. As above, the cells can be formulated in a slow release matrix. When administered in this fashion, the formulation can be administered by a means appropriate for the matrix used. Other methods and modes of administration applicable to the present invention are well known to the skilled artisan.

Dendritic cell compositions can be used by themselves in the treatment of an individual. In addition, the compositions can be used in combination with any other method to treat a tumor. For example, the methods of the present invention can be used in combination with surgical resection of a tumor, chemotherapy (cytotoxic drugs, apoptotic agents, antibodies, and the like), radiation therapy, cryotherapy, brachytherapy, immune therapy (administration of antigen specific mature activated dendritic cells, NK cells, antibodies specific for tumor antigens, etc.), and the like. Any and all of these methods can also be used in any combination. Combination treatments can be concurrent or sequential and can be administered in any order as determined by the treating physician.

To make a dendritic cell vaccine for a subject, the subject's immune cells are obtained through a blood draw. For systemic administration, the monocytes are differentiated into dendritic cells, matured, activated and loaded with biomarkers from the subject's own tumor tissue. The loading of biomarkers into the dendritic cells "educates" the cells about what the immune system needs to attack. The activated, educated dendritic cells are then isolated with very high purity and are administered to the subject through a simple intradermal injection in the upper arm. The dendritic cells then convey the tumor biomarker information to the rest of the immune system agents (T cells, B cells and others) which then target cells with these biomarkers.

For intratumoral administration, the monocytes are differentiated into dendritic cells and partially matured. The cells are then administered by injection directly into the tumors. After injection into the tumors, dendritic cells pick up the tumor biomarkers in situ and convey the tumor biomarker information to the rest of the immune system agents (T cells, B cells and others) which then target cells with these biomarkers.

In some embodiments, dendritic cell compositions can be used as a first treatment, or a primer, for a second immunotherapy treatment (such as immune checkpoint inhibitor). By administering the dendritic cell compositions before the checkpoint inhibitor(s), the immune system (specifically T-cells) become activated which allows an enhanced response to checkpoint inhibitors. It is further reminded that Suv39h1 inhibition has also been shown to enhance immune checkpoint effect (in particular anti-PD1 effect).

Dendritic cell therapy provokes anti-tumor responses by causing dendritic cells to present tumor antigens to lymphocytes, which activates them, priming them to kill other cells that present the antigen. Dendritic cells are antigen presenting cells (APCs) in the mammalian immune system. In cancer treatment they aid cancer antigen targeting. One example of cellular cancer therapy based on dendritic cells is sipuleucel-T.

### Virus-based cancer vaccines

The virus-based vaccine can be divided into three forms: inactivated, live attenuated, or subunit vaccines against the virus that can cause the tumor; the oncolytic virus vaccine and the virus vector vaccine. Epstein-Barr virus, HBV, Hepatitis C virus, and HPV are the most common cancer-related viruses. Viruses are naturally immunogenic and their genetic material can be engineered to contain sequences encoding tumor antigens. Oncolytic virus contain herpes simplex virus, adenovirus, measles, vaccinia, reovirus, vesicular stomatitis virus The engineered virus vaccines can present tumor antigens in large quantities in the immune system and produce anti-tumor immunity. Furthermore, the oncolytic virus can be used as a vector as well. Except for providing tumor antigens, the virus itself can also lyse the tumor, release tumor antigens, further increase the vaccine's effectiveness, and produce long-term immune memory. In addition to the herpes simplex virus, the adenovirus is a frequently used oncolytic virus, used as delivery vectors for specific genes as well. Adenovirus is easy to manipulate, has a very broad spectrum of host cell tropism and can be rapidly prepared in large quantities. Other vectors include vaccinia virus, lentivirus and adeno-associated virus. Lentivirus and adeno-associated virus have the unique ability to stably and long-termly express the transgene in non-dividing cells the same as adenovirus.

### Peptide-based subunit vaccines

Peptide-based subunit vaccines employ targeting distinct tumor antigens, which may be delivered as peptides/proteins typically composed of known or predicted tumor antigen epitopes or as genetically-engineered DNA vectors, viruses, bacteria, or the like

Peptide-based subunit vaccines, include chemical and biosynthetic preparations of predicted or known specific tumor antigens. Due to the limitation of MHC polymorphism and the small size of antigen epitopes themselves, the immunogenicity of peptide-based vaccines can be weak weak. It is often difficult to cause a robust immune response, which also leads to immune tolerance. Adjuvants are combined with peptide-based vaccines to enhance the immune response generally. The present invention that enhance immune response associated to vaccination fulfill an important therapeutic need.

In some embodiments, peptide-based cancer vaccines comprise both CD8+ T epitopes and CD4+ T cell epitopes. CD8+ T cell epitopes activate CTLs tumor immunity through the antigens cross-presentation pathway, while CD4+ T cells activate helper T cells to maintain the function of CTLs.

Proteins or peptides may be made by any technique known to those of skill in the art, including the expression of proteins, polypeptides or peptides through standard molecular biological techniques, the isolation of proteins or peptides from natural sources, in vitro translation, or the chemical synthesis of proteins or peptides.

Peptides can be readily synthesized chemically utilizing reagents that are free of contaminating bacterial or animal substances (Merrifield RB: Solid phase peptide synthesis. I. The synthesis of a tetrapeptide. J. Am. Chem. Soc. 85:2149-54, 1963).

The use of the polypeptides provided herein, or portions thereof, under in vivo conditions may require their chemical modification since the polypeptides themselves may not have a sufficiently long serum and/or tissue half-life. It may also be advantageous to modify the polypeptides in order to impose a conformational restraint upon them. This might be useful, for example, to mimic a naturally-occurring conformation of the polypeptide in the context of the native protein in order to optimize the effector immune responses that are elicited. Modified polypeptides are referred to herein as "analog" polypeptides. The term "analog" extends to any functional and/or structural equivalent of a peptide characterized by its increased stability and/or efficacy in- vivo or in- vitro in respect of the practice of the invention. The term "analog" also is used herein to extend to any amino acid derivative of the polypeptides as described herein.

Analogs of the polypeptides or peptides contemplated herein include, but are not limited to, modifications to side chains, and incorporation of unnatural amino acids and/or their derivatives, non-amino acid monomers and cross-linkers. Other methods which impose conformational constraint on the polypeptides or their analogs are also contemplated. It will be apparent that the polypeptides of the invention can be modified in a variety of different ways without significantly affecting the functionally important immunogenic behavior thereof. Possible modifications to the polypeptide sequence may include the following:
The said polypeptides of the invention or analogs thereof may occur as single length or as multiple tandem or non-tandem repeats. A single type of polypeptide or analog may form the repeats or the repeats may be composed of different molecules including suitable carrier molecules.

The immunogenicity of the polypeptides of the present invention may also be modulated by coupling to fatty acid moieties to produce lipidated polypeptides. The polypeptides may also be conjugated to a lipidated amino acid.

With regard to peptide vaccines, there are a number of strategies for amplifying an immunogen's effectiveness, particularly as related to the art of vaccines. For example, cyclization or circularization of a peptide can increase the peptide's antigenic and immunogenic potency.

A small peptide antigen can also be conjugated to a suitable carrier, usually a protein molecule. It can thus allow multiple copies of an antigen, such as a peptide, to be conjugated to a single larger carrier molecule. Additionally, the carrier may possess properties which facilitate transport, binding, absorption or transfer of the antigen. Suitable carriers include, e.g., keyhole limpet hemocyanin (KLH), serum albumin, purified protein derivative of tuberculin (PPD), ovalbumin, non-protein carriers and many others. The conjugation between a peptide and a carrier can be accomplished using one of the methods known in the art. Specifically, the conjugation can use bifunctional cross-linkers as binding agents as detailed, for example, by Means and Feeney, "A recent review of protein modification techniques" Bioconjugate Chem. 1 :2-12 (1990).

Alternatively, a nucleic acid (e.g., a polynucleotide) encoding an antigenic peptide may be used to produce the antigenic peptide in vitro. The polynucleotide may be, e.g., DNA, cDNA, PNA, CNA, RNA, either single-and/or double-stranded, or native or stabilized forms of polynucleotides.

An expression vector capable of expressing a polypeptide can also be prepared. Expression vectors for different cell types are well known in the art and can be selected without undue experimentation. Generally, the DNA is inserted into an expression vector, such as a plasmid, in proper orientation and correct reading frame for expression, if necessary, the DNA may be linked to the appropriate transcriptional and translational regulatory control nucleotide sequences recognized by the desired host (e.g., bacteria), although such controls are generally available in the expression vector. The vector is then introduced into the host bacteria for cloning using standard techniques (see, e.g., Sambrook et al. (1989) Molecular Cloning, A Laboratory Manual, Cold Spring Harbor Laboratory, Cold Spring Harbor, N.Y.).

Expression vectors comprising the isolated polynucleotides, as well as host cells containing the expression vectors, are also contemplated. The antigenic peptides may be provided in the form of RNA or cDNA molecules encoding the desired antigenic peptides. One or more antigenic peptides may also be encoded by a single expression vector.

The term "polynucleotide encoding a polypeptide" encompasses a polynucleotide which includes only coding sequences for the polypeptide as well as a polynucleotide which includes additional coding and/or non-coding sequences. Polynucleotides can be in the form of RNA or in the form of DNA. DNA includes cDNA, genomic DNA, and synthetic DNA; and can be double- stranded or single-stranded, and if single stranded can be the coding strand or non-coding (anti- sense) strand.

The polynucleotides may comprise the coding sequence for antigenic peptide fused in the same reading frame to a polynucleotide which aids, for example, in expression and/or secretion of a polypeptide from a host cell (e.g., a leader sequence which functions as a secretory sequence for controlling transport of a polypeptide from the cell ). In some embodiments, the polynucleotides can comprise the coding sequence for the antigenic peptide fused in the same reading frame to a marker sequence that allows, for example, for purification of the encoded polypeptide, which may then be incorporated into the vaccine or immunogenic composition. In some embodiments, the polynucleotides may comprise the coding sequence for one or more of the antigenic peptides fused in the same reading frame to create a single concatamerized neoantigenic peptide construct capable of producing multiple antigenic peptides.

Once assembled (e.g., by synthesis, site-directed mutagenesis, or another method), the polynucleotide sequences encoding a particular isolated polypeptide of interest can be inserted into an expression vector and optionally operatively linked to an expression control sequence appropriate for expression of the protein in a desired host. Proper assembly can be confirmed by nucleotide sequencing, restriction mapping, and expression of a biologically active polypeptide in a suitable host. As well known in the art, in order to obtain high expression levels of a transfected gene in a host, the gene can be operatively linked to transcriptional and translational expression control sequences that are functional in the chosen expression host.

Recombinant expression vectors may be used to amplify and express DNA encoding antigenic peptides. Recombinant expression vectors are typically replicable DNA constructs which have synthetic or cDNA-derived DNA fragments encoding a tumor specific antigenic peptide or a bioequivalent analog operatively linked to suitable transcriptional or translational regulatory elements derived from mammalian, microbial, viral or insect genes. A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences. Such regulatory elements can include an operator sequence to control transcription. The ability to replicate in a host, usually conferred by an origin of replication, and a selection gene to facilitate recognition of transformants can additionally be incorporated. DNA regions are operatively linked when they are functionally related to each other. For example, DNA for a signal peptide (secretory leader) is operatively linked to DNA for a polypeptide if it is expressed as a precursor which participates in the secretion of the polypeptide; a promoter is operatively linked to a coding sequence if it controls the transcription of the sequence; or a ribosome binding site is operatively linked to a coding sequence if it is positioned so as to permit translation. Generally, operatively linked means contiguous, and in the case of secretory leaders, operatively linked means contiguous and in the reading frame. Structural elements intended for use in yeast expression systems include a leader sequence enabling extracellular secretion of translated protein by a host cell. Alternatively, where recombinant protein is expressed without a leader or transport sequence, it can include an N-terminal methionine residue. This residue can optionally be subsequently cleaved from the expressed recombinant protein to provide a final product.

Useful expression vectors for eukaryotic hosts, especially mammals or humans include, for example, vectors comprising expression control sequences from SV40, bovine papilloma virus, adenovirus and cytomegalovirus. Useful expression vectors for bacterial hosts include known bacterial plasmids, such as plasmids from Escherichia coli, including pCR 1, pBR322, pMB9 and their derivatives, wider host range plasmids, such as Ml 3 and filamentous single- stranded DNA phages.

Suitable host cells for expression of a polypeptide include prokaryotes, yeast, insect or higher eukaryotic cells under the control of appropriate promoters. Prokaryotes include gram negative or gram positive organisms, for example E. coli or bacilli. Higher eukaryotic cells include established cell lines of mammalian origin. Cell-free translation systems could also be employed. Appropriate cloning and expression vectors for use with bacterial, fungal, yeast, and mammalian cellular hosts are well known in the art (see Pouwels et al, Cloning Vectors: A Laboratory Manual, Elsevier,. Y., 1985).

Various mammalian or insect cell culture systems are also advantageously employed to express recombinant protein. Examples of suitable mammalian host cell lines include the COS -7 lines of monkey kidney cells, described by Gluzman (Cell 23: 175, 1981), and other cell lines capable of expressing an appropriate vector including, for example, L cells, C127, 3T3, Chinese hamster ovary (CHO), 293, HeLa and BHK cell lines. Mammalian expression vectors can comprise nontranscribed elements such as an origin of replication, a suitable promoter and enhancer linked to the gene to be expressed, and other 5 ' or 3' flanking nontranscribed sequences, and 5' or 3' nontranslated sequences, such as necessary ribosome binding sites, a polyadenylation site, splice donor and acceptor sites, and transcriptional termination sequences.

The proteins produced by a transformed host can be purified according to any suitable method. Such standard methods include chromatography (e.g., ion exchange, affinity and sizing column chromatography, and the like), centrifugation, differential solubility, or by any other standard technique for protein purification. Affinity tags such as hexahistidine, maltose binding domain, influenza coat sequence, glutathione-S-transferase, and the like can be attached to the protein to allow easy purification by passage over an appropriate affinity column. Isolated proteins can also be physically characterized using such techniques as proteolysis, nuclear magnetic resonance and x-ray crystallography.

### Nucleic acid base cancer vaccine compositions

Cancer DNA vaccines are based on bacterial plasmids that encode one or several oncology antigens inducing innate immunity activation and adaptive immune responses. In some embodiments, DNA plasmids are directly transfected into APCs, mainly through intradermal delivery.

In some embodiments, in addition to the combined administration with a KMT inhibitor which is the subject of the present invention, other adjuvants can be used to enhance the immunogenicity of DNA vaccines, such as CpG motifs, polymer, nanoparticle, liposome, and small molecule agonists.

Typically, nucleic acid-based vaccines include multiple antigens. Typically, nucleic acid-based vaccines include a plurality or more than one, preferably 2 to 20, more preferably 2 to 20, most preferably 2 to 6 of antigen or epitope-encoding nucleic acid. Said combinations typically comprise more than one antigen or epitope-encoding nucleic acid, preferably encoding different peptides or proteins which comprise or provide preferably different epitopes, particularly of different tumor antigens, or pathogenic antigens.

In some embodiment antigens can be administered to an individual in need thereof by use of a plasmid. These plasmids typically comprises a strong viral promoter to drive the in vivo transcription and translation of the nucleic acid of interest (see e.g., Mor, et al,(1995). The Journal of Immunology 155 (4): 2039-2046). Intron may also be included to improve mRNA stability and increase protein expression (Leitner et al, (1997). The Journal of Immunology 159 (12): 6112-61 19). Plasmids also typically include a strong polyadenylation/transcriptional termination signal, (Alarcon et al" (1999). Adv. Parasitol. Advances in Parasitology 42: 343-410; Robinson et al., (2000). Adv. Virus Res. Advances in Virus Research 55: 1-74). Multi cistronic vectors can be constructed to express more than one immunogen, or to express an immunogen and an immunostimulatory molecule (Lewis et al., (1999). Advances in Virus Research (Academic Press) 54: 129-88).

It is to be noted that advantageously in some embodiments, a multi cistronic vector can be constructed to include one or more immunogens as above defined and an inhibitor of a SUV KMT as previously defined herein.

In some embodiments, a vaccine or immunogenic composition may include separate DNA plasmids encoding, for example, one or more antigenic peptides/polypeptides and/or encoding an inhibitor of a SUV39 KMT as previously defined. The choice of expression vectors typically depends upon the peptide/polypeptides to be expressed, and is well within the skill of the ordinary artisan.

Plasmids can be introduced in vivo by a number of different methods. The two most popular approaches are injection of DNA in saline, using a standard hypodermic needle, and gene gun delivery. A schematic outline of the construction of a DNA vaccine plasmid and its subsequent delivery by these two methods into a host is illustrated at Scientific American (Weiner et al., (1999) Scientific American 281 (I): 34-41). Alternative delivery methods may include aerosol instillation of naked DNA on mucosal surfaces, such as the nasal and lung mucosa, (Lewis et al., (1999). Advances in Virus Research (Academic Press) 54: 129-88) and topical administration of pDNA to the eye and vaginal mucosa (Lewis et al., (1999) Advances in Vims Research (Academic Press) 54: 129-88). Mucosal surface delivery has also been achieved using cationic liposome-DNA preparations, biodegradable microspheres, attenuated Shigella or Listeria vectors for oral administration to the intestinal mucosa, and recombinant adenovirus vectors.

In some embodiments, one or more antigenic peptides may be encoded and expressed in vivo using a viral-based system (e.g., an adenovirus system, an adeno associated virus (AAV) vector, a poxvirus, or a lentivirus). In some embodiments, the vaccine or immunogenic composition may include a viral based vector for use in a human individual in need thereof, such as, for example, an adenovirus. Plasmids that can be used for adeno associated virus, adenovirus, and lentivirus delivery are well known in the field. Typically, AAV and lentivirus delivery are widely used and comply well with the present invention.

In some embodiments, a nucleic acid construct is a mammalian expression cassette, preferably human expression cassette, wherein the one or more coding sequences of the one or more antigens are operably linked to appropriate regulatory sequence(s) for their expression in an individual's target cells or tissue(s). In some particular embodiments, the target cell(s) or tissue(s) is epithelial cell(s) or tissue(s). Such sequences which are well-known in the art include in particular a promoter, and further regulatory sequences capable of further controlling the expression of a transgene, such as without limitation, enhancer, terminator and intron. In some particular embodiments, the expression cassette comprises a promoter; preferably further comprises one or more of an enhancer, terminator or intron. As previously mentioned in some embodiments the expression cassette may include a sequence coding for a KMT inhibitor, said coding sequence may be operably linked to the sequences coding for the one or more antigens or may include regulatory sequences. In other embodiments, the KMT inhibitor can be provided as a separate expression cassette.

The promoter may be a tissue-specific, ubiquitous, constitutive or inducible promoter that is functional in the individual's target cells or tissue, in particular epithelial cell(s) or tissue(s). Examples of constitutive promoters which can be used in the present invention include without limitation: phosphogly cerate kinase promoter (PGK), elongation factor- 1 alpha (EF-1 alpha) promoter including the short form of said promoter (EFS), viral promoters such as cytomegalovirus (CMV) immediate early enhancer and promoter (optionally with the CMV enhancer), cytomegalovirus enhancer/chicken beta actin (CAG) promoter, SV40 early promoter and retroviral 5' and 3' LTR promoters including hybrid LTR promoters. Preferred ubiquitous promoter is CMV promoter. Examples of inducible promoters which can be used in the present invention include Tetracycline-regulated promoters. The promoters are advantageously human promoters, i.e., promoters from human cells or human viruses. Such promoters are well-known in the art and their sequences are available in public sequence data bases.

In some preferred embodiments, the nucleic acid construct is DNA, wherein the coding sequence of the antigen is operably linked to appropriate regulatory sequence(s) for their expression in an individual's target cells or tissue(s) as disclosed above. The DNA construct advantageously comprises a mammalian expression cassette as disclosed above.

In some embodiments, the nucleic acid construct is RNA, preferably mRNA, wherein the coding sequence the antigen is operably linked to appropriate regulatory sequence(s) for their expression in an individual's target cells or tissue(s). mRNA vaccines are well-known in the art (reviewed in Jackson et ah, Vaccines, 2020, 5, 11, doi.10.1038). mRNA is delivered into the host cell cytoplasm where expression generates the antigen of interest. mRNA construct comprises a cap structure, 5' and 3 'untranslated regions (UTRs), and open reading frame (ORF), and a 3'poly(A) tail. mRNA construct may be non-replicating mRNA (MRM) or self-amplifying mRNA (SAM). SAM comprises the inclusion of genetic replication machinery derived from positive- strand mRNA viruses, most commonly alphavimses such as Sindbis and Semliki- Forest viruses. In SAM constructs, the ORF encoding viral structural protein is replaced by the transcript encoding the vaccine antigen of interest, and the viral RNA-dependent RNA polymerase is retained to direct cytoplasmic amplification of the replicon construct. Trans- replicating RNA are disclosed for example in WO 2017/162461. RNA replicon from alphavims suitable for gene expression are disclosed in WO 2017/162460. mRNA manufacturing process uses plasmid DNA (pDNA) containing a DNA-dependent RNA polymerase promoter, such as T7, and the corresponding sequence for the mRNA construct. The pDNA is linearized to serve as a template for the DNA-dependent RNA polymerase to transcribe the mRNA, and subsequently degraded by a DNase process step. The addition of the 5 'cap and the 3 'poly (A) tail can be achieved during the in vitro transcription step or enzymatically after transcription. Enzymatic addition of the cap can be accomplished by using guanylyl transferase and 2'-0-methyltransferase to yield a CapO(N7MeGpppN) or CapI (NTMeQpppN2 oMe) structure, respectively, while the poly-A tail can be achieved through enzymatic addition via poly-A polymerase. mRNA is then purified using standard methods

In some embodiments of RNA vaccines as per the present invention, the antigen or epitope-encoding RNA is mono-, bi-, or multicistronic, preferably as defined herein. Bi- or multicistronic RNAs typically comprise two (bicistronic) or more (multicistronic) open reading frames (ORF). An open reading frame in this context is a sequence of codons that is translatable into a peptide or protein. The coding sequences in a bi- or multicistronic epitope-encoding RNA preferably encode distinct epitopes (or antigens or variants or fragments thereof comprising said epitopes) as defined herein. Bi- or even multicistronic epitope-encoding RNAs, may encode, for example, at least two, three, four, five, six or more (preferably different) epitopes (or antigens or variants or fragments thereof comprising said epitopes) as defined herein. The term "encoding two or more epitopes/pathway inhibitors" may mean, without being limited thereto, that the bi- or even multicistronic epitope-encoding RNA, may encode e.g. at least two, three, four, five, six or more (preferably different) epitopes (or antigens or variants or fragments thereof comprising said epitopes).

In some embodiments, the coding sequences encoding two or more epitopes (or antigens or variants or fragments thereof comprising said epitopes) may be separated in the bi- or multicistronic RNA by at least one IRES (internal ribosomal entry site) sequence. The term "IRES" (internal ribosomal entry site) refers to an RNA sequence that allows for translation initiation. An IRES can function as a sole ribosome binding site, but it can also serve to provide a bi- or even multicistronic epitope-encoding RNA as defined above, which encodes several epitopes (or antigens or variants or fragments thereof comprising said epitopes), which are to be translated by the ribosomes independently of one another. Examples of IRES sequences, which can be used according to the invention, are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

According to further embodiments the at least one coding sequence of the epitope-encoding RNA of the inventive combination may encode at least two, three, four, five, six, seven, eight and more epitopes (or antigens or variants or fragments thereof comprising said epitopes) as defined herein linked with or without an amino acid linker sequence, wherein said linker sequence can comprise rigid linkers, flexible linkers, cleavable linkers (e.g., self-cleaving peptides) or a combination thereof. Therein, epitopes (or antigens or variants or fragments thereof comprising said epitopes) may be identical or different or a combination thereof.

Preferably, the epitope-encoding RNA comprises a length of about 50 to about 20000, or 100 to about 20000 nucleotides, preferably of about 250 to about 20000 nucleotides, more preferably of about 500 to about 10000, even more preferably of about 500 to about 5000.

The epitope-encoding RNA of the inventive combination may further be single stranded or double stranded. When provided as a double stranded RNA, the epitope-encoding RNA of the inventive combination preferably comprises a sense and a corresponding antisense strand.

In preferred embodiments, the epitope-encoding RNA of the inventive combination is an mRNA, a viral RNA or a replicon RNA, preferably a mRNA.

In some embodiments, the one or more nucleic acid of a vaccine composition (notably the antigen or epitope encoding RNA(s)) may thus be provided as a "stabilized" nucleic acids, i.e. which is essentially resistant to in vivo degradation (e.g. by an exo- or endo-nuclease).

Such stabilization can be effected, for example, by a modified phosphate backbone of the epitope-encoding RNA (or any other nucleic acid, in particular RNA, as defined herein). A backbone modification in connection with the present invention is a modification in which phosphates of the backbone of the nucleotides contained in said RNA (or any other nucleic acid, in particular RNA, as defined herein) are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized epitope-encoding RNAs (or other nucleic acids, in particular RNAs, as defined herein) may further include, for example: non-ionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-O-(1-thiophosphate)). Specific modifications are described, which are preferably capable of "stabilizing" the epitope-encoding RNA of the inventive combination (or any other nucleic acid, in particular RNA, as defined herein) as well detailed in US2020085944A1.

In any of the above-described embodiments, the nucleic acid construct as above defined may comprise at least one open frame encoding for an antigenic peptide and an open frame encoding for a KMT inhibitor (typically a suv39h1 inhibitor) as previously defined.

Various vectors have been used to improve the delivery efficiency of mRNA cancer vaccines, including several viral, non-viral, and cell-based vehicles among which LNPs are the most widely used delivery system (Delivery methods for RNA vaccines suitable for the present invention in particular related to LNPs have been recently review in Liu, J., Fu, M., Wang, M. et al. Cancer vaccines as promising immuno-therapeutics: platforms and current progress. J Hematol Oncol 15, 28 (2022), the content of which is summarized after). LNPs are mainly composed of ionizable amino lipid-like molecules, phospholipids, cholesterol, as well as lipid-anchored PEG. Optimization of ionizable lipids focuses on regulating head groups, connectors, and alkyl chains to regulate acid dissociation constant (pKa), fusion properties and metabolic behavior. Representative ionizable lipids include dilinoleylmethyl-4-dimethylaminobutyrate (DLin-MC3-DMA), 1,2-dioleoyl-sn-glycerol-3-phosphoethanolamine (DOPE). Furthermore, the ideal lipid material should be metabolized and cleared quickly after mRNA delivery, thus reducing the toxic side effects caused by the carrier and allowing multiple administrations. The uptake pathways of LNPs mainly contain apolipoprotein or albumin receptor-mediated endocytosis and non-specific pinocytosis. Recently, LNPs have been modified to achieve targeted delivery. For example, LNPs modified with mannose could target DCs through the mannose receptor CD206 (see Beissert T, Perkovic M, Vogel A, Erbar S, Walzer KC, Hempel T, et al. A trans-amplifying RNA vaccine strategy for induction of potent protective immunity. Mol Ther. 2020;28(1):119-28). Moreover, LNPs prepared by chip-based microfluidic devices have the advantages of high stability and repeatability, which is beneficial to GMP production of LNPs.

In preferred embodiments, the at least one RNA of (pharmaceutical) composition or vaccine (or any other nucleic acid, in particular RNA, as defined herein) is thus provided in a complexed form, i.e. complexed or associated with one or more (poly- )cationic compounds, preferably with (poly-)cationic polymers, (poly-)cationic peptides or proteins, e.g. protamine, (poly-)cationic polysaccharides and/or (poly-)cationic lipids. In this context, the terms "complexed" or "associated" refer to the essentially stable combination of the at least one RNA (or said other nucleic acid, in particular RNA) with one or more of the aforementioned compounds into larger complexes or assemblies without covalent binding.

In some embodiments, the RNA (or any other nucleic acid, in particular RNA, as defined herein) is complexed or associated with lipids (in particular cationic and/or neutral lipids) to form one or more liposomes, lipoplexes, lipid nanoparticles, or nanoliposomes. Therefore, in some embodiments, RNA composition or vaccine (or any other nucleic acid, in particular RNA, as defined herein) is provided in the form of a lipid-based formulation, in particular in the form of liposomes, lipoplexes, and/or lipid nanoparticles comprising said RNA (or said other nucleic acid, in particular RNA). It is also conceivable to provide the KMT inhibitor (notably the scuv39h1 inhibitor) complexed or associated with lipids to form one or more liposomes, lipoplexes, or lipid nanoparticles.

In some embodiments, RNAs of the (pharmaceutical) composition or vaccine (or any other nucleic acid, in particular RNA, as defined herein) are formulated as lipoplexes, i.e. cationic lipid bilayers sandwiched between nucleic acid (e.g. (epitope-encoding) RNA or DNA) layers.

In some embodiments, RNAs of the (pharmaceutical) composition or vaccine (or any other nucleic acid, in particular RNA, as defined herein) are formulated as emulsions.

In another embodiments, said RNAs (and optionally inhibitor of KMT) are formulated in a cationic oil-in-water emulsion where the emulsion particle comprises an oil core and a cationic lipid which can interact with the nucleic acid(s) anchoring the molecule to the emulsion particle. In some embodiments, said RNA is formulated in a water-in-oil emulsion comprising a continuous hydrophobic phase in which the hydrophilic phase is dispersed.

In some embodiments, the RNA of the vaccine composition (or any other nucleic acid, in particular RNA, as defined herein) is is complexed or associated with a cationic or polycationic compound ("(poly-)cationic compound") and/or a polymeric carrier. The term "(poly-)cationic compound" typically refers to a charged molecule, which is positively charged (cation) at a pH value typically from 1 to 9, preferably at a pH value of or below 9 (e.g. from 5 to 9), of or below 8 (e.g. from 5 to 8), of or below 7 (e.g. from 5 to 7), most preferably at a physiological pH, e.g. from 7.3 to 7.4.

Poly-)cationic compounds being particularly preferred agents for complexation or association of RNA of the (pharmaceutical) composition or vaccine (or any other nucleic acid, in particular RNA, as defined herein) include protamine, nucleoline, spermine or spermidine, or other cationic peptides or proteins, such as poly-L-lysine (PLL), poly-arginine, basic polypeptides, cell penetrating peptides (CPPs), including HIV-binding peptides, HIV-1 Tat (HIV), Tat-derived peptides, Penetratin, VP22 derived or analog peptides, HSV VP22 (Herpes simplex), MAP, KALA or protein transduction domains (PTDs), PpT620, prolin-rich peptides, arginine-rich peptides, lysine-rich peptides, MPG-peptide(s), Pep-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, or histones.

In some embodiments, the RNA of the (pharmaceutical) composition or vaccine (or any other nucleic acid, in particular RNA, as defined herein) is complexed or associated with a polymeric carrier. A "polymeric carrier" suitable to the present ivention might be a polymeric carrier formed by disulfide-crosslinked cationic components. The disulfide-crosslinked cationic components may be the same or different from each other. The polymeric carrier can also contain further components.

### Pharmaceutical compositions and methods of administration

Typically, the compositions of the present invention are pharmaceutical compositions and formulations for administration, preferably sterile compositions and formulations.

A pharmaceutical composition of the disclosure generally comprises a vaccine composition and/or a KMT inhibitor in association with pharmaceutically acceptable carriers. Main pharmaceutically acceptable carriers include saline, solvents, dispersion media, coatings, antibacterial and antifungal agents, isotonic and absorption delaying agents, and the like, compatible with pharmaceutical administration. Supplementary active compounds can further be incorporated into the compositions. A pharmaceutical composition is formulated to be compatible with its intended route of administration.

The present invention therefore encompasses pharmaceutical compositions comprising an effective amount of one or more compounds according to the present invention (including a pharmaceutically acceptable salt, thereof), optionally in combination with a pharmaceutically acceptable carrier, excipient or additive.

When administered as a combination, the (therapeutic) agents or components (i.e. the vaccine or immunogenic composition and the KMT inhibitor notably the Suv39h1) of the present invention can be formulated as separate compositions that are given at the same time or different times, or the two (therapeutic) components can be given as a single composition.

The compositions may be administered once daily, twice daily, once every two days, once every three days, once every four days, once every five days, once every six days, once every seven days, once every two weeks, once every three weeks, once every four weeks, once every two months, once every six months, or once per year. The dosing interval can be adjusted according to the needs of individual patients. For longer intervals of administration, extended release or depot formulations can be used.

The compositions of the invention can be used to treat diseases and disease conditions that are acute, and may also be used for treatment of chronic conditions. In particular, the compositions of the invention are used in methods to treat or prevent a neoplasia or an infectious disease.

In certain embodiments, the compounds of the invention are administered for time periods exceeding two weeks, three weeks, one month, two months, three months, four months, five months, six months, one year, two years, three years, four years, or five years, ten years, or fifteen years; or for example, any time period range in days, months or years in which the low end of the range is any time period between 14 days and 15 years and the upper end of the range is between 15 days and 20 years (e.g., 4 weeks and 15 years, 6 months and 20 years). In some cases, it may be advantageous for the compounds of the invention to be administered for the remainder of the patient's life. In some embodiments, the patient is monitored to check the progression of the disease or disorder, and the dose is adjusted accordingly. In some embodiments, treatment according to the invention is effective for at least two weeks, three weeks, one month, two months, three months, four months, five months, six months, one year, two years, three years, four years, or five years, ten years, fifteen years, twenty years, or for the remainder of the subject's life.

As described herein, in certain embodiments, administration of the KMT inhibitor is initiated before initiation of administration of the vaccine or immunogenic composition. In other embodiments, administration of the inhibitor is initiated after initiation of administration of the vaccine or immunogenic composition. In still other embodiments, administration of the KMT inhibitor is initiated simultaneously with the initiation of administration of the vaccine or immunogenic composition.

Administration of KMT the inhibitor, such as a suv39h1 inhibitor, may continue every 2, 3, 4, 5, 6, 7, 8 or more weeks after the first administration of the said KMT inhibitor.

In combination therapies, one or more KMT inhibitors, such as a Suv39h1 inhibitor, may be administered to the patient throughout the treatment regimen.

In certain embodiments, administration of the KMT inhibitor, such as a Suv39h1 inhibitor, is withheld during the week prior to administration of the vaccine or immunogenic composition. In other embodiments, administration of the KMT inhibitor, is withheld during administration of the vaccine or immunogenic composition.

Surgical resection uses surgery to remove abnormal tissue in cancer, such as mediastinal, neurogenic, or germ cell tumors, or thymoma. In certain embodiments, administration of the KMT inhibitor, is initiated following tumor resection. In other embodiments, administration of a cancer vaccine or immunogenic composition is initiated 1, 2, 3, 4, 5, 6, 7, 8, 9, 10, 11, 12, 13, 14, 15 or more weeks after tumor resection. In some embodiments, administration of the cancer vaccine or immunogenic composition is initiated 4, 5, 6, 7, 8, 9, 10, 11 or 12 weeks after tumor resection.

Prime/boost regimens refer to the successive administrations of a vaccine or immunogenic or immunological compositions. In certain embodiments, administration of the vaccine or immunogenic composition is in a prime/ boost dosing regimen, for example administration of the neoplasia vaccine or immunogenic composition at weeks 1, 2, 3 or 4 as a prime and administration of the vaccine or immunogenic composition is at months 2, 3 or 4 as a boost. In another embodiment heterologous prime -boost strategies are used to elicit a greater cytotoxic T-cell response (see Schneider et ah, Induction of CD8+ T cells using heterologous prime-boost immunization strategies, Immunological Reviews Volume 170, Issue 1, pages 29-38, August 1999). In another embodiment DNA encoding antigens is used to prime followed by a protein boost. In another embodiment protein is used to prime followed by boosting with a virus encoding the antigen. In another embodiment a virus encoding the antigen is used to prime and another virus is used to boost. In another embodiment protein is used to prime and DNA is used to boost. In some embodiments, a DNA vaccine or immunogenic composition is used to prime a T-cell response and a recombinant viral vaccine or immunogenic composition is used to boost the response. In some embodiments, a viral vaccine or immunogenic composition is co-administered with a protein or DNA vaccine or immunogenic composition to act as an adjuvant for the protein or DNA vaccine or immunogenic composition. The patient can then be boosted with either the viral vaccine or immunogenic composition, protein, or DNA vaccine or immunogenic composition (see Hutchings et al., Combination of protein and viral vaccines induces potent cellular and humoral immune responses and enhanced protection from murine malaria challenge. Infect Immun, 2007 Dec;75(I2):5SI9-26. Epub 2007 Oct 1).

As used herein, the term "fixed intermittent dosing regimen" refers to repeating cycles of preplanned drug administration in which the drug is administered on one or more consecutive days ("days on") followed by one or more consecutive days of rest on which the drug is not administered ("days off).

The pharmaceutical compositions can be processed in accordance with conventional methods of pharmacy to produce medicinal agents for administration to patients in need thereof, including humans and other mammals.

One of ordinary skill in the art from this disclosure and the knowledge in the art will recognize that a therapeutically effective amount of one of more compounds according to the present invention may vary with the condition to be treated, its severity, the treatment regimen to be employed, the pharmacokinetics of the agent used, as well as the patient (animal or human) treated.

To prepare the pharmaceutical compositions according to the present invention, a therapeutically effective amount of one or more of the compounds according to the present invention can be intimately admixed with a pharmaceutically acceptable carrier according to conventional pharmaceutical compounding techniques to produce a dose. A carrier may take a wide variety of forms depending on the form of preparation desired for administration, e.g., ocular, oral, topical or parenteral, including gels, creams ointments, lotions and time released implantable preparations, among numerous others. In preparing pharmaceutical compositions in oral dosage form, any of the usual pharmaceutical media may be used. Thus, for liquid oral preparations such as suspensions, elixirs and solutions, suitable carriers and additives including water, glycols, oils, alcohols, flavoring agents, preservatives, coloring agents and the like may be used. For solid oral preparations such as powders, tablets, capsules, and for solid preparations such as suppositories, suitable carriers and additives including starches, sugar carriers, such as dextrose, mannitol, lactose and related carriers, diluents, granulating agents, lubricants, binders, disintegrating agents and the like may be used. If desired, the tablets or capsules may be enteric- coated or sustained release by standard techniques.

The active compound is included in the pharmaceutically acceptable earner or diluent in an amount sufficient to deliver to a patient a therapeutically effective amount for the desired indication, without causing serious toxic effects in the patient treated,

The vaccine or immunogenic composition and the KMT inhibitor, such as suv39h1 inhibitor, and any additional agents, may be administered by injection, orally, parenterally, by inhalation spray, rectally, vaginally, or topically in dosage unit formulations containing conventional pharmaceutically acceptable carriers, adjuvants, and vehicles. The term parenteral as used herein includes, into a lymph node or nodes, subcutaneous, intravenous, intramuscular, intrasternal, infusion techniques, intraperitoneally, eye or ocular, intravitreal, intrabuccal, transdermal, intranasal, into the brain, including intracranial and intradural, into the joints, including ankles, knees, hips, shoulders, elbows, wrists, directly into tumors, and the like, and in suppository form. In certain embodiments, the vaccine or immunogenic composition and/or the KMT inhibitor, are administered intravenously or subcutaneously.

Application of the subject therapeutics may be local, so as to be administered at the site of interest. In certain embodiments, the KMT inhibitor, such as suv39h1 inhibitor, is administered subcutaneously near the site of administration of the vaccine or immunogenic composition. It is to be understood by one skilled in the art administering the compositions that the concentration of the KMT inhibitor, administered to the subject may be changed based on the nature of the inhibitor, its specificity and the location of administration.

The combination therapy according to the present invention may be utilized in combination with at least one known other therapeutic agent, or a pharmaceutically acceptable salt of said agent.

With reference to cancer treatment, a combined cancer treatment can include but is not limited to cancer chemotherapeutic agents, cytotoxic agents, hormones, anti-angiogens, radiolabelled compounds, immunotherapy, surgery, cryotherapy, and/or radiotherapy.

Conventional cancer chemotherapeutic agents include alkylating agents, antimetabolites, anthracyclines, topoisomerase inhibitors, microtubule inhibitors and B-raf enzyme inhibitors.

Alkylating agents include the nitrogen mustards (such as mechlorethamine, cyclophosphamide, ifosfamide, melphalan and chlorambucil), ethylenamine and methylenamine derivatives (such as altretamine, thiotepa), alkyl sulfonates (such as busulfan), nitrosoureas (such as carmustine, lomustine, estramustine), triazenes (such as dacarbazine, procarbazine, temozolomide), and platinum-containing antineoplastic agents (such as cisplatin, carboplatin, oxaliplatin).

Antimetabolites include 5-fluorouracil (5-FU), 6-mercaptopurine (6-MP), Capecitabine (Xeloda^{®}), Cytarabine (Ara-C^{®}), Floxuridine, Fludarabine, Gemcitabine (Gemzar^{®}), Hydroxyurea, Methotrexate, Pemetrexed (Alimta^{®}).

Anthracyclines include Daunorubicin, Doxorubicin (Adriamycin^{®}), Epirubicin. Idarubicin. Other anti-tumor antibiotics include Actinomycin-D, Bleomycin, Mitomycin-C, Mitoxantrone.
Topoisomerase inhibitors include Topotecan, Irinotecan (CPT-11), Etoposide (VP-16), Teniposide or Mitoxantrone
Microtubule inhibitors include Estramustine, Ixabepilone, the taxanes (such as Paclitaxel, Docetaxel and Cabazitaxel), and the vinca alkaloids (such as Vinblastine, Vincristine, Vinorelbine, Vindesine and Vinflunine)
B-raf enzyme inhibitors include vemurafenib (Zelboraf), dabrafenib (Tafinlar), and encorafenib (Braftovi)
Immunotherapy includes but is not limited to immune checkpoint modulators (i.e. inhibitors and/or agonists), cytokines, immunomodulating monoclonal antibodies, cancer vaccines. Preferably immunotherapy includes administration immune checkpoint modulators. Examples include inhibitors of (e.g. antibodies that bind specifically to and inhibit activity of) PD-1, CTLA4, LAG3, BTLA, OX2R, TIM-3, TIGIT, LAIR-1, PGE2 receptors, and/or EP2/4 Adenosine receptors including A2AR. Preferably, the immune checkpoint modulators comprise anti-PD-1 and/or anti-PDL-1 inhibitors (e.g., anti-PD-1 and/or anti-PDL-1 antibodies).

In some embodiment, the combined cancer treatment is cell therapy an comprise administration of immune cell engineered to express one or more antigen receptors against one or more antigens. In some embodiments thereof, the said one or more antigens are also included in the vaccine composition. Well-suited cell therapy are typically described in WO2018234370A1, WO2021013950A1 or WO2023126458A1, the content of which is included herein by reference in their entirety.

Pharmaceutical vaccine or immunogenic compositions of the invention are administered in combination with a KMT inhibitor to a patient in an amount sufficient to elicit an immune response, typically a cell mediated immune response, more particularly a T cell mediated response and notably an effective CTL response to the pathogen or tumor antigen and to cure or at least partially arrest symptoms and/or complications. An amount adequate to accomplish this is defined as "therapeutically effective dose." Amounts effective for this use can depend on, e.g., the vaccine composition the KMT inhibitor, the manner of administration, the stage and severity of the disease being treated, the weight and general state of health of the patient, and the judgment of the prescribing physician. The patient's response and condition can be monitored by measuring specific CTL activity in the patient's blood to adapt doses and administration regimen.

### Therapeutic Methods

The present invention provides methods of stimulating, enhancing or raising an immune response against an infectious disease or a tumor in an individual, said method comprising administering a vaccination regimen and an immune stimulation regimen to the individual;
wherein the immune stimulation regimen comprises administration of KMT inhibitor, preferably wherein the KMT inhibitor is a Suv39H1 inhibitor;
wherein the immune stimulation regimen and the vaccination regimen are administered sequentially in any order or simultaneously,

The vaccination regimen comprises the administration of a vaccine composition as previosuly defined.

The invention also provides a method of raising an immune response to an antigen or enhancing or augmenting an immune response to an antigen in an individual, comprising administering to the individual an effective amount of KMT inhibitor; typically wherein an immune function or immune health of the individual is improved;

The invention also pertains to a method of treating and/or preventing an infectious disease or a tumor in an individual, said method comprising the administration to the individual of a medical preparation comprising:
a first component that elicits an immune response in the individual;
a second component comprising an KMT inhibitor notably a suv39h1 inhibitor;
wherein the first and second components can be administered sequentially in any order or simultaneously.

The invention also encompasses a method of treating and/or preventing a disease, disorder or condition associated with the expression or the elevated expression of an antigen in an individual, optionally wherein the disease is an infectious disease or a neoplastic disease (tumor), said method comprising the administration to the individual of:
a first component that elicits an immune response in the individual a second component comprising a KMT inhibitor;
wherein the first and second components can be administered sequentially in any order or simultaneously.

Typically the immune response is a response again one or more pathogen or tumor antigens as herein described. The immune response typically include a cell-mediated immune response, notably a T cell response, notably a T cell response, notably a CTL response. When the antigen is a tumor antigen, the methods of the invention lead to an increased neoplasia/tumor specific immune response in a subject, and are aimed at treating and or alleviating a symptom of cancer in a subject.

The herein methods and compositions may be used for a patient that has been diagnosed as having cancer, or at risk of developing cancer.

Typically, the described combination of the invention is administered in an amount sufficient to induce a CTL response. Administration of a KMT inhibitor may allow to reduce vaccination regimen and/or to increase and or extend immune response notably the immune response associated with particular type of antigen, such as antigen that have a low affinity and/or are expressed at low expression levels. This might be of high relevance for tumor specific antigen derived from dark or grey genome. In some embodiment, the composition, medical preparation and methods of the invention prevent and or limit recall infection or tumor relapse.

Patients may be screened before administration and after the administration of the compositions described herein. Patients may undergo screening assessments that document historical health status as well as their current and future health status in general and as related to their underlying disease. The screening assessments may include tests like vital signs (including diastolic and systolic blood pressure, heart rate, temperature, weight, and height), electrocardiograms, symptom directed physical exam, hematology (including hematocrit, hemoglobin, RBC count, WBC count with differential, and platelet count), chemistry (including tests for glucose, urea nitrogen, creatinine, sodium, potassium, calcium, total and direct bilirubin, AST, ALT, alkaline phosphatase, lactate dehydrogenase (LDH), and adrenocorticotropic hormone), liver function tests (such as detecting levels of AST, ALT, total and direct bilirubin), pregnancy testing, CT or MRI, surgical or core needle biopsy of a primary or metastatic tumor site for DNA and RNA sequencing, immunological analysis. Biopsies may be used to evaluate the presence of T-cell infiltrates in the tumor and their localization with respect to tumor margins by tests like immunohistochemistry, western blot analysis, RNA and DNA analysis. The presence of tumor-associated macrophages and DCs within the tumor micro-environment may also be evaluated. The list of markers for analysis may include, but not be limited to, the following: CD3, CD4, CD8, CD45RO, PD-L1, PD-I, FoxP3, Granzyme B, Perforin, CD68, CD163, MHC Class I, MHC Class II, CD83 AND CD1 lb.

Immune response parameters may be analyzed for changes over time from baseline levels before the administration of the treatments and may include summaries of characterization of nucleic acids (e.g., DNA mutations, transcript abundance), histopathology, and immune cell analyses in tissues obtained at the Pretreatment, Pre-vaccination Treatment, and Vaccination Treatment phases as well as at the time of preliminary assessment. Reporting the test results may include tables depicting shifts from earlier time points in order to compare changes in immune parameters. Descriptive statistics and frequency distributions may be used as appropriate. Immunological analyses may include summaries for CD8+ and CD4+ T-cell response measured by ex vivo IFN-g ELISpot and assessed through spot counts. Analyses can be used to assess changes from pretreatment to pre-vaccination treatment to vaccination treatment to preliminary assessment. Reporting may include medians and inter-quartile ranges, as well as tables depicting shifts from earlier time points for each patient. Additionally, nonparametric tests (e.g., Wilcoxon signed-rank test) may be used to determine differences in the ELISpot data between time points as appropriate. Fold changes in biomarkers measured on a continuous scale may be summarized and compared across response categories using the Wilcoxon rank-sum test between treatment arms for every cohort. For multi-gene assays, genes may be grouped into analysis sets to characterize biological functions of the cells as appropriate.

Tests and results may include a detailed characterization of the phenotype and abundance of antigen-specific T cells, both in the periphery and in the tumor microenvironment. The abundance of regulatory cells such as regulatory T cells or myeloid-derived suppressor cells, and T-cell recognition, activation, and cytotoxicity may also be evaluated with PBMCs and tumor cells. Additionally, ex vivo induction of neoantigen T-cell responses may also be performed on peripheral blood and leukopheresis samples. Presence of circulating tumor DNA (ctDNA) and vaccine-specific antibody responses may be evaluated after treatment with the compositions described herein.

The herein-described compositions and methods may be used on patients in need thereof with any cancer according to the general flow process comprising subject identification, collecting informed consent and pre-screening the patients. Patients can then undergo an assessment of the specific type of cancer and the mutations causing the cancer. The nucleic acid material collected from a patient may be used for exome sequencing (tumor and normal tissue), RNA sequencing for the preparation of specific personalized vaccine. Patients in need thereof may receive a series of priming vaccinations with a mixture of personalized tumor-specific peptides. Additionally, over a 4 week period the priming may be followed by two boosts during a maintenance phase. All vaccinations are subcutaneously delivered. The vaccine or immunogenic composition is evaluated for safety, tolerability, immune response and clinical effect in patients and for feasibility of producing vaccine or immunogenic composition and successfully initiating vaccination within an appropriate time frame. The first cohort can consist of 5 patients, and after safety is adequately demonstrated, an additional cohort of 10 patients may be enrolled. Peripheral blood is extensively monitored for peptide-specific T-cell responses and patients are followed for up to two years to assess disease recurrence.

For instance, the subject receiving the inventive combination, (pharmaceutical) composition or vaccine according to the invention may be a patient with cancer, preferably as defined herein, or a related condition, receiving chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors), antibody therapy and/or inhibitory and/or stimulatory checkpoint molecules (e.g. CTLA4 inhibitors), or a patient, who has achieved partial response or stable disease after having received one or more of the treatments specified above. Or, the subject receiving the inventive combination, (pharmaceutical) composition or vaccine according to the invention may be a patient with an infectious disease, preferably as defined herein, receiving antibiotic, antifungal or antiviral therapy.

In a further aspect, the present invention thus also relates to the use of the inventive combination, for supporting another therapy of cancer or an infectious disease. « Support" of the treatment or prophylaxis of cancer may be any combination of a conventional cancer therapy method of such as surgery, radiation therapy, chemotherapy (e.g. first-line or second-line chemotherapy), chemoradiation, treatment with tyrosine kinase inhibitors, treatment with inhibitory and/or stimulatory checkpoint molecules, preferably CTLA4 inhibitors, antibody therapy or any combination of these, and a therapy using the inventive combination, (pharmaceutical) composition or vaccine as defined herein.

Administration of the inventive combination, (pharmaceutical) composition or vaccine according to the invention may be accomplished prior to, simultaneously and/or subsequently to administering another therapeutic or subjecting the patient to another therapy that is useful for treatment of the particular disease or condition to be treated.

In preferred embodiments, the inventive combination is used for treatment or prophylaxis of cancer.

As used herein, the term "cancer" refers to a neoplasm characterized by the uncontrolled and usually rapid proliferation of cells that tend to invade surrounding tissue and to metastasize to distant body sites. The term encompasses benign and malignant neoplasms. Malignancy in cancers is typically characterized by anaplasia, invasiveness, and metastasis; whereas benign malignancies typically have none of those properties. The terms includes neoplasms characterized by tumor growth as well as cancers of blood and lymphatic system. Specific examples of cancer that can be treated with the inventive combination, (pharmaceutical) composition or vaccine include Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS-Related; Lymphoma, Central Nervous System (Primary); Lymphoma, Cutaneous T-Cell; Lymphoma, Hodgkin's, Adult; Lymphoma, Hodgkin's; Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood", Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor.

### Infectious Diseases

The inventive combination, pharmaceutical composition or kit may also be used for treating infectious diseases. The term "infection" or "infectious disease" relates to the invasion and multiplication of microorganisms such as bacteria, viruses, and parasites that are not normally present within the body. An infection may cause no symptoms and be subclinical, or it may cause symptoms and be clinically apparent. An infection may remain localized, or it may spread through the blood or lymphatic system to become systemic. Infectious diseases in this context, preferably include viral, bacterial, fungal or protozoological infectious diseases. Specific examples of infectious diseases that can be treated with the inventive combination, (pharmaceutical) composition or vaccine include Acinetobacter infections, African sleeping sickness (African trypanosomiasis), AIDS (Acquired immunodeficiency syndrome), Amoebiasis, Anaplasmosis, Anthrax, Appendicitis, Arcanobacterium haemolyticum infections, Argentine hemorrhagic fever, Ascariasis, Aspergillosis, Astrovirus infections, Athlete's foot, Babesiosis, Bacillus cereus infections, Bacterial meningitis, Bacterial pneumonia, Bacterial vaginosis (BV), Bacteroides infections, Balantidiasis, Baylisascaris infections, Bilharziosis, BK virus infections, Black piedra, Blastocystis hominis infections, Blastomycosis, Bolivian hemorrhagic fever, Borrelia infectionss (Borreliosis), Botulism (and Infant botulism), Bovine tapeworm, Brazilian hemorrhagic fever, Brucellosis, Burkholderia infections, Buruli ulcer, Calicivirus infections (Norovirus and Sapovirus), Campylobacteriosis, Candidiasis (Candidosis), Canine tapeworm infections, Cat-scratch disease, Chagas Disease (American trypanosomiasis), Chancroid, Chickenpox, Chlamydia infections, Chlamydia trachomatis infections, Chlamydophila pneumoniae infections, Cholera, Chromoblastomycosis, Climatic bubo, Clonorchiasis, Clostridium difficile infections, Coccidioidomycosis, Cold, Colorado tick fever (CTF), Common cold (Acute viral rhinopharyngitis; Acute coryza), Condyloma acuminata, Conjunctivitis, Creutzfeldt-Jakob disease (CJD), Crimean-Congo hemorrhagic fever (CCHF), Cryptococcosis, Cryptosporidiosis, Cutaneous larva migrans (CLM), Cutaneous Leishmaniosis, Cyclosporiasis, Cysticercosis, Cytomegalovirus infections, Dengue fever, Dermatophytosis, Dientamoebiasis, Diphtheria, Diphyllobothriasis, Donavanosis, Dracunculiasis, Early summer meningoencephalitis (FSME), Ebola hemorrhagic fever, Echinococcosis, Ehrlichiosis, Enterobiasis (Pinworm infections), Enterococcus infections, Enterovirus infections, Epidemic typhus, Epiglottitis, Epstein-Barr Virus Infectious Mononucleosis, Erythema infectiosum (Fifth disease), Exanthem subitum, Fasciolopsiasis, Fasciolosis, Fatal familial insomnia (FFI), Fifth disease, Filariasis, Fish poisoning (Ciguatera), Fish tapeworm, Flu, Food poisoning by Clostridium perfringens, Fox tapeworm, Free-living amebic infections, Fusobacterium infections, Gas gangrene, Geotrichosis, Gerstmann-Straussler-Scheinker syndrome (GSS), Giardiasis, Glanders, Gnathostomiasis, Gonorrhea, Granuloma inguinale (Donovanosis), Group A streptococcal infections, Group B streptococcal infections, Haemophilus influenzae infections, Hand foot and mouth disease (HFMD), Hantavirus Pulmonary Syndrome (HPS), Helicobacter pylori infections, Hemolytic-uremic syndrome (HUS), Hemorrhagic fever with renal syndrome (HFRS), Henipavirus infections, Hepatitis A, Hepatitis B, Hepatitis C, Hepatitis D, Hepatitis E, Herpes simplex, Herpes simplex type I, Herpes simplex type II, Herpes zoster, Histoplasmosis, Hollow warts, Hookworm infections, Human bocavirus infections, Human ewingii ehrlichiosis, Human granulocytic anaplasmosis (HGA), Human metapneumovirus infections, Human monocytic ehrlichiosis, Human papillomavirus (HPV) infections, Human parainfluenza virus infections, Hymenolepiasis, Influenza, Isosporiasis, Japanese encephalitis, Kawasaki disease, Keratitis, Kingella kingae infections, Kuru, Lambliasis (Giardiasis), Lassa fever, Legionellosis (Legionnaires' disease, Pontiac fever), Leishmaniasis, Leprosy, Leptospirosis, Lice, Listeriosis, Lyme borreliosis, Lyme disease, Lymphatic filariasis (Elephantiasis), Lymphocytic choriomeningitis, Malaria, Marburg hemorrhagic fever (MHF), Marburg virus, Measles, Melioidosis (Whitmore's disease), Meningitis, Meningococcal disease, Metagonimiasis, Microsporidiosis, Miniature tapeworm, Miscarriage (prostate inflammation), Molluscum contagiosum (MC), Mononucleosis, Mumps, Murine typhus (Endemic typhus), Mycetoma, Mycoplasma hominis, Mycoplasma pneumonia, Myiasis, Nappy/diaper dermatitis, Neonatal conjunctivitis (Ophthalmia neonatorum), Neonatal sepsis (Chorioamnionitis), Nocardiosis, Noma, Norwalk virus infections, Onchocerciasis (River blindness), Osteomyelitis, Otitis media, Paracoccidioidomycosis (South American blastomycosis), Paragonimiasis, Paratyphus, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Pfeiffer's glandular fever, Plague, Pneumococcal infections, Pneumocystis pneumonia (PCP), Pneumonia, Polio (childhood lameness), Poliomyelitis, Porcine tapeworm, Prevotella infections, Primary amoebic meningoencephalitis (PAM), Progressive multifocal leukoencephalopathy, Pseudo-croup, Psittacosis, Q fever, Rabbit fever, Rabies, Rat-bite fever, Reiter's syndrome, Respiratory syncytial virus infections (RSV), Rhinosporidiosis, Rhinovirus infections, Rickettsial infections, Rickettsialpox, Rift Valley fever (RVF), Rocky mountain spotted fever (RMSF), Rotavirus infections, Rubella, Salmonella paratyphus, Salmonella typhus, Salmonellosis, SARS (Severe Acute Respiratory Syndrome), Scabies, Scarlet fever, Schistosomiasis (Bilharziosis), Scrub typhus, Sepsis, Shigellosis (Bacillary dysentery), Shingles, Smallpox (Variola), Soft chancre, Sporotrichosis, Staphylococcal food poisoning, Staphylococcal infections, Strongyloidiasis, Syphilis, Taeniasis, Tetanus, Three-day fever, Tick-borne encephalitis, Tinea barbae (Barber's itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete's foot), Tinea unguium (Onychomycosis), Tinea versicolor (Pityriasis versicolor), Toxocariasis (Ocular Larva Migrans (OLM) and Visceral Larva Migrans (VLM)), Toxoplasmosis, Trichinellosis, Trichomoniasis, Trichuriasis (Whipworm infections), Tripper, Trypanosomiasis (sleeping sickness), Tsutsugamushi disease, Tuberculosis, Tularemia, Typhus, Typhus fever, Ureaplasma urealyticum infections, Vaginitis (Colpitis), Variant Creutzfeldt-Jakob disease (vCJD, nvCJD), Venezuelan equine encephalitis, Venezuelan hemorrhagic fever, Viral pneumonia, Visceral Leishmaniosis, Warts, West Nile Fever, Western equine encephalitis, White piedra (Tinea blanca), Whooping cough, Yeast fungus spots, Yellow fever, Yersinia pseudotuberculosis infections, Yersiniosis, and Zygomycosis.

### Kits and Co-Packaging

In an aspect, the invention provides kits containing any one or more of the elements discussed herein to allow administration of the combination therapy. Elements may be provided individually or in combinations, and may be provided in any suitable container, such as a vial, a bottle, or a tube. In some embodiments, the kit includes instructions in one or more languages, for example in more than one language. In some embodiments, a kit comprises one or more reagents for use in a process utilizing one or more of the elements described herein. Reagents may be provided in any suitable container. For example, a kit may provide one or more delivery or storage buffers. Reagents may be provided in a form that is usable in a particular process, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more of the vectors, proteins and/or one or more of the polynucleotides described herein. The kit may advantageously al low the provision of all elements of the systems of the invention. Kits can involve vector(s) and/or particle(s) and/or nanoparticle(s) containing or encoding nucleic acids (typically RNAs)(s) for 1 -50 or more antigens to be administered to an animal, mammal, primate, rodent, etc., with such a kit including instructions for administering to such a eukaryote; and such a kit can optionally include any of the anti-cancer agents described herein. The kit may include any of the components above (as well as instructions for use with any of the methods of the present invention.

In one embodiment the kit contains at least one vial with an immunogenic composition or vaccine and at least one vial with a KMT inhibitor. In one embodiment kits may comprise ready to use components that are mixed and ready to administer. In one aspect a kit contains a ready to use immunogenic or vaccine composition and a ready to use KMT inhibitor. The ready to use immunogenic or vaccine composition may comprise separate vials containing different pools of immunogenic compositions. The immunogenic compositions may comprise one vial containing a viral vector or DNA plasmid and the other vial may comprise immunogenic protein. The ready to use KMT inhibitor may comprise a cocktail of KMT inhibitors or a single KMT inhibitor agent or component. In another embodiment a kit may contain a ready to use KMT inhibitor and an immunogenic composition or vaccine in a ready to be reconstituted form. The immunogenic or vaccine composition may be freeze dried or lyophilized. The kit may comprise a separate vial with a reconstitution buffer that can be added to the lyophilized composition so that it is ready to be administered. The buffer may advantageously comprise an adjuvant or emulsion according to the present invention. In another embodiment the kit may comprise a ready to reconstitute KMT inhibitor and a ready to reconstitute immunogenic composition or vaccine. In this aspect both may be lyophilized. In this aspect separate reconstitution buffers for each may be included in the kit. The buffer may advantageously comprise an adjuvant or emulsion according to the present invention. In another embodiment the kit may comprise single vials containing a dose of immunogenic composition and KMT inhibitor agent that are administered together. In another aspect multiple vials are included so that one vial is administered according to a treatment timeline. One vial may only contain the KMT inhibitor agent or component for one dose of treatment, another may contain both the KMT inhibitor agent or component and immunogenic composition for another dose of treatment and one vial may only contain the immunogenic composition for yet another dose. In a further aspect the vials are labeled for their proper administration to a patient in need thereof. The immunogen or KMT inhibitor agent or component of any embodiment may be in a lyophilized form, a dried form or in aqueous solution as described herein. The immunogen may be a live attenuated virus, protein, or nucleic acid as described herein.

The inventive combination may also be used in combination therapy. Any other therapy useful for treating or preventing the diseases and disorders defined herein may be combined with the uses and methods disclosed herein.

Although the present invention and its advantages have been described in detail, it should be understood that various changes, substitutions and alterations can be made herein without departing from the spirit and scope of the invention as defined in the appended claims.

The present invention is further illustrated in the following Examples which are given for illustration purposes only and are not intended to limit the invention in any way.

### EXAMPLES

### Material and methods

### Mice, virus strains and cell lines

C57BL/6J male mice were obtained from Charles River and used at 6-10 weeks of age. Suv39h1tm1Jnw C57BL/6J (Suv39h1-KO) mice were kindly provided by T. Jenuwein and previously described (Peters, A. H. et al. Loss of the Suv39h histone methyltransferases impairs mammalian heterochromatin and genome stability. Cell 107, 323-337 (2001).), were backcrossed with C57BI6/J mice for at least nine generations. C57BL/6J-Tg(TcraTcrb)1100Mjb/J mice (OT-I), B6.SJL-Ptprca Pepcb/BoyJ (CD45.1). OT-I mice were crossed with CD45.1 mice to obtain OT-I/CD45.1/CD45.2 mice. Suv39h1Flox/Flox (official name is B6-Suv39h1tm1Ciphe) were originally produced at the Centre d'Immunologie de Marseille (CIPHE, B. Malissen) and were subsequently bred in the animal facility of Institut Curie, they are described in Niborski, L. L. et al. CD8+T cell responsiveness to anti-PD-1 is epigenetically regulated by Suv39h1 in melanomas. Nat Commun 13, 3739 (2022).

Mice were housed in Curie Institute SPF animal facilities. Live animal experiments were performed in accordance with the guidelines of the Ministere de l'enseignement superieur, de la recherche et de l'innovation (Project authorization: APAFIS #34372-2021121522311823 v1).

Influenza A/HKX31 (H3N2) and A/PR8 (H1N1) virus were purchased from Charles River. Influenza A/HKX31 (H3N2) and A/PR8 (H1N1) virus expressing the OVA peptide SIINFEKL, (X31-OVA and PR8-OVA, respectively) were kindly provided by Dr. Stephen Turner and Dr. Richard Webby (Jenkins MR, Webby R, Doherty PC, Turner SJ. Addition of a Prominent Epitope Affects Influenza A Virus-Specific CD8 + T Cell Immunodominance Hierarchies When Antigen Is Limiting. J Immunol. 1 sept 2006;177(5):2917-25).

B16F10-expressing OVA (B16F10-OVA) stably expressing Luciferase (B16F10-OVA-Luc) were produced by transducing B16F10-OVA cells with lentiviral particles produced with the pTRIP-SFFV-mtagBFP-2A plasmid (kindly provided by Dr. Nicolas Manel), as previously described (Cerboni et al J Exp Med. 2017 Jun 5;214(6):1769-1785).

### In-vivo staining and tissue preparation

5' before killing the mice, 15 ug of CD45.1-PE, CD45.1-APC, CD45.2-PE, CD45.2-APC, CD8a-PE or CD8a-APC were injected intravenously in the retroorbital sinus in 100 ul of PBS.

Lungs were digested using gentle MACS^{®} homogenizer (Milteny) in CO2 independent medium with DNAse I (Roche # 11284932001) and Liberase^{®} (Roche # 05-401-020-001) as previously described.

Liver was scratched using a 100 uM cell strainer and a syringe plunge in FACS buffer. Lymphocytes from from digested lung, IELs, LP and skin and smashed liver, samples were enriched by 40%/80% Percoll^{®} (Sigma-Aldrich) gradient as previously described ( Pertoft H, Laurent TC, Låås T, Kågedal L. Density gradients prepared from colloidal silica particles coated by polyvinylpyrrolidone (Percoll). Anal Biochem. 15 juill 1978;88(1):271-82.).

Mediastinal, inguinal and mesenteric lymphnodes and spleen were scratched using a 40 um cell strainer (Falcon^{®}) and a syringe plunge in CO2 independent medium (Gibco) supplemented with 10% FBS (Eurobio).

Finally, cell suspensions were stained with the indicated fluorochrome coupled antibodies and analysed by FACS.

### Cell staining for flow cytometry analysis

Cell suspensions were prepared in PBS and stained with Near IR or Acqua LIVE/DEAD^{™} Fixable Cell Stain Kit (Fisher Scientific) according to manufacturer's instructions to exclude dead cells and then labelled in PBS 0.5% BSA (Sigma-Aldrich) and 2 mM EDTA (Gibco) with antibodies. For some experiments, cells were fixed in 2% PFA (Electron Microscopy Sciences) during 20' at room temperature. Cell suspensions were analysed in a BD FacsVerse or a BD LSRFortessa cytometers. Data was analysed by Flowjo 10 software version: 10.8.0.

### Adoptive transfer

Secondary lymphoid organs (spleen, mesenteric, axillary, inguinal, brachial, cervical and lumbar lymph nodes) from Suv39H1 KO or WT OT-I male mice were collected, pooled and homogenized into single cell suspensions. Naive (CD44-) CD8+ T cells were enriched using the naive CD8+ T-cell isolation kit (Miltenyi Biotec, 130-096-543) according to manufacturer's instructions. 1 × 106 or 5x104 cells of a 1:1 mix of Suv39H1 KO and WT naive CD8+ T cells were adoptively transferred into Rag-KO or C57BL/6J male mice respectively. Cells were transferred intravenously by retroorbital injection. 24 h later, C57BL/6J mice were infected with the influenza virus strain X31-OVA.

### Tumours

1 × 106 B16-OVA-Luc cells were injected intravenously in the tale of the mice. Tumor burden was measured by bioluminescence imaging using Xenogen IVIS Imaging System (PerkinElmer) two times per week. Acquired bioluminescence data was analyzed using Living Image software (PerkinElmer) and expressed in average radiance (photons/sec/cm2/sr). The humane endpoint was defined by a maximum of 20% weight loss and additional clinical signs of distress.

### Flu infection

Mice were infected by intranasal instillation with a sublethal dose of Influenza X31 - OVA in 20 ul of PBS in the left nostril. Control group mice were instilled intranasally with 20 ul of PBS.

All infections were performed on anesthetized mice with ketamine (100mg/kg, Merial) and xylazine (7,5mg/kg, Bayer) in PBS administered intraperitoneally. The humane endpoint was defined by a maximum of 30% weight loss and additional clinical signs of distress.

### In vitro T cell culture

Secondary lymphoid organs (spleen, mesenteric, axillary, inguinal, brachial, cervical and lumbar lymph nodes) from Suv39H1 KO or WT OT-I male mice were collected, pooled and homogenized into single cell suspensions. Naive (CD44-) CD8+ T cells were enriched using the naive CD8+ T-cell isolation kit (Miltenyi Biotec, 130-096-543) according to manufacturer's instructions. Cells were then stained with Cell Trace Violet (Thermo Fisher) according to the manufacturer's instructions (In brief 1:1000 dilution in PBS, 1ml per cell population, 15min 37C, fill up to 10ml with complete medium, wash). 1x104 naive OTI CD8+ T cells were incubated for different time points with 2.5x104 splenocytes from CD3-KO mice preloaded with the indicated concentration of peptides or stimulated on αCD3 (BDBioscience) coated flat-bottomed 96-well plates (Corning Inc., Corning, NY; 10ug/ml, coating in PBS ON, seal the plates with parafilm) with 1ug/ml soluble □CD28 (BDBiosciences) in complete medium [RPMI, 100mM Hepes, 2mM Glutamax, 50uM 2-mercaptoethanol, 100U/100ug/ml Penicillin/Streptomycin, 1X Non-essential amino acids, 1mM Sodium Pyruvate (all Gibco, Thermo Fisher), 10% FCS (eurobio, Courtaboeuf, France)].

### Effect of SUV39H1 inactivation in human naïve T cells

The effect of SUV39H1 inactivation was investigated in human naive T cells. Total CD3+ T cells were purified from peripheral blood mononuclear cells (PBMCs), isolated from anonymous healthy donor leukapheresis rings (provided by EFS-Etablissement Francais du Sang), by density gradient centrifugation using Lymphoprep. Naive (CD45RA+CCR7+CD27+), Central Memory (CD45RA-CCR7+CD27+), and Effector Memory (CD45RO-CCR7-) were sorted from PBMC-purified total CD3+ T cells using BD FACSAria IIu sorter (Fig 2A.).

SUV39H1 inactivation by Crispr/Cas9 was performed in the sorted and bulk populations as follows: T cells were plated at a concentration 106 cells /ml using X-VIVO 15 media supplemented with 5 % human serum (Sigma Aldrich) and 0.5 µM β-mercaptoethanol, and stimulated with Dynabeads human T-Activator CD3/CD28 in a ratio of 1 bead per cell. Cells were recovered from the cultures 48 hours after and then subjected to magnetic removal of activation beads. We used a duplex (crRNA:tracrRNA) guide RNA format, which was generated by annealing at equimolar concentrations to form the duplex at 95 °C for 5 min. RNPs were then formed by combining precomplexed duplex (crRNA:tracrRNA) with Hifi Cas9 Nuclease V3 (IDT) at a molar ratio of 3:1 and incubating them 10 min at room temperature. Nucleofection was done with P3 Primary Cell 4D-Nuleofector^{®} X kit S adding between 2 x 106 cells with 10 µg of Cas9 in 5 µl of RNP and 20 µl of nucleofection solution. After electroporation using the EO-115 program (4D-Nucleofector Core Unit, Lonza), 200 µl of complete X-VIVO 15 with 500 IU/ml rhIL-2 was added to each reaction and cells were cultured at 32°C. After 16 h, cells were diluted in complete X-VIVO 15 with 500 IU/ml rhIL-2 at a concentration of 106 cells/ml. Cells were collected after 72 h (D0) to check KO efficiency, phenotyping, and in vitro restimulation with Dynabeads as above.

SUV39H1 inactivation efficiency was tested indirectly by looking into the levels of H3K9me3, which were reduced in SUV39H1-KO T cells produced from all 3 sorted populations (Fig. B).

### Results

As illustrated in figure 1, the results show that naive T cells from SUV39H1-deficient mice show increased initial response, more particularly figure 1D illustrates that naive T cells deficient for Suv39h1 exhibit and increased response at lower antigen load or at lower antigen affinity as compared to naive wild type T cells.

Effect of SUV39H1 inactivation in human naive T cells is illustrated on Figure 2. Total CD3- and naive-derived SUV39H1-KO T cells showed increased frequencies of memory CD45RO+CD27+ cells compared to mock T cells (Fig. 2C). In contrast, neither central memory- nor effector memory-derived T cells showed enrichment for memory T cells in SUV39H1-KO compared to mock (Fig. 2C). A week after stimulation with aCD3/aCD28 beads, naive-derived SUV39H1-KO were enriched in memory CD62L+ cells and depleted in exhausted TIM-3+PD-1+ cells, like total CD3-derived cells (Fig. D, E). In central memory-derived SUV39H1-KO cells, SUV39H1 inactivation had no effect on the proportions of memory/effector differentiation (Fig. 2 D, E). Effector memory-derived T cells (both mock and SUV39H1-KO) failed to survive after restimulation in vitro. These results indicate that SUV39H1 primarily contributes to heterochromatin formation at stem/memory loci in differentiating naive, but not in already committed central memory T cells.

Figure 3 shows that Suv39h1 deficient mice have increased memory population at homeostatic state, in particular the results shows that the numbers of central memory T cells (CD44+CD62L+) is increased in lymphoid organs (e.g., spleen, lymph nodes) while Effector Memory T cells (CD44+CD62L-) are more elevated in tissues (e.g., liver). Moreoever the results show an increased numbers of CD69+ tissues resident T cells in Suv39h1 KO mice.

Figure 4 provides in vivo evidence that Suv39h1 provides better protection in flu recall infections. In a model of Heterosubtypic flu infection, SUV39H1-KO CD8+ T cells expand faster and CD69+/CD103+ resident memory cells are increased following vaccination.

The inventors have further noted that decreased viral titer is decreased after vaccination upon Suv39h1 inhibition (data not shown).

## Claims

1. A medical preparation comprising:
a first component that elicits an immune response in an individual a second component comprising an inhibitor of a lysine methyl transferase (KMT);
wherein the first and second components can be administered to the individual sequentially in any order or simultaneously;
optionally wherein the medical preparation is for treating and/or preventing a disease, disorder or condition associated with the expression or the elevated expression of an antigen, optionally wherein the disease is an infectious disease or a tumor;
optionally wherein the medical preparation prevents recall infections or tumor relapses, optionally wherein the medical preparation prevents, reduces or delays tumor metastasis growth.

2. A method of treating and/or preventing an infectious disease or a tumor in an individual, said method comprising the administration to the individual of a medical preparation comprising:
a first component that elicits an immune response in the individual;
a second component comprising a KMT inhibitor;
wherein the first and second components can be administered sequentially in any order or simultaneously.

3. A method of treating and/or preventing a disease, disorder or condition associated with the expression or the elevated expression of an antigen in an individual, optionally wherein the disease is an infectious disease or a neoplastic disease (tumor), said method comprising the administration to the individual of:
a first component that elicits an immune response in the individual a second component comprising KMT inhibitor;
wherein the first and second components can be administered sequentially in any order or simultaneously.

4. A medical preparation of claim 1 or a therapeutic method of claim 2 or 3, wherein the first component comprises (i) antigen derived from a pathogen or a cancer cell, (ii) a peptide comprising one or more epitopes for inducing an immune response against an antigen, (iii) a polynucleotide encoding the peptide, (iv) one or more antigen presenting cells (APCs) comprising the peptide or the polynucleotide encoding the peptide, (v) a T cell receptor (TCR) specific for the neoepitope in complex with an HLA protein, (vi) a chimeric antigen receptor (CAR) specific for the neoepitope, or an (vii) an immune cell comprising a receptor as defined in iv) or v), or a combination thereof.

5. The medical preparation of claim 1, wherein the medical preparation raises, enhances or augments an immune response to an antigen, optionally wherein the immune response is a cell-mediated immune response, notably a T cell mediated immune response, notably wherein the immune response comprises the generation of antigen-specific T cells.

6. A medical preparation or a therapeutic method according to any preceding claim, wherein the first component is an immunogenic or vaccine composition, optionally wherein the first component is present in a formulation for targeting the lymphatic system, e.g., secondary lymphoid organs, in particular spleen, optionally wherein the first component is present in a formulation for targeting APCs, optionally dendritic cells, optionally wherein dendritic cells are immature dendritic cells.

7. A medical preparation or a therapeutic method according to any preceding claim, wherein the second component increases sensitivity and initial response of naive T cells; and/or increases expansion and recall response of memory cells.

8. A medical preparation or a therapeutic method according to any preceding claim, wherein the first and second components are present in the same or separate formulations.

9. A method of stimulating an immune response against an infectious disease or a tumor in an individual, said method comprising administering a vaccination regimen and an immune stimulation regimen to the individual;
wherein the immune stimulation regimen comprises administration of an inhibitor of a KMT inhibitor, preferably wherein the KMT inhibitor is a Suv39H1 inhibitor;
wherein the immune stimulation regimen and the vaccination regimen are administered sequentially in any order or simultaneously,
optionally wherein the immune stimulation regimen increases sensitivity and initial response of naive T cells; and/or increases expansion and recall response of memory cells.

10. The method of claim 9, wherein the vaccination regimen comprises administration of a vaccine composition comprising (i) and antigen derived from pathogen or a cancer cell, (ii) a peptide comprising an an epitope for inducing an immune response against an antigen, (iii) a polynucleotide encoding the peptide, (iv) one or more APCs comprising the peptide or the polynucleotide encoding the peptide (v), or a combination thereof.

11. A method of raising an immune response to an antigen or enhancing or augmenting an immune response to an antigen in an individual, comprising administering to the individual an effective amount of a KMT inhibitor;
optionally wherein the immune response is a cell-mediated immune response, notably a T cell mediated immune response, notably wherein the immune response comprises the generation of antigen-specific T cells;
optionally wherein the immune is a response to a vaccination regimen.

12. A pharmaceutical composition comprising an KMT inhibitor, wherein the said composition increases sensitivity and initial response of naive T cells; and/or increases expansion and recall response of memory cells; wherein the pharmaceutical composition is a vaccine composition and/or is an immune cell composition; optionally wherein, the vaccine composition comprises (i) an antigen derived from a pathogen or a cancer cell, (ii) a peptide comprising an epitope of a protein for inducing an immune response against an antigen, (iii) a polynucleotide encoding the peptide, (iv) one or more APCs comprising the peptide or the polynucleotide encoding the peptide, or a combination thereof.
optionally wherein, the immune response against an infectious disease or a tumor is an immune response against one or more infectious disease antigens or is an immune response against one or tumor antigens, optionally wherein the one or more antigens is a tumor antigen, optionally wherein the antigen is a tumor associated antigen, optionally wherein the antigen is a tumor specific antigen.

13. A medical preparation, a therapeutic method or a composition according to any preceding claim, wherein the antigen is derived from a cancer cell or a human pathogen; optionally wherein the antigen is derived from a human pathogen selected from the group consisting of bacteria, virus, fungi, parasitic microorganisms and multicellular parasites ; optionally wherein the antigen is derived from a human pathogen and the human pathogen is selected from coronavirus, Herpes simplex virus (HSV), HIV, hepatitis B virus, hepatitis C virus, meningitis B, Haemophilus influenza type B, pertussis, diphtheria, tetanus, influenza virus, RSV, HPV, measles, rubella virus, mumps virus, HCMV, VZV, Dengue virus, poliovirus, Ebola virus and rotavirus, or any combination of two or more thereof.

14. A medical preparation, a therapeutic method or a composition according to any preceding claim, wherein the KMT inhibitor is a nucleic acid, a peptide, a ribonucleic complex, a small molecule or a combination thereof.

15. A medical preparation, a therapeutic method or a composition according to any preceding claim, wherein the KMT inhibitor is selected from SUV39H1 (KMT1A), SUV39H2 (KMT1B), G9a (EHMT2/KMT1C), G9a-like protein 1; GLP (EHMT1/KMT1D), SETDB1 (KMT1E), and SETDB2 (KMT1F).

16. A medical preparation, a therapeutic method or a composition according to any preceding claim, wherein the KMT inhibitor targets HP1 proteins.

17. A medical preparation, a therapeutic method or a composition according to any preceding claim, wherein the antigen is a neoantigen or a grey or dark genome antigen; optionally wherein the antigen is expressed at low expression level, typically as compared to TAAs.

18. A therapeutic method according to any preceding claim, wherein the individual has no detectable neoplasia or tumor but is at high risk for disease recurrence.

19. A therapeutic method according to any preceding claim, wherein the stimulating method further comprises administration of a checkpoint inhibition regimen.

20. The medical preparation or the method according to any preceding claim, which is for use in cancer in combination with at least one further therapeutic agent, optionally a chemotherapeutic agent, or an immunotherapeutic agent, optionally a checkpoint inhibitor.
